# EUROPÄISCHE PATENTANMELDUNG

(11) **EP 2 562 174 A1**
(43) Veröffentlichungstag der Anmeldung: **27.02.2013**
(21) Anmeldenummer: 11178566.3
(22) Anmeldetag: 24.08.2011
(51) Int. Cl.: C07D 401/12, C07D 403/12, C07D 403/14, C07D 407/14, C07D 413/12, C07D 413/14, C07D 417/14, A01N 43/40, A01N 43/54, A01N 43/653, A01N 43/713

(54) **Herbizid wirksame 6-Oxo-1,6-dihydropyrimidin-5-carboxamide und 2-Oxo-1,2-dihydropyridin-3-carboxamide**

(71) Anmelder: Bayer CropScience AG, 40789 Monheim (DE)
(72) Erfinder: Die Erfindernennung liegt noch nicht vor

(57) **Zusammenfassung**

Es werden 6-Oxo-1,6-dihydropyrimidin-5-carboxamide und 2-Oxo-1,2-dihydropyridin-3-carboxamide der allgemeinen Formel (I) als Herbizide beschrieben.

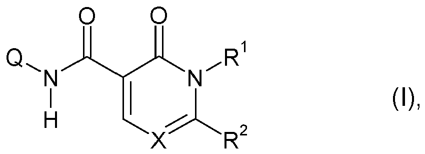

In dieser Formel (I) stehen R¹ und R² für Reste wie Wasserstoff, organische Reste wie Alkyl, und andere Reste wie Halogen. Q steht für einen Tetrazolyl-, Triazolyl oder Oxadiazolylrest.

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2011/031658 sind bestimmte herbizide wirksame 6-Oxo-1,6-dihydro-pyrimidin-5-carbonyl-Derivate bekannt. WO2007/088876 offenbart bestimmte herbizide wirksame 2-Oxo-1,2-dihydropyridin-Derivate. WO 2011/035874 A1 offenbart herbizid wirksame N-(1,2,5-Oxadiazol-3-yl)-benzamide. Die herbizide Wirksamkeit und/oder die Kulturpflanzenverträglichkeit der in diesen Schriften genannten Verbindungen ist jedoch nicht immer ausreichend.

Aufgabe der vorliegenden Erfindung war die Bereitstellung von herbizid wirksamen Verbindungen mit gegenüber den aus dem Stand der Technik bekannten Verbindungen verbesserten Eigenschaften.

Es wurde nun gefunden, dass N-(1,2,5-Oxadiazol-3-yl)-, N-(1,3,4-Oxadiazol-2-yl), N-(Tetrazol-5-yl)- und N-(Triazol-5-yl)-6-oxo-1,6-dihydropyrimidin-5-carboxamide und N-(1,2,5-Oxadiazol-3-yl)-, N-(1,3,4-Oxadiazol-2-yl), N-(Tetrazol-5-yl)- und N-(Triazol-5-yl)-2-oxo-1,2-dihydropyridin-3-carboxamide als Herbizide besonders gut geeignet sind.

Ein Gegenstand der vorliegenden Erfindung sind somit 6-Oxo-1,6-dihydropyrimidin-5-carboxamide und 2-Oxo-1,2-dihydropyridin-3-carboxamide der Formel (I) oder deren Salze 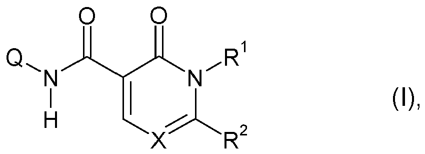 worin die Substituenten folgende Bedeutungen haben
X bedeutet N oder C-R³,
Q bedeutet einen Rest Q1, Q2, Q3 oder Q4, 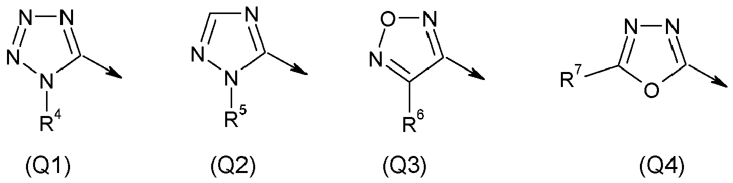
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfimyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(S-Phenyl), W¹-(SO₂-Phenyl), W²-(SO₂CH₂-Phenyl) oder W²-(SCH₂-Phenyl), wobei die Phenylringe der sieben vorstehend genannten Reste jeweils u Substituenten R⁸ tragen, oder
R¹ bedeutet G oder W²G,
R² bedeutet Phenyl oder W³-(Phenyl), wobei die Phenylringe der zwei vorstehend genannten Reste jeweils u Substituenten R⁸ tragen, oder
R² bedeutet G, W⁴G, Wasserstoff, Cyano, Hydroxy, Amino, Nitro, CHO, C(=O)OH, C(=O)NH₂, C(=S)NH₂, C(=O)NHCN, C(=O)NHOH, SH, SO₂NH₂, SO₂NHCN, SO₂NHOH, SF₅, NHCHO, NHNH₂, NHOH, NHCN, NHC(O)NH₂, (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₁-C₆)-Cycloalkyl-(C₁-C₆)-cycloalkyl, Halogen-(C₁-C₆)-Cycloalkyl-(C₁-C₆)-alkyl, (C₁-C₆)-Alkyl-(C₃-C₇)-cycloalkyl-(C₁-C₆)-alkyl, (C₃-C₈)-Cycloalkenyl, Halogen-(C₃-C₈)-Cycloalkenyl, (C₁-C₆)-Alkoxy (C₁-C₆)-alkyl, (C₁-C₆)-Alkoxy (C₁-C₆)-alkenyl, (C₁-C₆)-Alkyl-(C₃-C₇)-cycloalkyl, (C₁-C₆)-Alkoxy-(C₃-C₇)-cycloalkyl, (C₁-C₆)-Cycloalkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₂-C₈)-Alkylthioalkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxycarbonyl, (C₁-C₆)-Alkylamino-carbonyl, Di-(C₁-C₆)-alkyl-amino-carbonyl, (C₃-C₇)-Cycloalkyl-amino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-(C₁-C₆)-halogen-alkyl, Halogen-(C₁-C₆)-alkoxy-(C₁-C₆)-halogen-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₇)-Cycloalkyl-(C₁-C₆)-alkoxy, (C₂-C₆)-Alkenyloxy, Halogen-(C₂-C₆)-alkenyloxy, (C₂-C₆)-Alkinyloxy, Halogen-(C₃-C₆)-alkinyloxy, (C₁-C₆)-Alkoxy-(C₁-C₆)-alkoxy, (C₁-C₈)-Alkyl-carbonyloxy, Halogen-(C₁-C₈)-alkylcarbonyloxy, (C₃-C₇)-Cycloalkyl-carbonyloxy, (C₁-C₆)-Alky-carbonyl-(C₁-C₆)-alkoxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₁₀)-Cycloalkylsulfonyl, (C₁-C₆)-Alkylcarbonylthio, (C₁-C₆)-Alkyl(thiocarbonyl)thio, (C₃-C₈)-Cycloalkylsulfinyl, (C₁-C₆)-Alkylaminosulfonyl, Di-(C₁-C₆)-alkylaminosulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₈)-Cycloalkylamino, (C₂-C₈)-Alkylcarbonylamino, Halogen-(C₂-C₈)-alkylcarbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Halogen-tri-(C₁-C₄)-alkylsilyl,
   oder
R¹ und R² bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils m Stickstoffatome, n Sauerstoffatome, n Schwefelatome und n Elemente S(O), S(O)₂ oder C(O) als Ringglieder umfasst, dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₃-C₈)-Cycloalkyl, (C₂-C₈)-Alkoxyalkyl und Phenyl substituiert sind,
   dessen Stickstoffatome durch n Reste aus der Gruppe bestehend aus (C₁-C₆)-Alkyl und Phenyl substituiert sind,
   und worin die vorstehend genannten Phenylreste durch u Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
R³ bedeutet Wasserstoff, Halogen, Nitro. Cyano, (C₁-C₆)-Alkyl, (C₂-C₆)-Alkenyl, (C₂-C₆)-Alkinyl, (C₃-C₈)-Cycoalkyl, (C₃-C₈)-Cycloalkyl-(C₁-C₆)-alkyl, (C₁-C₆)-Alkoxy, (C₁-C₆)-Alkoxy-(C₁-C₆)-alkyl, (C₁-C₆)-Alkylthio, Halogen-(C₁-C₆)-alkylthio, (C₁-C₆)-Alkysulfinyl, (C₁-C₆)-Alkylsulfonyl, (C₁-C₆)-Alkylsulfonyl-(C₁-C₆)-alkyl, Halogen-(C₁-C₆)-alkylsulfinyl, Halogen-(C₁-C₆)-alkylsulfonyl, Halogen-(C₁-C₆)-alkyl, Halogen-(C₂-C₆)-alkenyl, Halogen-(C₁-C₆)-alkoxy, Phenyl oder W³-(Phenyl), wobei die Phenylringe der zwei vorstehend genannten Reste jeweils u Substituenten R⁸ tragen,
R⁴ bedeutet jeweils durch s Reste aus der Gruppe bestehend aus Nitro, Cyano, SiR¹¹₃, PO(OR¹¹)₃, S(O)ₙ-(C₁-C₆)-Alkyl, (C₁-C₆)-Alkoxy, Halogen-(C₁-C₆)-alkoxy, N(R⁹)₂, COR⁹, COOR⁹, OCOR⁹, OCO₂R⁹, NR⁹COR⁹, NR⁹SO₂R¹⁰, (C₃-C₆)-Cycloalkyl, Heteroaryl, Heterocyclyl, Phenyl, D-Heteroaryl, D-Heterocyclyl, D-Phenyl und D-Benzyl substituiertes (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei Heteroaryl, Heterocyclyl, Phenyl, D-Heteroaryl, D-Heterocyclyl, D-Phenyl und D-Benzy jeweils durch s Reste aus der Gruppe bestehend aus Methyl, Ethyl, Methoxy, Trifluormethyl und Halogen substituiert sind, und wobei Heterocyclyl n Oxogruppen trägt, oder
R⁴ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes (C₃-C₇)-Cycloalkyl, Heteroaryl, Heterocyclyl oder Phenyl,
R⁵ bedeutet jeweils durch s Reste aus der Gruppe bestehend aus Nitro, Cyano, SiR¹¹₃, PO(OR¹¹)₃, S(O)ₙ-(C₁-C₆)-Alkyl, (C₁-C₆)-Alkoxy, Halogen-(C₁-C₆)-alkoxy, N(R⁹)₂, COR⁹, COOR⁹, OCOR⁹, OCO₂R⁹, NR⁹COR⁹, NR⁹SO₂R¹⁰, (C₃-C₆)-Cycloalkyl, Heteroaryl, Heterocyclyl, Phenyl, D-Heteroaryl, D-Heterocyclyl, D-Phenyl und D-Benzyl substituiertes (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei Heteroaryl, Heterocyclyl, Phenyl, D-Heteroaryl, D-Heterocyclyl, D-Phenyl und D-Benzyl jeweils durch s Reste aus der Gruppe bestehend aus Methyl, Ethyl, Methoxy, Trifluormethyl und Halogen substituiert sind, und wobei Heterocyclyl n Oxogruppen trägt, oder R⁵ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes (C₃-C₇)-Cycloalkyl, Heteroaryl, Heterocyclyl oder Phenyl,
R⁶ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfanyl, Methylsulfinyl, Methylsulfonyl, (C₁-C₆)-Alkylcarbonyl-amino, (C₁-C₆)-Alkoxycarbonylamino, Benzoylamino, Methoxycarbonyl, Ethoxy-carbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁷ bedeutet Wasserstoff, (C₁-C₆)-Alkyl, R¹²O-(C₁-C₆)-Alkyl, CH₂R¹³, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹², NHR¹², Methoxycarbonyl, Ethoxycarbonyl, Methylcarbonyl, Trifluormethyl-carbonyl, Dimethylamino, Acetylamino, Methylsulfanyl, Methylsulfinyl, Methylsulfonyl, oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R⁸ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino;
R⁹ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹⁰ bedeutet (C₁-C₆)-Alkyl, (C₂-C₆)-Alkenyl, (C₂-C₆)-Alkinyl oder Phenyl,
R¹¹ bedeutet (C₁-C₆)-Alkyl,
R¹² bedeutet Wasserstoff, jeweils durch s Reste aus der Gruppe bestehend aus Cyano, Halogen, Nitro, Rhodano, OR¹⁴, S(O)ₙR¹⁵, N(R¹⁴)₂, NR¹⁴OR¹⁴, COR¹⁴, OCOR¹⁴, SCOR¹⁵, NR¹⁴COR¹⁴, NR¹⁴SO₂R¹⁵, CO₂R¹⁴, COSR¹⁵, CON(R¹⁴)₂ und (C₁-C₄)-Alkoxy-(C₂-C₆)-alkoxycarbonyl substituiertes (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁴-Heteroaryl oder (C₁-C₆)-Alkyl-NR¹⁴-Heterocyclyl, wobei Heterocyclyl n Oxogruppen trägt,
R¹³ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R¹⁴ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁵ bedeutet (C₁-C₆)-Alkyl, (C₂-C₆)-Alkenyl oder (C₂-C₆)-Alkinyl,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
W³ bedeutet (C₁-C₁₀)-Alkylen, (C₂-C₆)-Alkenylen oder (C₂-C₆)-Alkinylen,
W⁴ bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
D bedeutet O, S, oder NR¹⁰,
m bedeutet 1, 2, 3 oder 4,
n bedeutet 0, 1 oder 2,
s bedeutet 0, 1, 2 oder 3,
u bedeutet 0, 1, 2, 3, 4 oder 5.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Erfindungsgemäße Verbindungen können beispielsweise nach der in Schema 1 angegebenen Methode durch basenkatalysierte Umsetzung eines 6-Oxo-1,6-dihydropyrimidin-5-carbonsaeurechlorids bzw. eines 2-Oxo-1,2-dihydropyridin-3-carbonsäurechlorids (II) mit einem Amin (III) hergestellt werden:

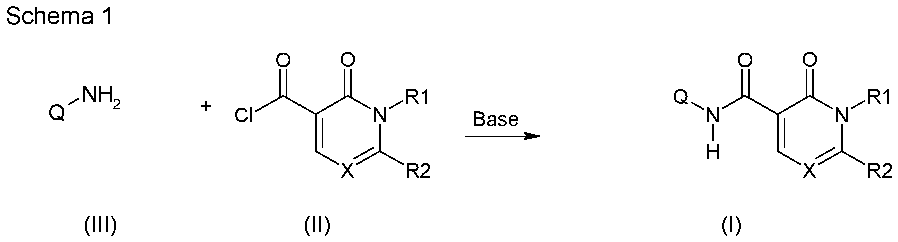

Die 6-Oxo-1,6-dihydropyrimidin-5-carbonsäurechloride der Formel (II) beziehungsweise die ihnen zugrunde liegenden 6-Oxo-1,6-dihydropyrimidin-5-carbonsäuren sind grundsätzlich bekannt und können beispielsweise gemäß den in WO 1993/21214 A1 oder WO 2011/031658 A1 beschriebenen Methoden hergestellt werden. Die 2-Oxo-1,2-dihydropyridin-3-carbonsäurechloride der Formel (II) beziehungsweise die ihnen zugrunde liegenden 2-Oxo-1,2-dihydropyridin-3-carbonsäuren sind grundsätzlich bekannt und können beispielsweise gemäß den in WO2007/088876 beschriebenen Methoden hergestellt werden. Die Amine der Formel (III), in denen Q (Q1) oder (Q2) bedeutet, sind grundsätzlich bekannt und können beispielsweise gemäß den in EP 10174893 beschriebenen Methoden hergestellt werden. Die Amine der Formel (III), in denen Q (Q3) bedeutet, sind grundsätzlich bekannt und können beispielsweise nach den in WO 2011/035874 A1 beschriebenen Methoden hergestellt werden. Die Amine der Formel (III), in denen Q (Q4) bedeutet, sind grundsätzlich bekannt und können beispielsweise gemäß den in EP 11159115 beschriebenen Methoden hergestellt werden.

Erfindungsgemäße Verbindungen können auch nach der in Schema 2 angegebenen Methode durch Umsetzung einer 6-Oxo-1,6-dihydropyrimidin-5-carbonsäure bzw. einer 2-Oxo-1,2-dihydropyridin-3-carbonsäure der Formel (IV) mit einem Amin (III) hergestellt werden:

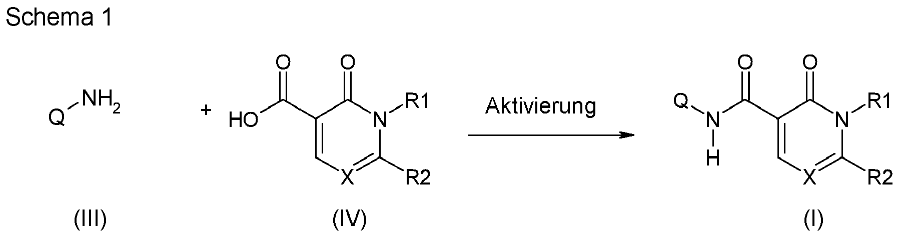

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Ganz besonders bevorzugt sind die in den Tabellen 1 bis 423 genannten Verbindungen der allgemeinen Formel (I), die analog der hier genannten Methoden erhältlich sind:

Die verwendeten Abkürzungen bedeuten:

| | | | | | | | | | | | |
|---|---|---|---|---|---|---|---|---|---|---|---|
| Et | = | Ethyl | Me | = | Methyl | n-Pr | = | n-Propyl | i-Pr | = | Isopropyl |
| c-Pr | = | Cyclopropyl | Ph | = | Phenyl | | | | | | |
| thf | = | Tetrahydrofuranyl | | | | thp | = | Tetrahydropyranyl | | | |

**Tabelle 1: Erfindungsgemäße Verbindungen der Formel (I), worin Q für Q1, R⁴ für Methyl sowie R² für Phenyl steht:**

| | | | | | |
|---|---|---|---|---|---|
| 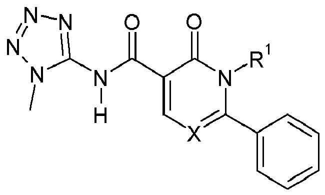 | | | | | |

| Nr. | R¹ | X | Nr. | R¹ | X |
|---|---|---|---|---|---|
| 1 | Me | N | 2 | Et | N |
| 3 | n-Pr | N | 4 | i-Pr | N |
| 5 | c-Pr | N | 6 | n-Bu | N |
| 7 | i-Bu | N | 8 | s-Bu | N |
| 9 | c-Bu | N | 10 | t-Bu | N |
| 11 | c-Pentyl | N | 12 | c-Hexyl | N |
| 13 | Ph | N | 14 | CH₂-c-Pr | N |
| 15 | CH₂-c-Bu | N | 16 | CH₂-SPh | N |
| 17 | CH₂SCH₃ | N | 18 | CH2CF3 | N |
| 19 | CH₂Ph | N | 20 | Ph(4-Me) | N |
| 21 | CH₂CH₂C≡CH | N | 22 | CH₂CH₂C≡CCH₃ | N |
| 23 | CH₂CH=CCl₂ | N | 24 | CH₂CH=CF₂ | N |
| 25 | CH₂CF=CF₂ | N | 26 | Ph(2,6-di-Me) | N |
| 27 | Ph(3,5-di-Me) | N | 28 | Ph(2-Me) | N |
| 29 | Ph(3-Me) | N | 30 | Ph(2,4-di-Me) | N |
| 31 | CH₂C≡CH | N | 32 | CH₂C≡CCH₃ | N |
| 33 | CH₂OMe | N | 34 | Ph(2-F) | N |
| 35 | Ph(3-F) | N | 36 | Ph(4-F) | N |
| 37 | Ph(2-Cl) | N | 38 | Ph(3-Cl) | N |
| 39 | Ph(4-Cl) | N | 40 | Ph(2-CN) | N |
| 41 | Ph(3-CN) | N | 42 | Ph(4-CN) | N |
| 43 | Ph(2-OMe) | N | 44 | Ph(3-OMe) | N |
| 45 | Ph(4-OMe) | N | 46 | Ph(2-NO₂) | N |
| 47 | Ph(3-NO₂) | N | 48 | Ph(4-NO₂) | N |
| 49 | Ph(2-CF₃) | N | 50 | Ph(3-CF₃) | N |
| 51 | Ph(4-CF₃) | N | 52 | Ph(2-Br) | N |
| 53 | Ph(3-Br) | N | 54 | Ph(4-Br) | N |
| 55 | Ph(2-OCF₃) | N | 56 | Ph(3-OCF₃) | N |
| 57 | Ph(4-OCF₃) | N | 58 | Ph(2-Et) | N |
| 59 | Ph(3-Et) | N | 60 | Ph(4-Et) | N |
| 61 | Ph(3,5-di-Cl) | N | 62 | Ph(2,4-di-Cl) | N |
| 63 | Ph(2,5-di-Cl) | N | 64 | Ph(2-Me-3F) | N |
| 65 | Ph(2-F-3-Me) | N | 66 | Ph(2,3-di-OMe) | N |
| 67 | Ph(2-Me-4F) | N | 68 | Ph(2-F-4-Me) | N |
| 69 | Ph(2,4-di-OMe) | N | 70 | Ph(2-Me-5F) | N |
| 71 | Ph(2-F-5-Me) | N | 72 | Ph(2,5-di-OMe) | N |
| 73 | Ph(3-Me-4F) | N | 74 | Ph(3-F-4-Me) | N |
| 75 | Ph(3,4-di-OMe) | N | 76 | Ph(3-Me-5F) | N |
| 77 | Ph(3-Cl-5-Me) | N | 78 | Ph(3,5-di-OMe) | N |
| 79 | Ph(3,5-di-F) | N | 80 | Ph(2,4-di-F) | N |
| 81 | Ph(2,5-di-F) | N | 82 | CH₂Ph(2-Me) | N |
| 83 | CH₂Ph(3-Me) | N | 84 | CH₂Ph(4-Me) | N |
| 85 | CH₂Ph(2-Me) | N | 86 | CH₂Ph(3-Me) | N |
| 87 | CH₂Ph(4-Me) | N | 88 | CH₂Ph(2-Cl) | N |
| 89 | CH₂Ph(3-Cl) | N | 90 | CH₂Ph(4-Cl) | N |
| 91 | CH₂CH₂OCH₂CF₃ | N | 92 | CH₂(thf-2-yl) | N |
| 93 | CH₂(thf-3-yl) | N | 94 | CH₂CH₂SCH₃ | N |
| 95 | CH₂CH₂SOCH₃ | N | 96 | CH₂CH₂SO₂CH₃ | N |
| 97 | CH₂CH₂OCH₃ | N | 98 | CH₂CH₂OCH₂CH₃ | N |
| 99 | CH₂CH₂CH₂OCH₂CH₃ | N | 100 | CH₂CH₂O(thf-2-yl) | N |
| 101 | CH₂CH₂O(thf-3-yl) | N | 102 | CH₂(thp-2-yl) | N |
| 103 | CH₂(thp-3-yl) | N | 104 | CH₂(thp-4-yl) | N |
| 105 | CH₂-CH(OMe)₂ | N | 106 | CH₂-CH(OEt)₂ | N |
| 107 | CH₂(dioxolan-2-yl) | N | 108 | CH₂(1,3-dioxan-2-yl) | N |
| 109 | CH₂(3-methyl-isoxazolin-5-yl | N | 110 | CH₂(3-methyl-isoxazol-5-yl | N |
| 111 | isoxazolin-4-yl | N | 112 | Thiazol-2-yl | N |
| 113 | Thiazol-3-yl | N | 114 | Oxazol-2-yl | N |
| 115 | 5-methylisoxazol-3-yl | N | 117 | 4-methyloxazol-2-yl | N |
| 117 | 4-methylthiazol-2-yl | N | 118 | 5-chloropyridin-2-yl | N |
| 119 | 5-methylpyridin-2-yl | N | 120 | 5-methoxypyridin-2-yl | N |
| 121 | 3-chloropyridin-4-yl | N | 122 | 3-methylpyridin-4-yl | N |
| 123 | 3-methoxypyridin-4-yl | N | 124 | CH₂CH₂CH=CH₂ | N |
| 125 | CHSOCH₃ | N | 126 | CH2SO₂CH₃ | N |
| 127 | Me | CH | 128 | Et | CH |
| 129 | n-Pr | CH | 130 | i-Pr | CH |
| 131 | c-Pr | CH | 132 | n-Bu | CH |
| 133 | i-Bu | CH | 134 | s-Bu | CH |
| 135 | c-Bu | CH | 136 | t-Bu | CH |
| 137 | c-Pentyl | N | 138 | c-Hexyl | CH |
| 139 | Ph | CH | 140 | CH2-c-Pr | CH |
| 141 | CH₂-c-Bu | CH | 142 | CH2-SPh | CH |
| 143 | CH₂SCH₃ | CH | 144 | CH₂CF₃ | CH |
| 145 | CH₂Ph | CH | 146 | Ph(4-Me) | CH |
| 147 | CH₂CH₂C≡CH | CH | 148 | CH₂CH₂C≡CCH₃ | CH |
| 149 | CH₂CH=CCl₂ | CH | 150 | CH₂CH=CF₂ | CH |
| 151 | CH₂CF=CF₂ | CH | 152 | Ph(2,6-di-Me) | CH |
| 153 | Ph(3,5-di-Me) | CH | 154 | Ph(2-Me) | CH |
| 155 | Ph(3-Me) | CH | 156 | Ph(2,4-di-Me) | CH |
| 157 | CH₂C≡CH | CH | 158 | CH₂C≡CCH₃ | CH |
| 159 | CH₂OMe | CH | 160 | Ph(2-F) | CH |
| 161 | Ph(3-F) | CH | 162 | Ph(4-F) | CH |
| 163 | Ph(2-Cl) | CH | 164 | Ph(3-Cl) | CH |
| 165 | Ph(4-Cl) | CH | 166 | Ph(2-CN) | CH |
| 167 | Ph(3-CN) | CH | 168 | Ph(4-CN) | CH |
| 169 | Ph(2-OMe) | CH | 170 | Ph(3-OMe) | CH |
| 171 | Ph(4-OMe) | CH | 172 | Ph(2-NO₂) | CH |
| 173 | Ph(3-NO₂) | CH | 174 | Ph(4-NO₂) | CH |
| 175 | Ph(2-CF₃) | CH | 176 | Ph(3-CF₃) | CH |
| 177 | Ph(4-CF₃) | CH | 178 | Ph(2-Br) | CH |
| 179 | Ph(3-Br) | CH | 180 | Ph(4-Br) | CH |
| 181 | Ph(2-OCF₃) | CH | 182 | Ph(3-OCF₃) | CH |
| 183 | Ph(4-OCF₃) | CH | 184 | Ph(2-Et) | CH |
| 185 | Ph(3-Et) | CH | 186 | Ph(4-Et) | CH |
| 187 | Ph(3,5-di-Cl) | CH | 188 | Ph(2,4-di-Cl) | CH |
| 189 | Ph(2,5-di-Cl) | CH | 190 | Ph(2-Me-3F) | CH |
| 191 | Ph(2-F-3-Me) | CH | 192 | Ph(2,3-di-OMe) | CH |
| 193 | Ph(2-Me-4F) | CH | 194 | Ph(2-F-4-Me) | CH |
| 195 | Ph(2,4-di-OMe) | CH | 196 | Ph(2-Me-5F) | CH |
| 197 | Ph(2-F-5-Me) | CH | 198 | Ph(2,5-di-OMe) | CH |
| 199 | Ph(3-Me-4F) | CH | 200 | Ph(3-F-4-Me) | CH |
| 201 | Ph(3,4-di-OMe) | CH | 202 | Ph(3-Me-5F) | CH |
| 203 | Ph(3-Cl-5-Me) | CH | 204 | Ph(3,5-di-OMe) | CH |
| 205 | Ph(3,5-di-F) | CH | 206 | Ph(2,4-di-F) | CH |
| 207 | Ph(2,5-di-F) | CH | 208 | CH₂Ph(2-Me) | CH |
| 209 | CH₂Ph(3-Me) | CH | 210 | CH₂Ph(4-Me) | CH |
| 211 | CH₂Ph(2-Me) | CH | 212 | CH₂Ph(3-Me) | CH |
| 213 | CH₂Ph(4-Me) | CH | 214 | CH₂Ph(2-Cl) | CH |
| 215 | CH₂Ph(3-Cl) | CH | 216 | CH₂Ph(4-Cl) | CH |
| 217 | CH₂CH₂OCH₂CF₃ | CH | 218 | CH₂(thf-2-yl) | CH |
| 219 | CH₂(thf-3-yl) | CH | 220 | CH₂CH₂SCH₃ | CH |
| 221 | CH₂CH₂SOCH₃ | CH | 222 | CH₂CH₂SO₂CH₃ | CH |
| 223 | CH₂CH₂OCH₃ | CH | 224 | CH₂CH₂OCH₂CH₃ | CH |
| 225 | CH₂CH₂CH₂OCH₂CH₃ | CH | 226 | CH₂CH₂O(thf-2-yl) | CH |
| 227 | CH₂CH₂O(thf-3-yl) | CH | 228 | CH₂(thp-2-yl) | CH |
| 229 | CH₂(thp-3-yl) | CH | 230 | CH₂(thp-4-yl) | CH |
| 231 | CH₂-CH(OMe)₂ | CH | 232 | CH₂-CH(OEt)₂ | CH |
| 233 | CH₂(dioxolan-2-yl) | CH | 234 | CH₂(1,3-dioxan-2-yl) | CH |
| 235 | CH₂(3-methyl-isoxazolin-5-yl | CH | 236 | CH₂(3-methyl-isoxazol-5-yl | CH |
| 237 | isoxazolin-4-yl | CH | 238 | Thiazol-2-yl | CH |
| 239 | Thiazol-3-yl | CH | 240 | Oxazol-2-yl | CH |
| 241 | 5methylisoxazol-3-yl | CH | 242 | 4-methyloxazol-2-yl | CH |
| 243 | 4-methylthiazol-2-yl | CH | 244 | 5-chloropyridin-2-yl | CH |
| 245 | 5-methylpyridin-2-yl | CH | 246 | 5-methoxypyridin-2-yl | CH |
| 247 | 3-chloropyridin-4-yl | CH | 248 | 3-methylpyridin-4-yl | CH |
| 249 | 3-methoxypyridin-4-yl | CH | 250 | CH₂CH₂CH=CH₂ | CH |
| 251 | CHSOCH₃ | CH | 252 | CH₂SO₂CH₃ | CH |

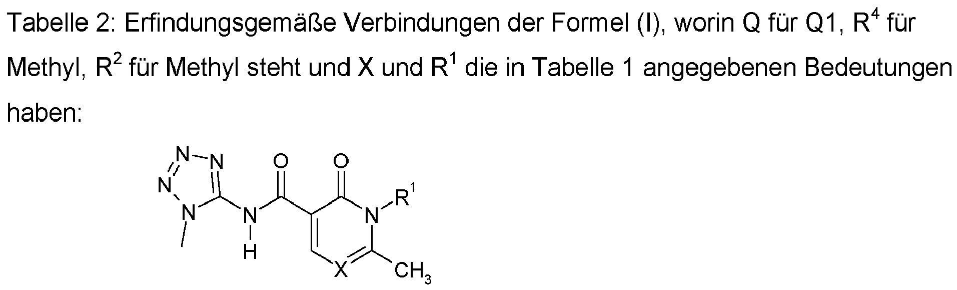

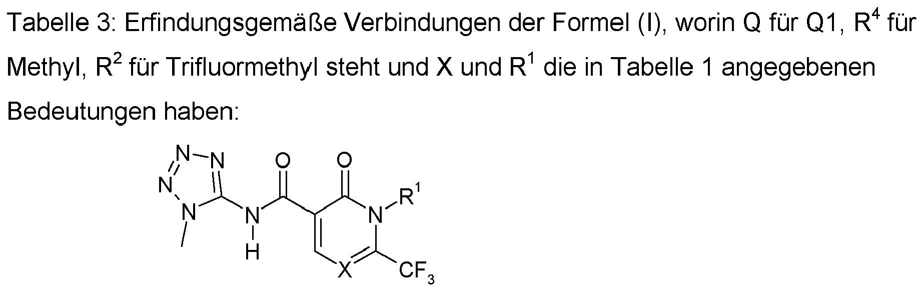

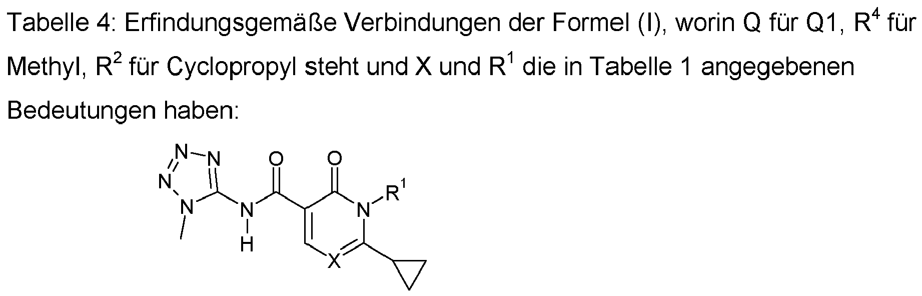

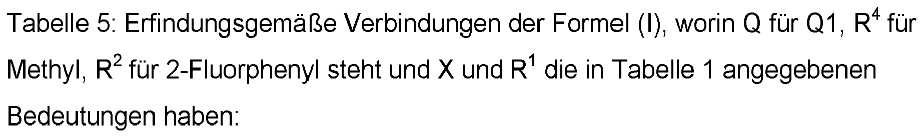

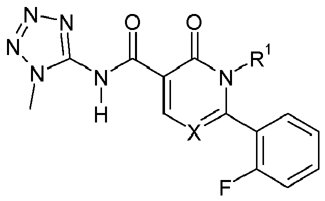

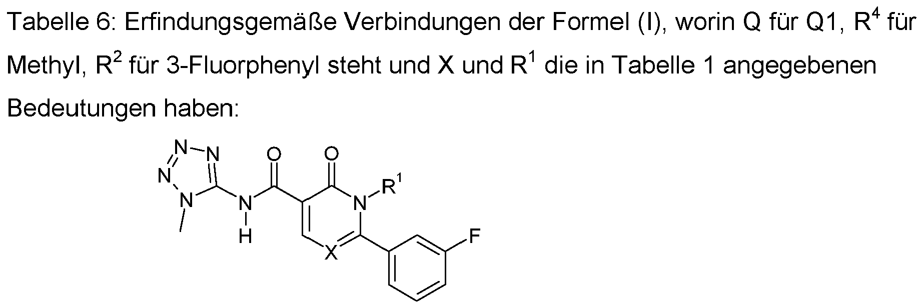

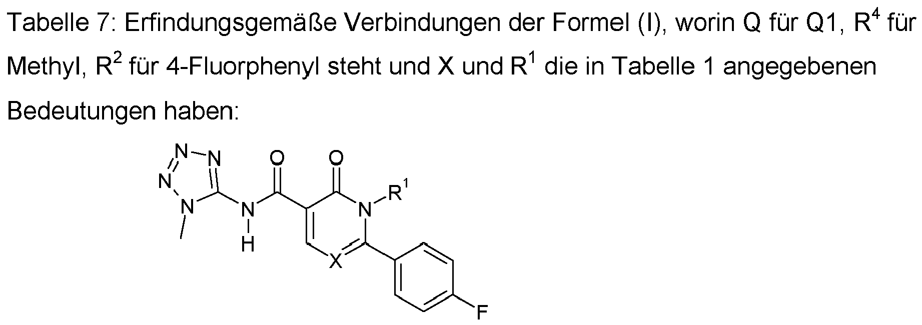

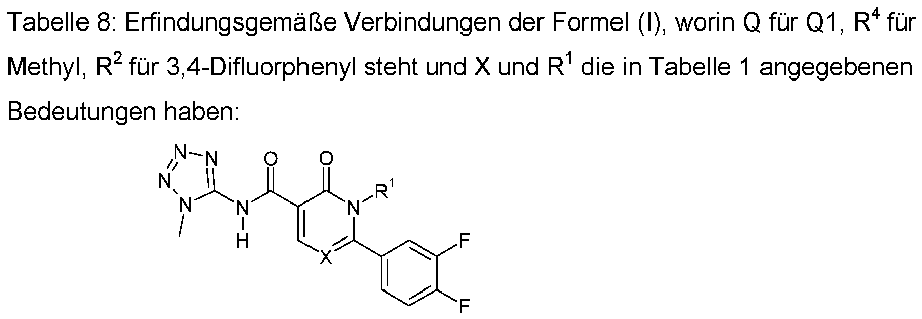

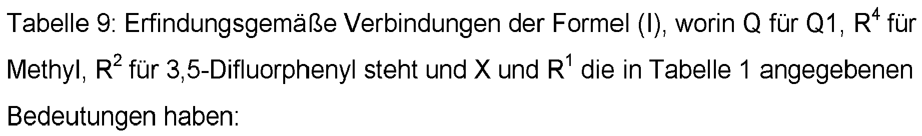

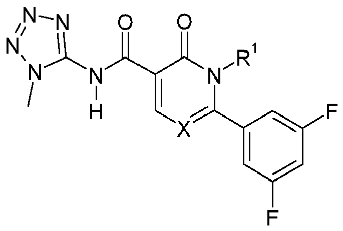

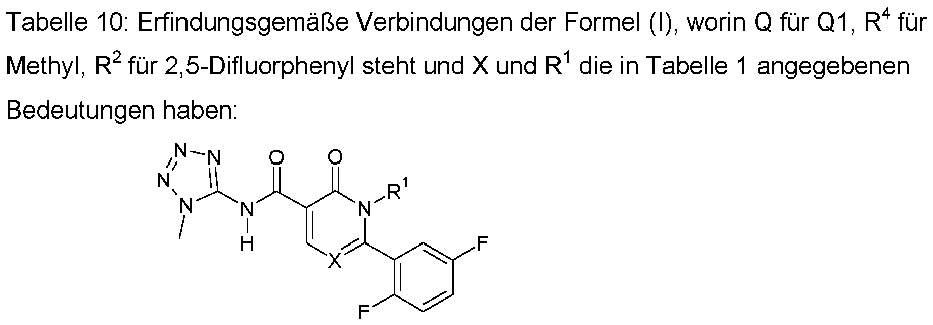

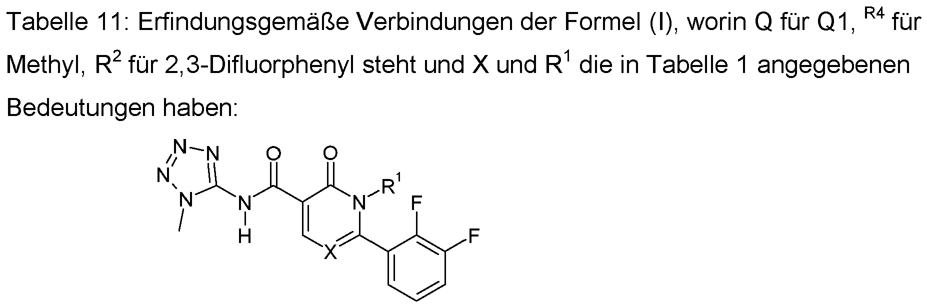

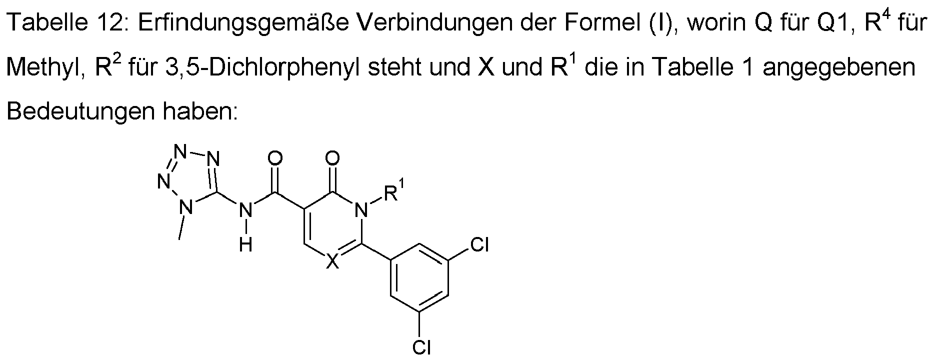

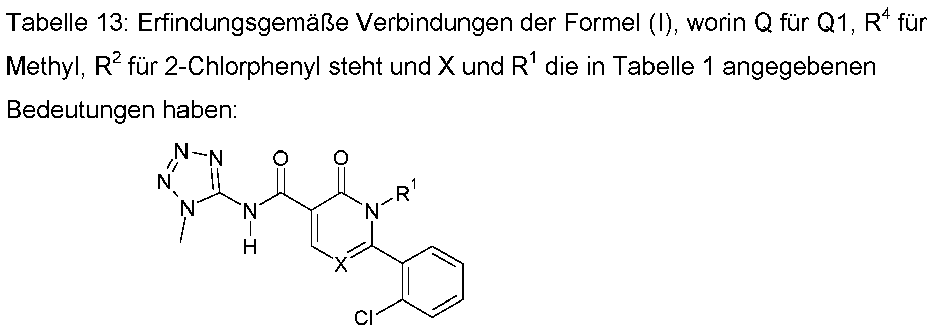

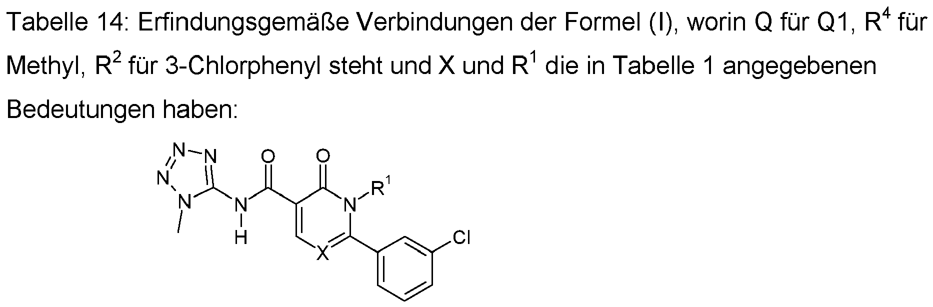

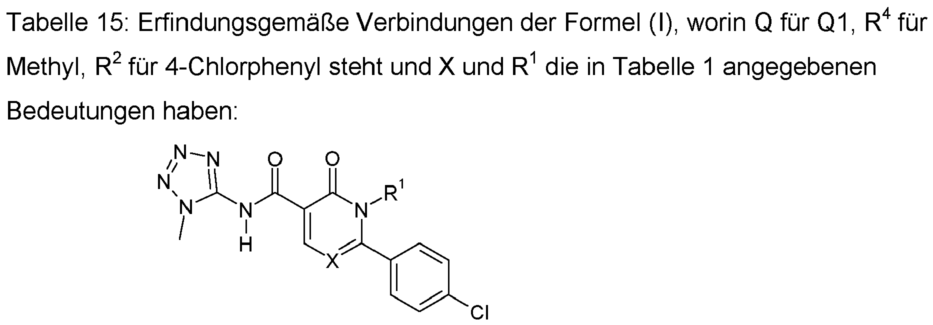

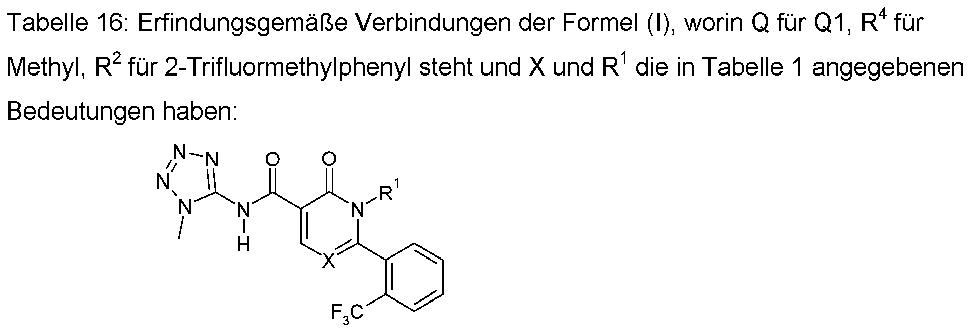

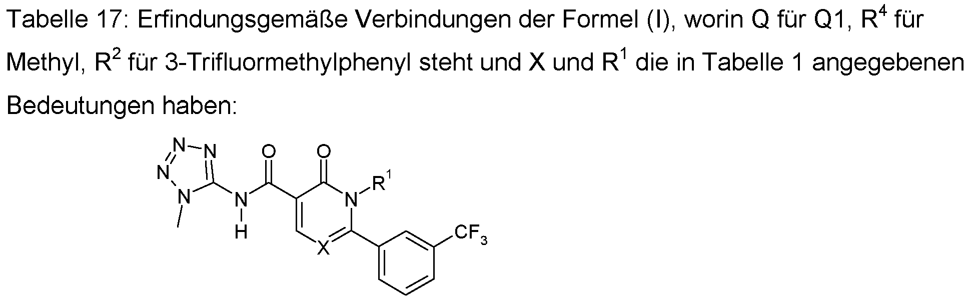

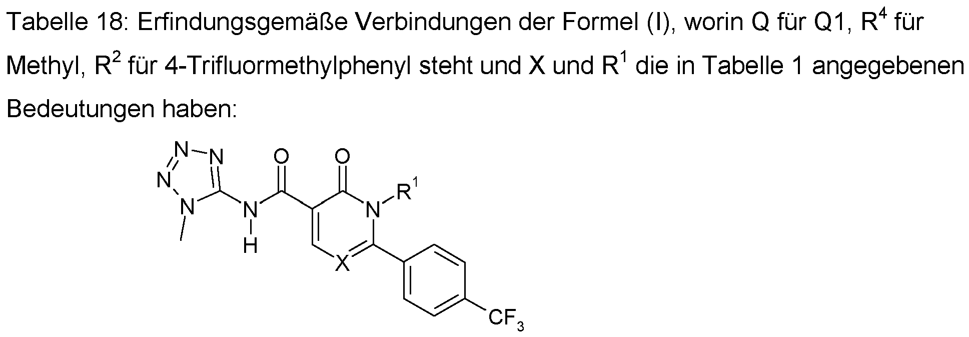

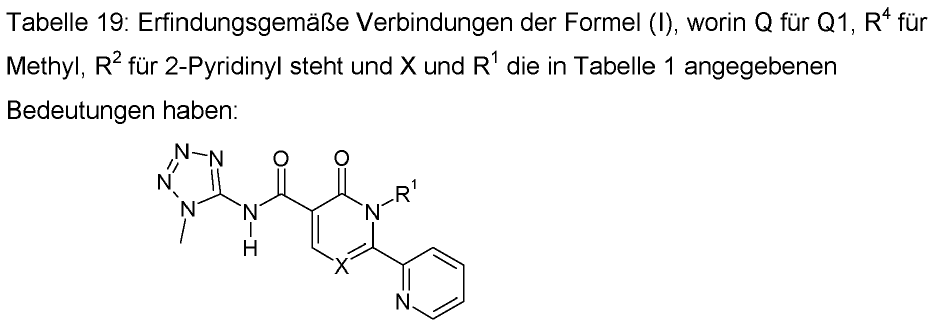

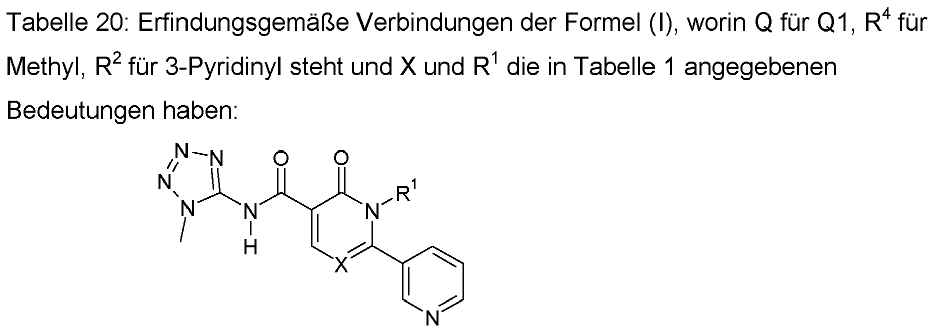

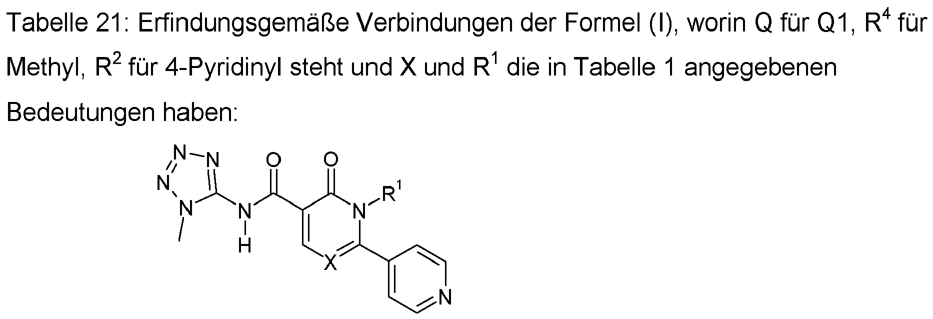

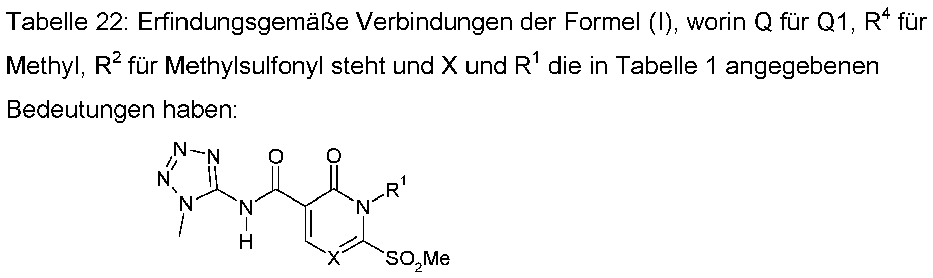

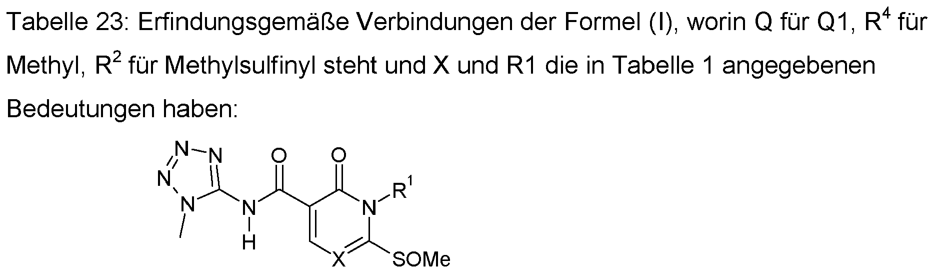

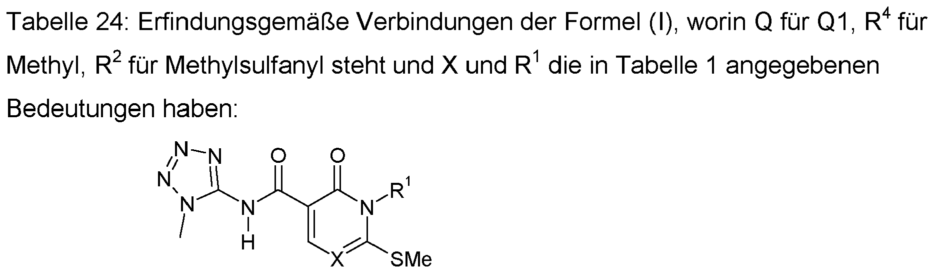

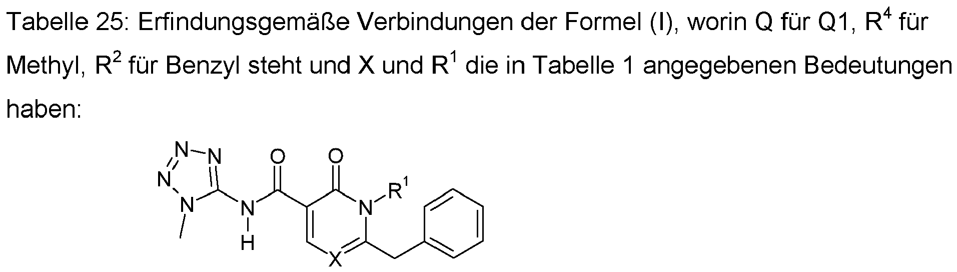

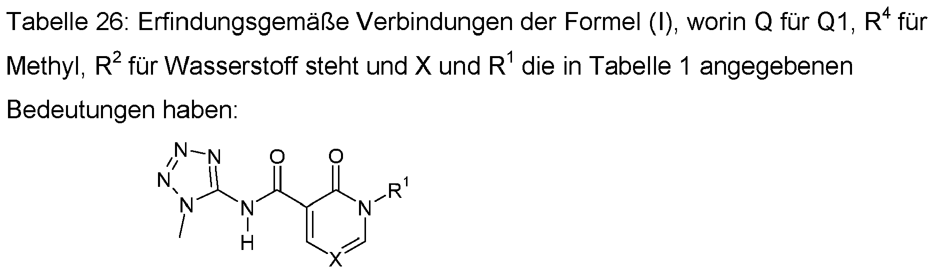

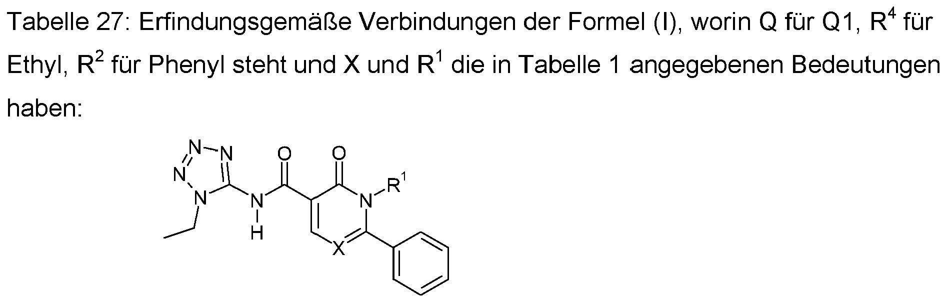

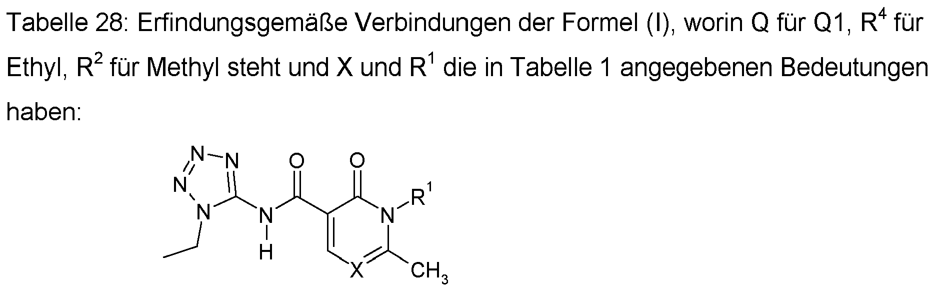

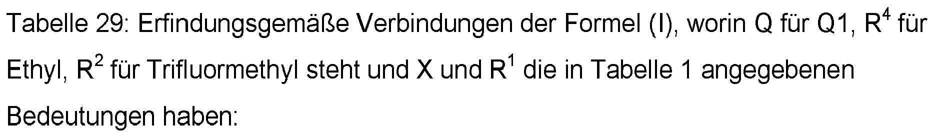

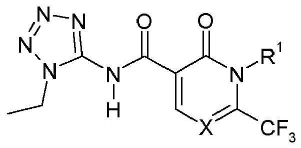

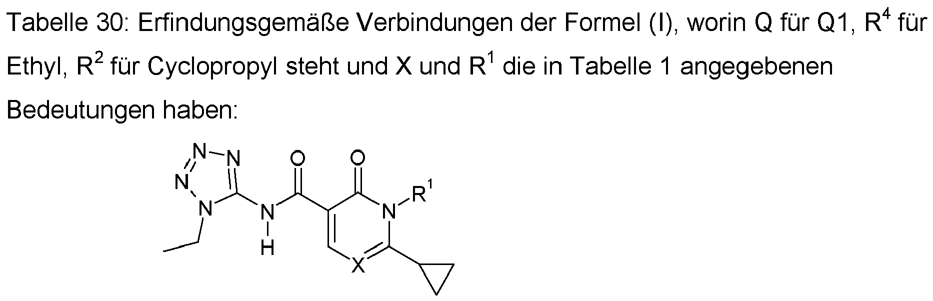

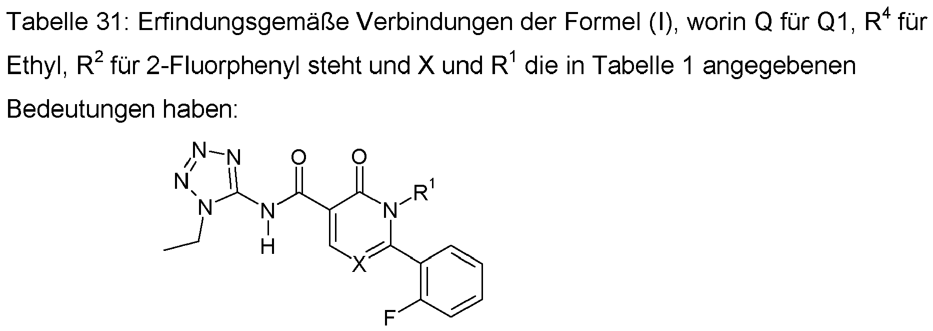

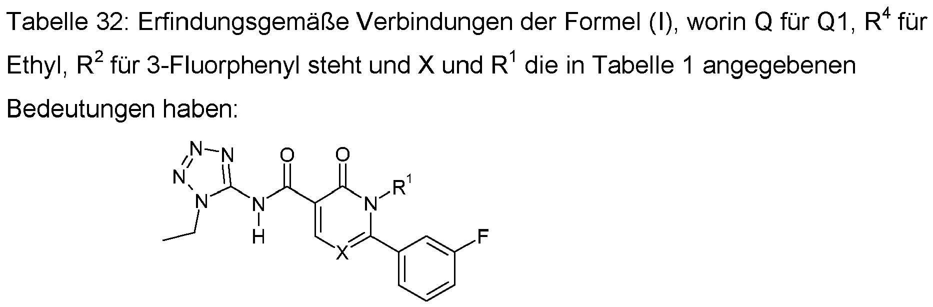

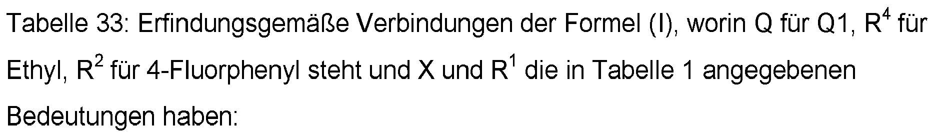

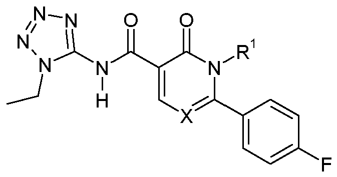

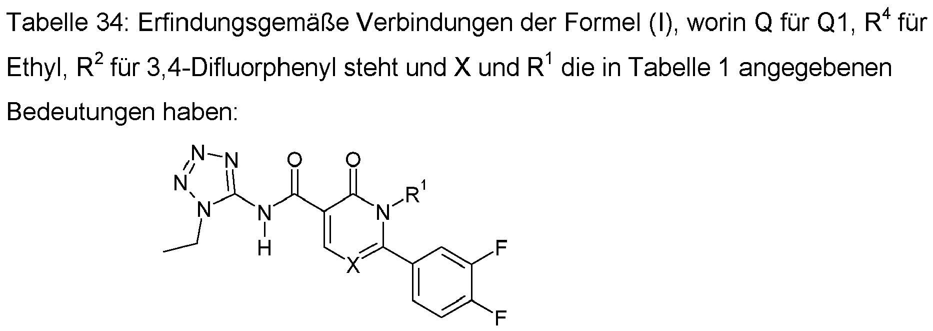

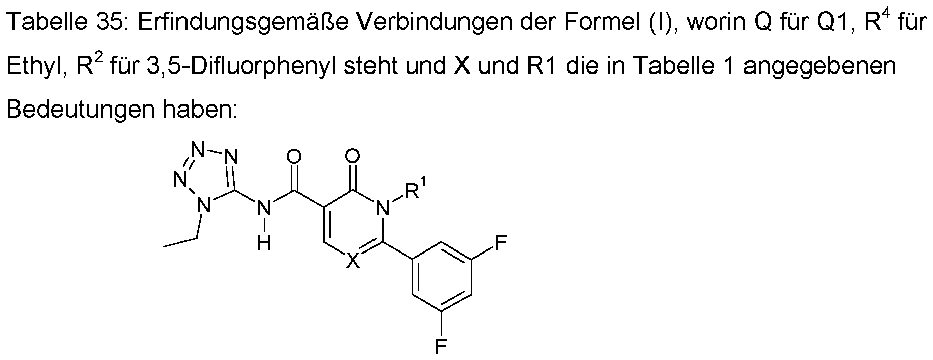

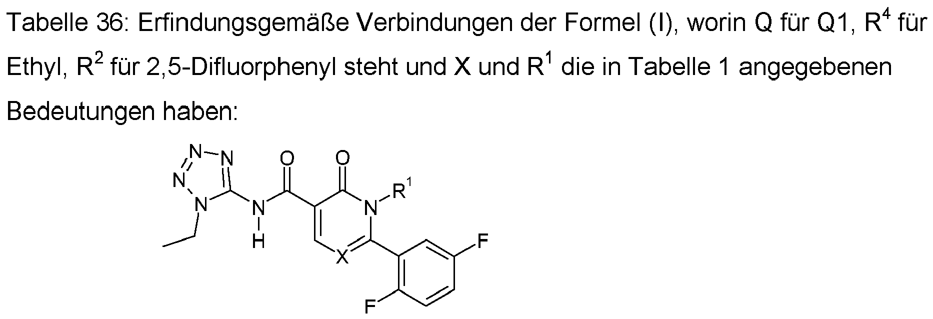

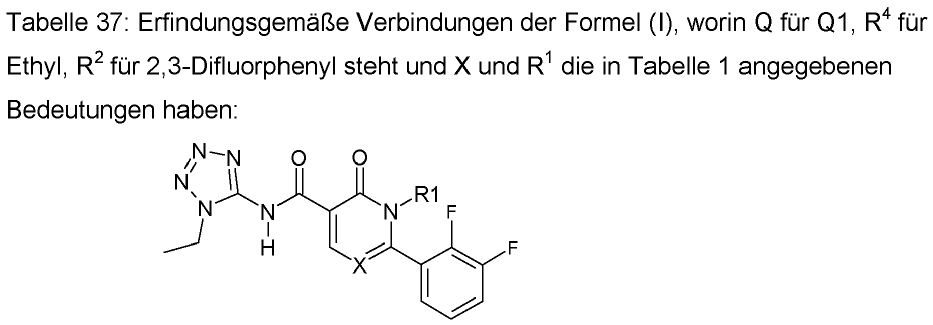

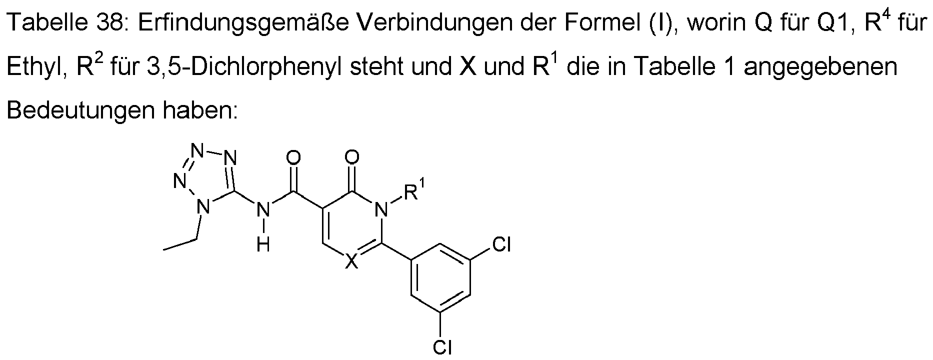

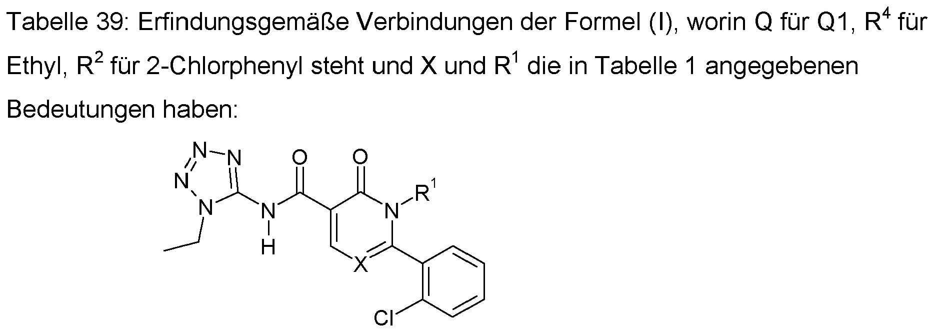

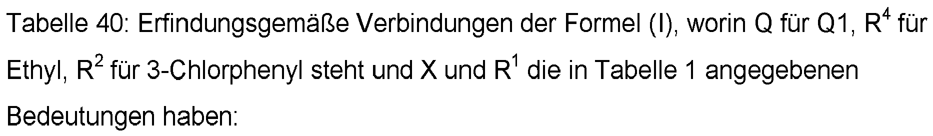

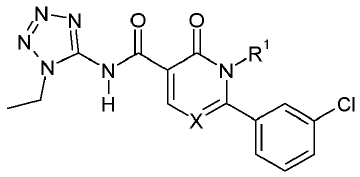

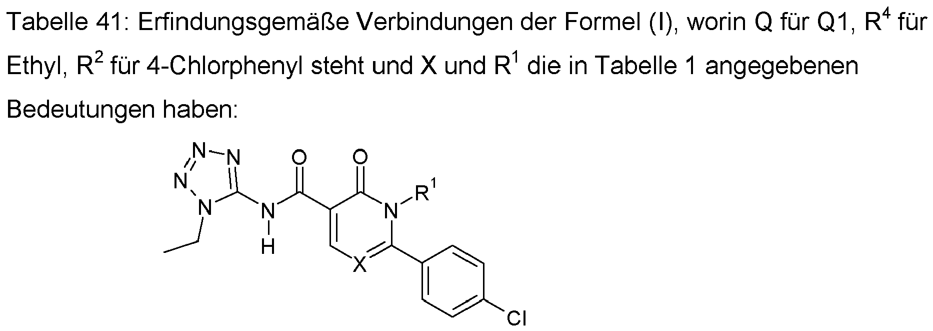

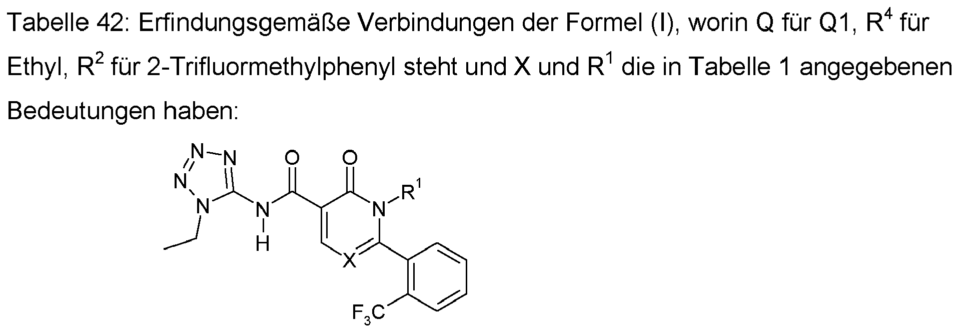

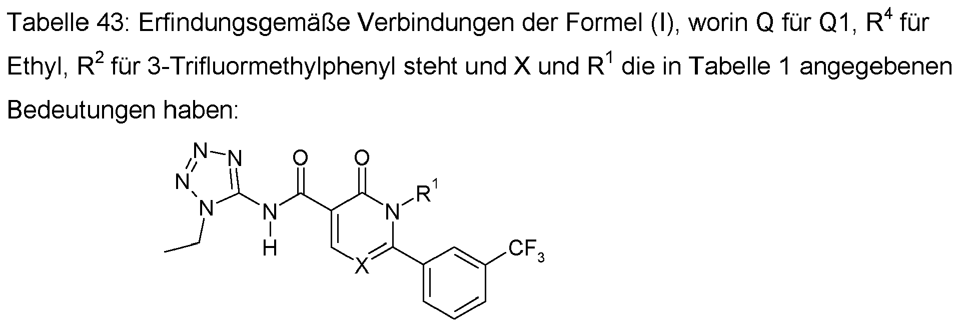

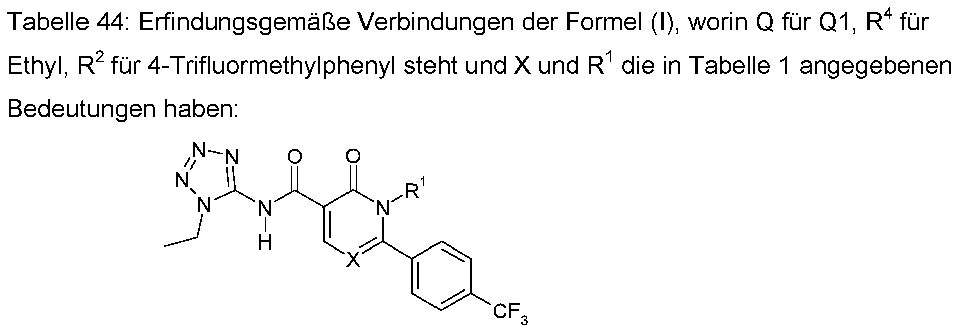

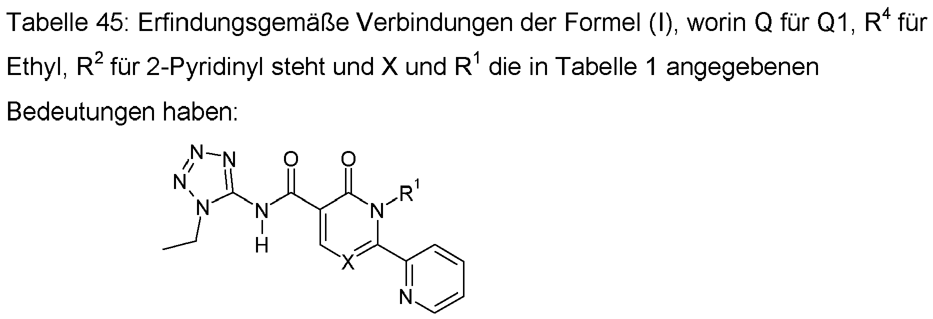

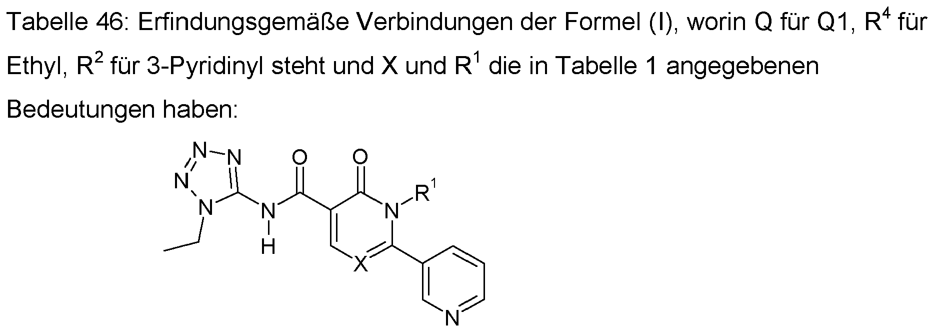

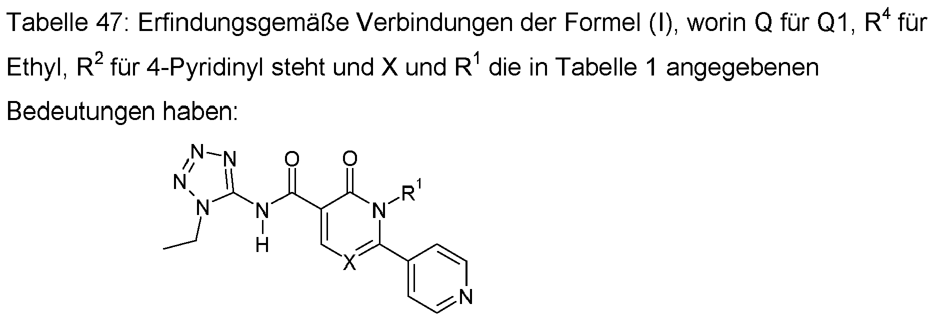

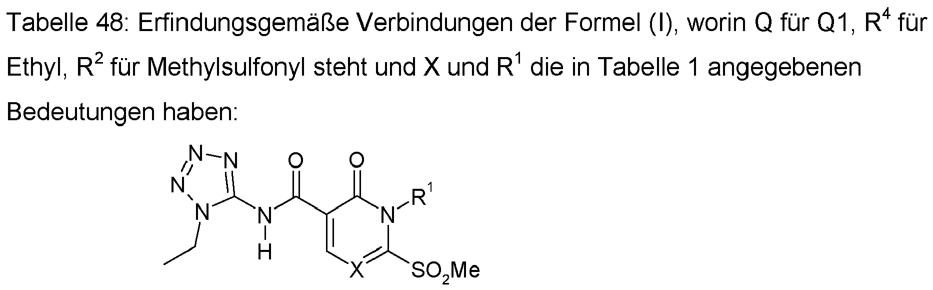

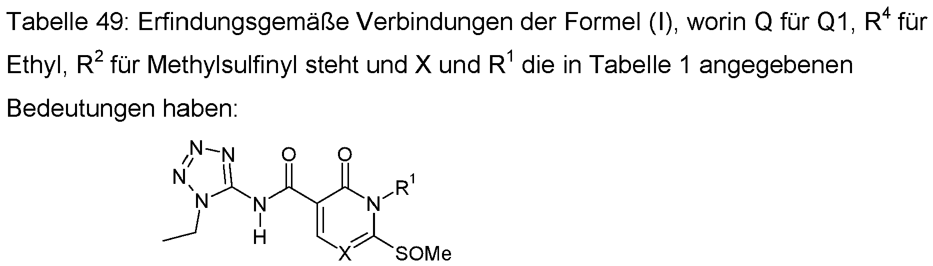

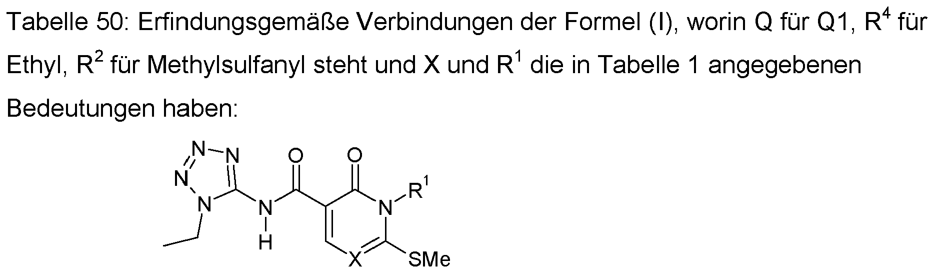

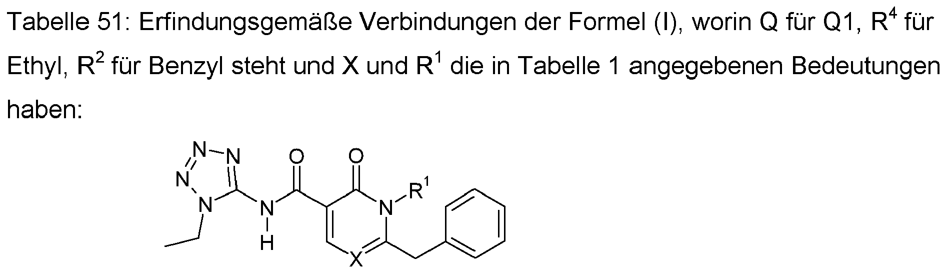

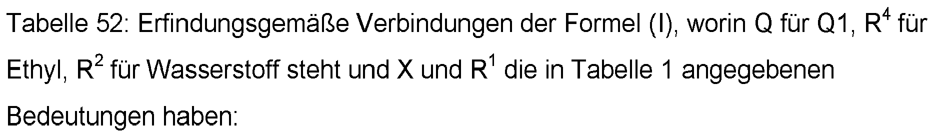

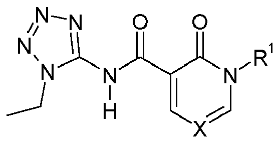

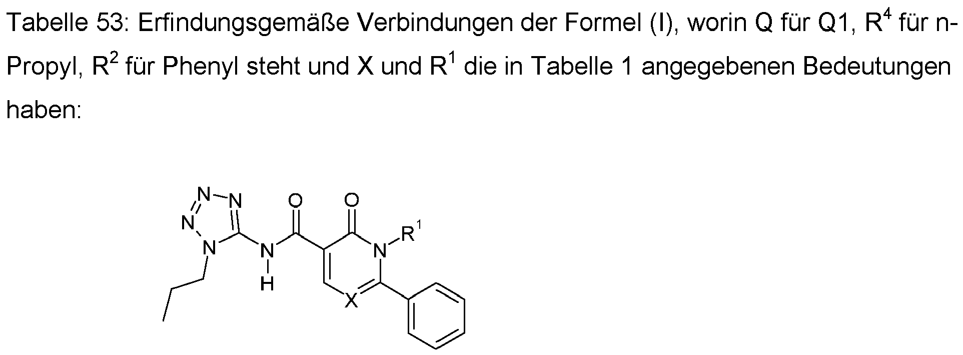

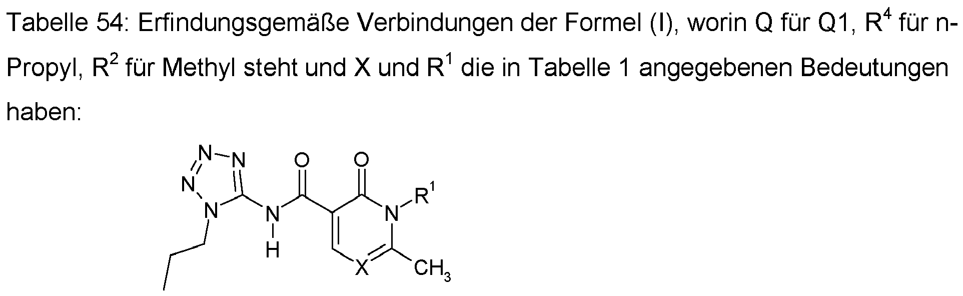

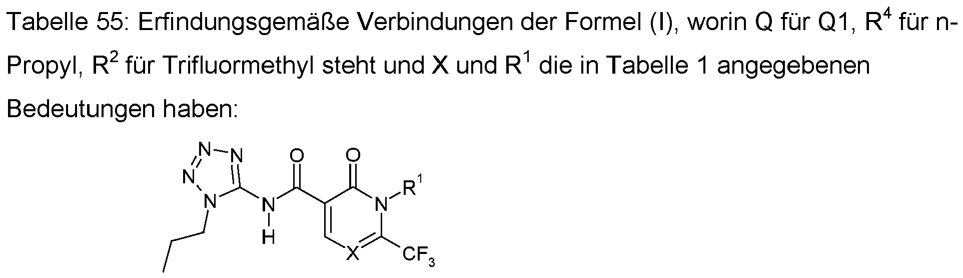

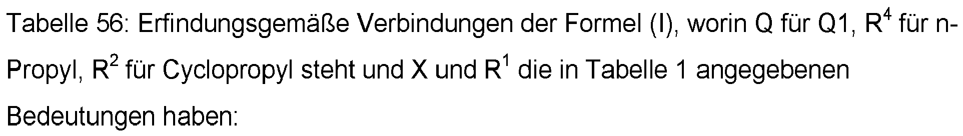

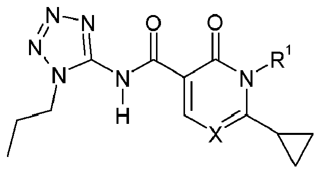

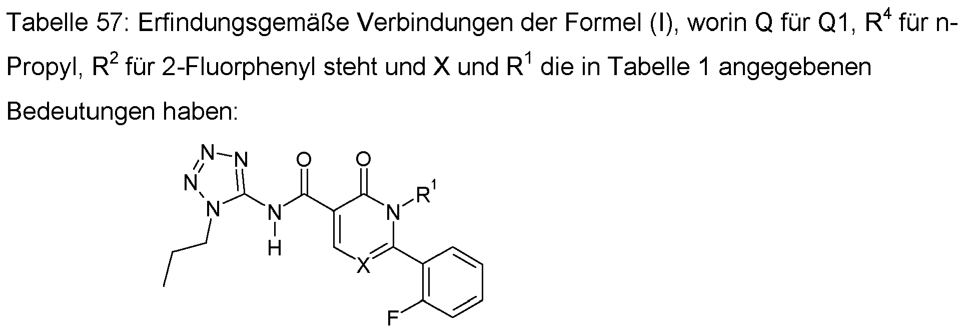

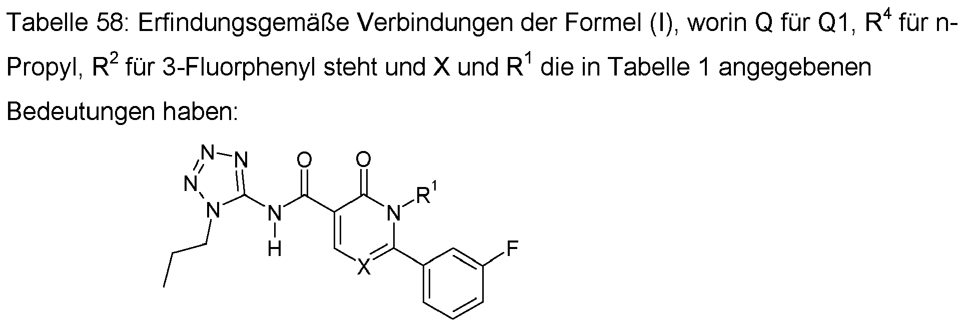

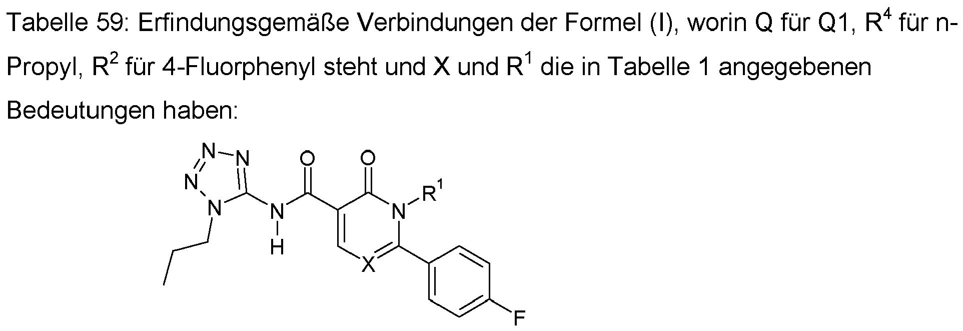

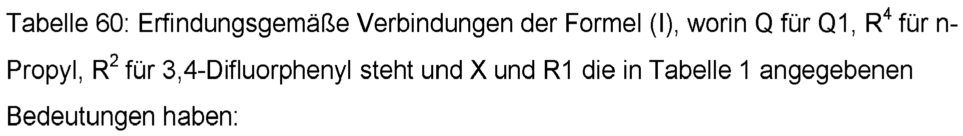

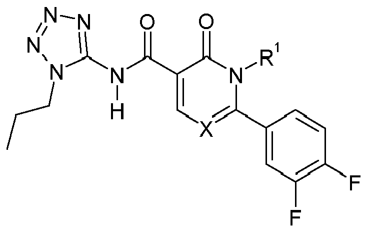

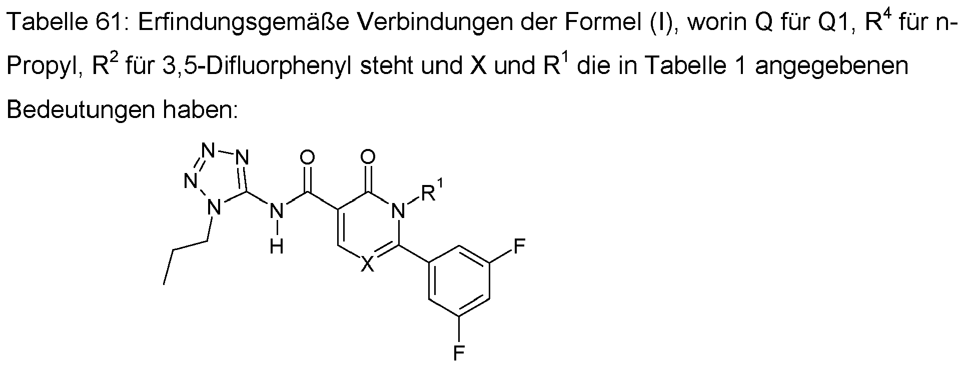

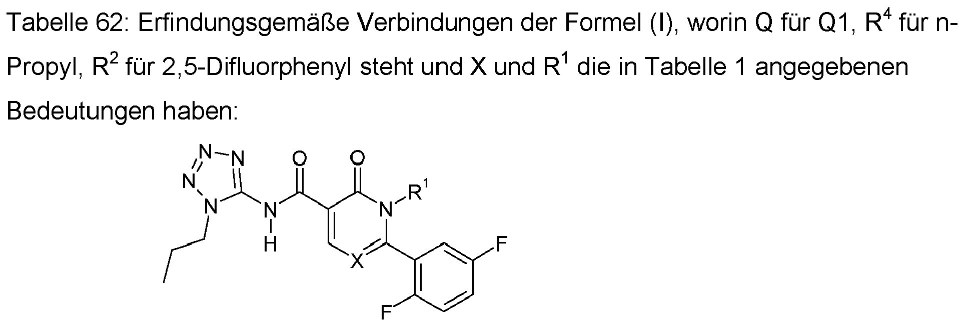

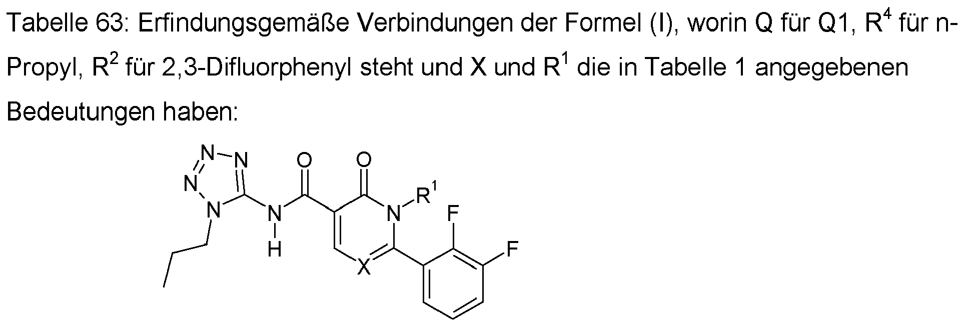

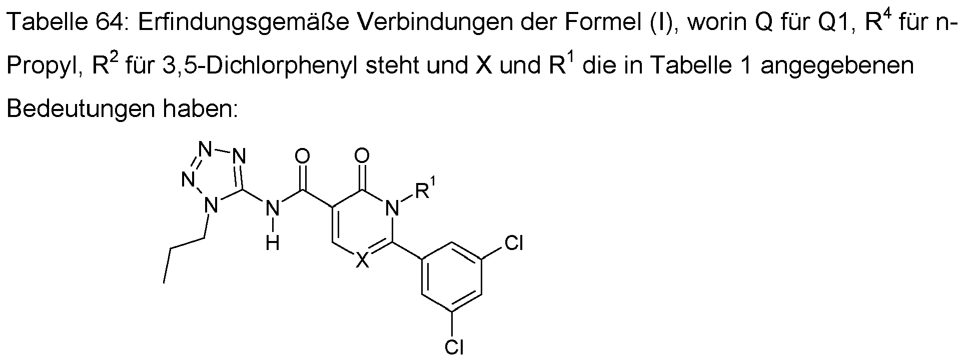

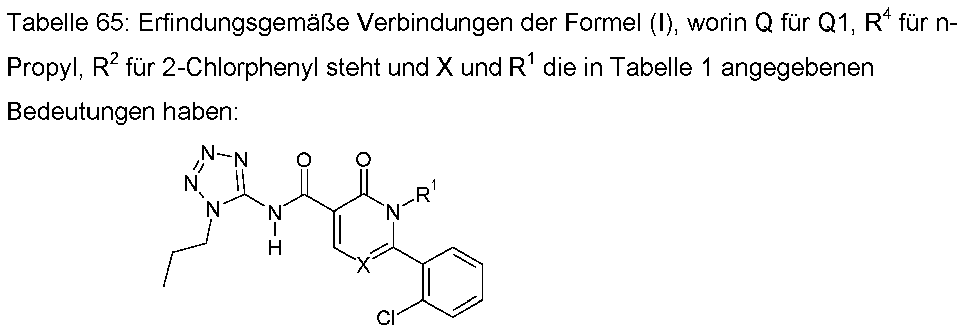

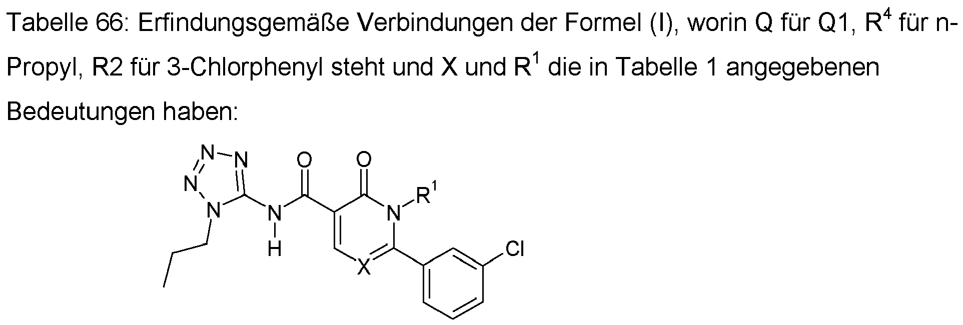

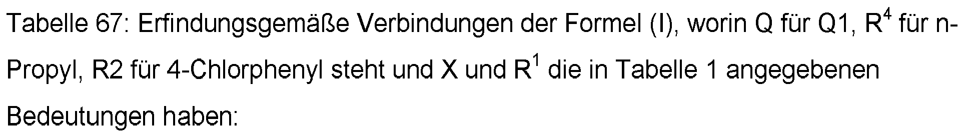

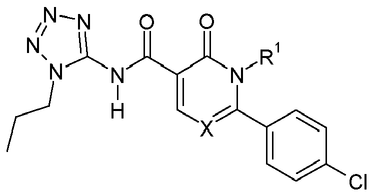

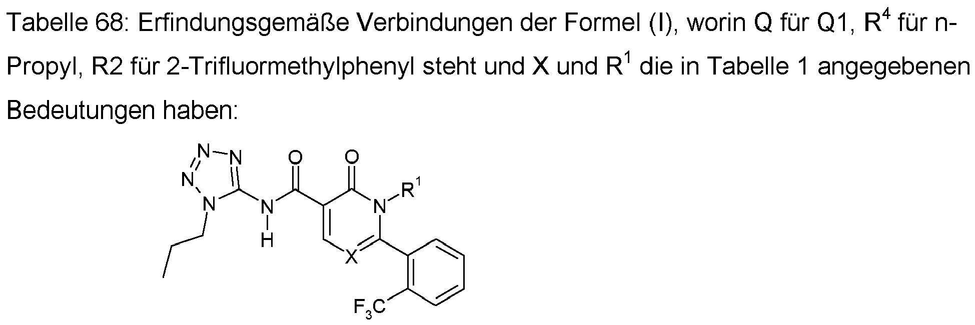

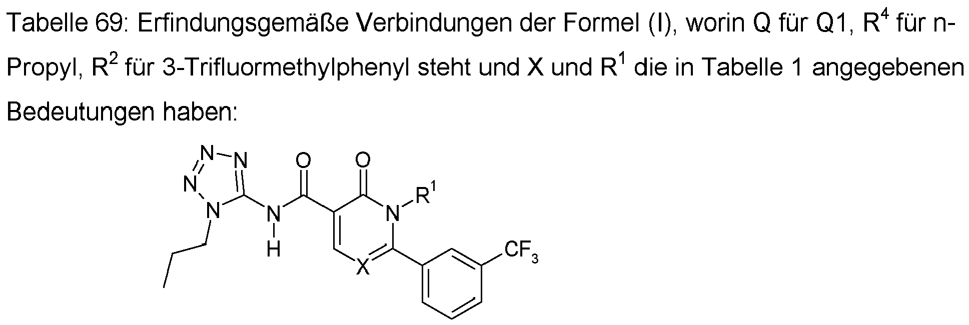

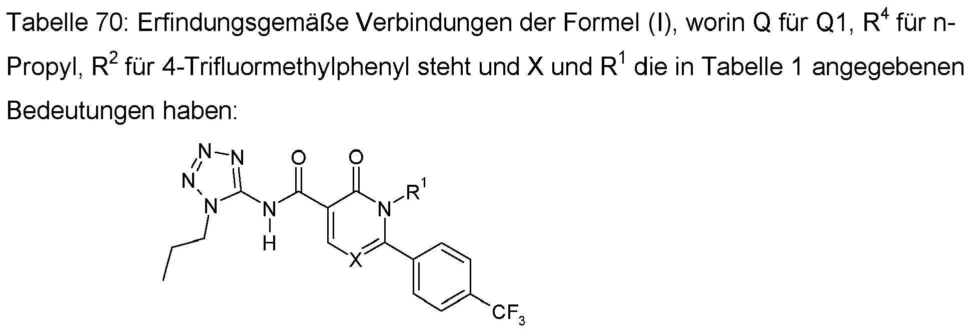

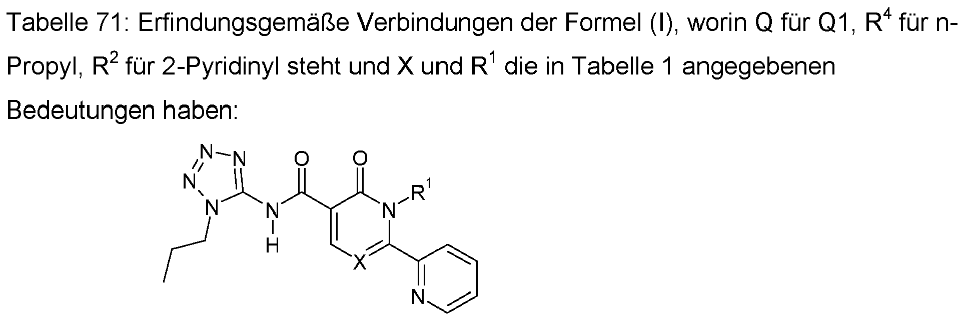

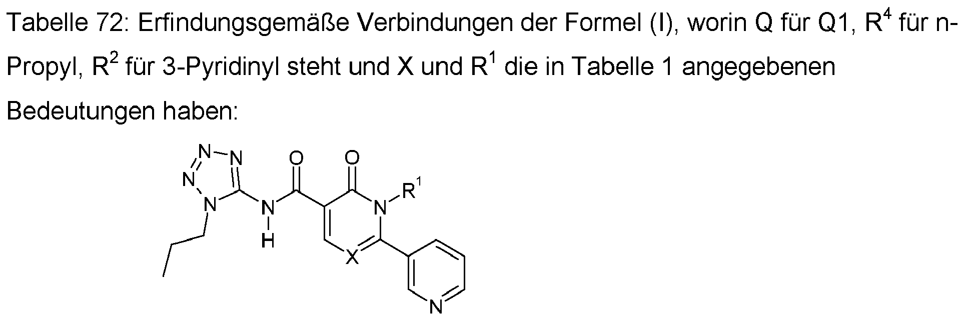

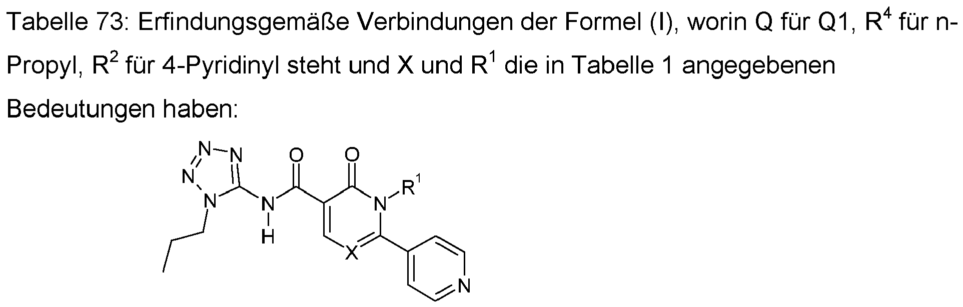

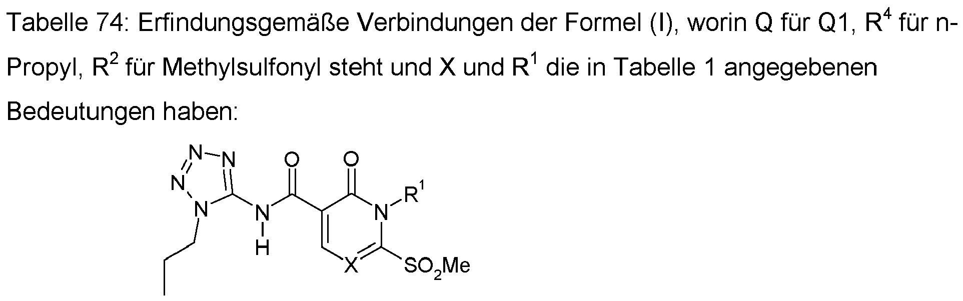

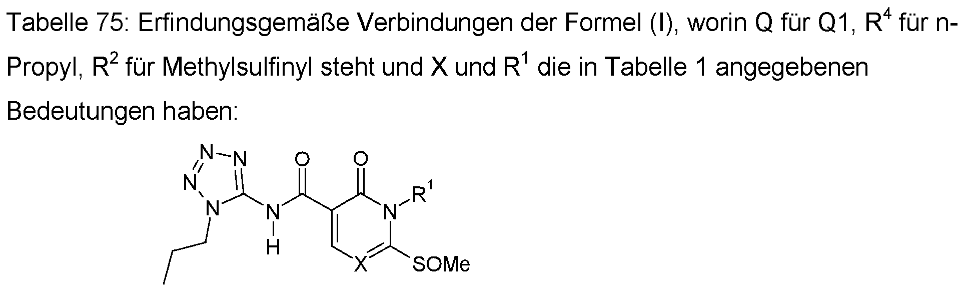

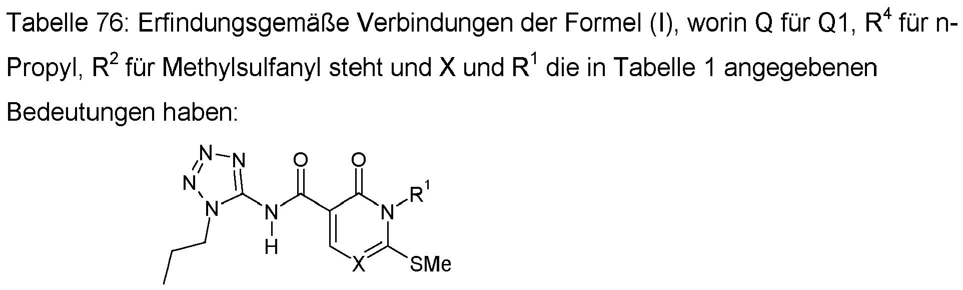

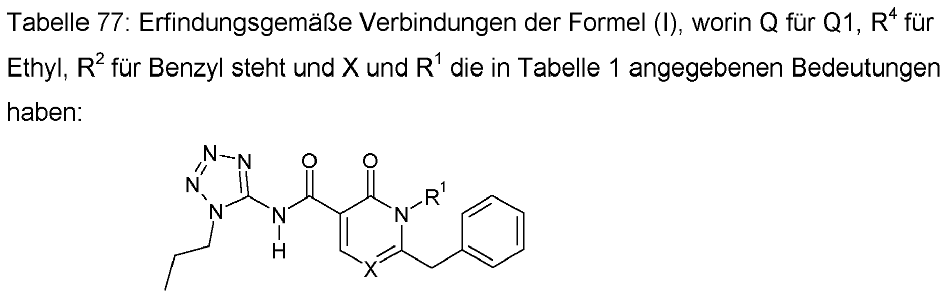

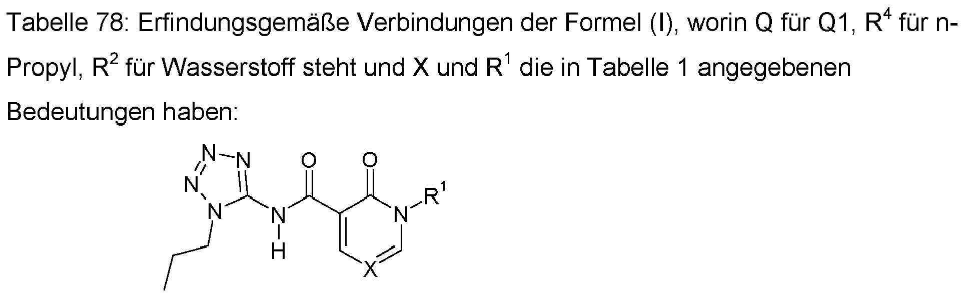

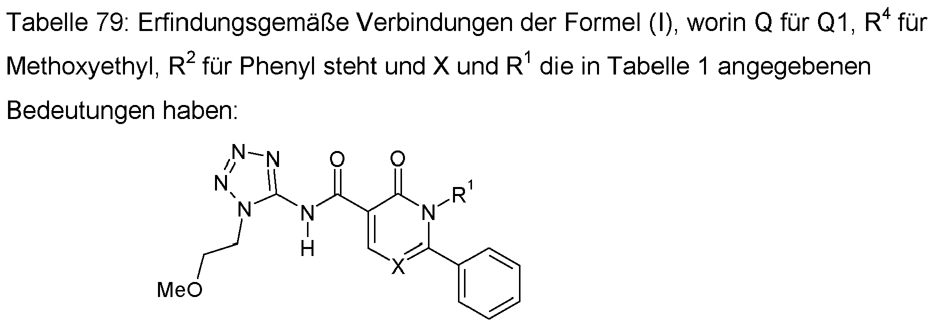

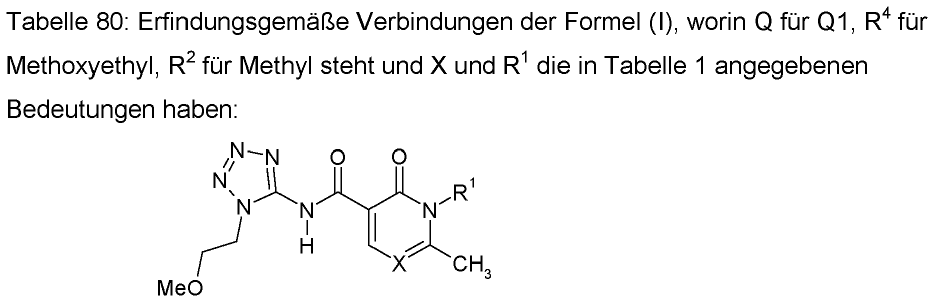

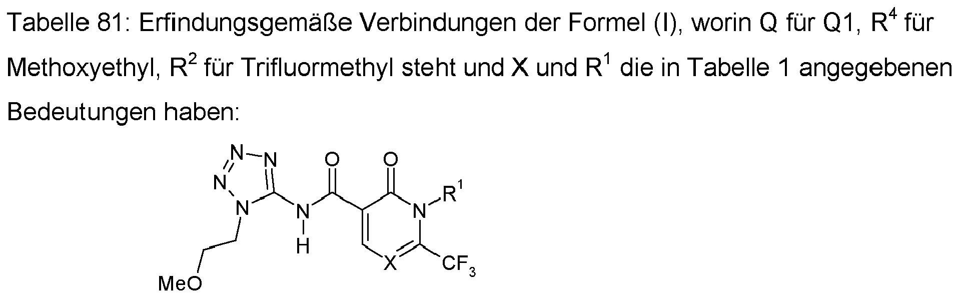

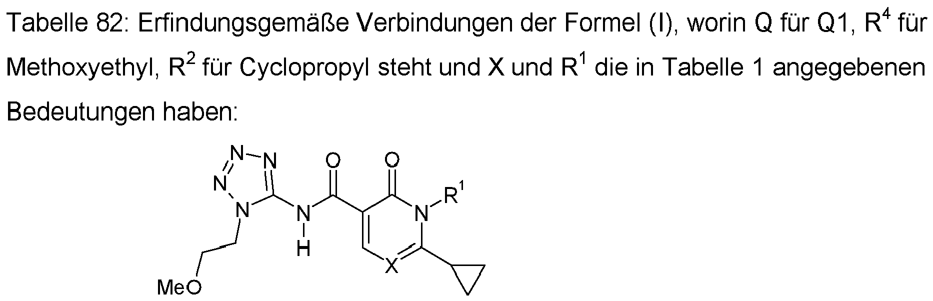

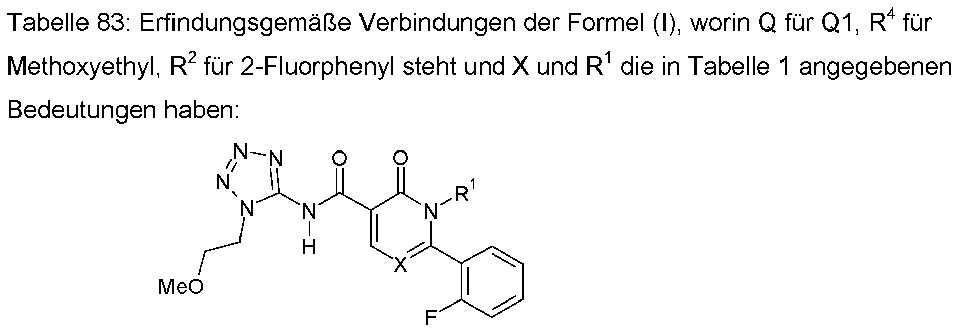

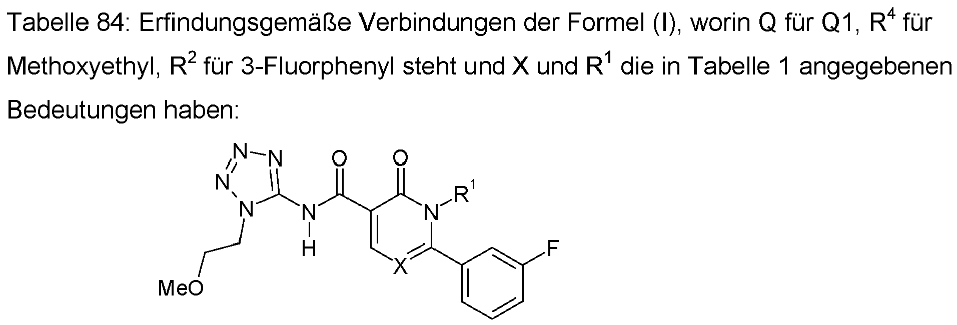

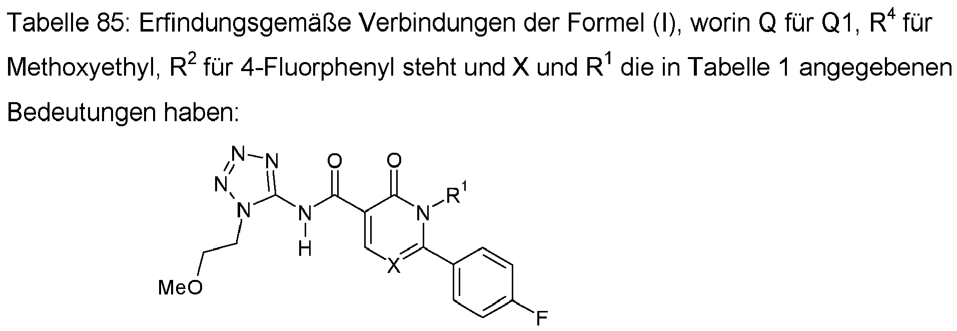

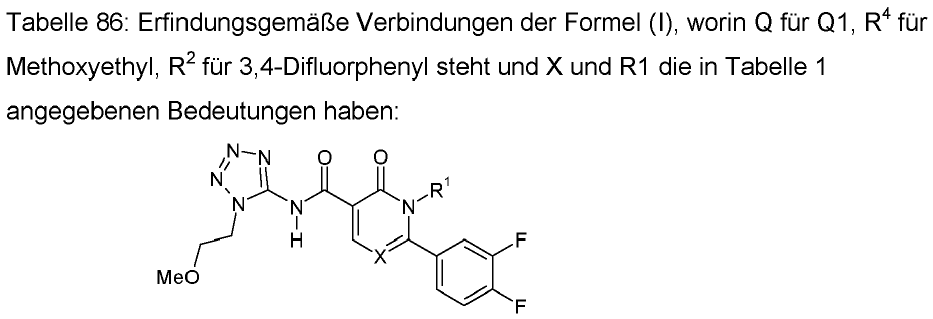

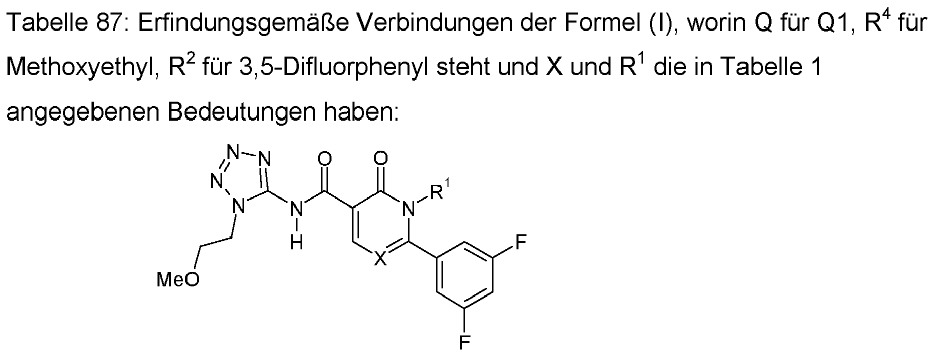

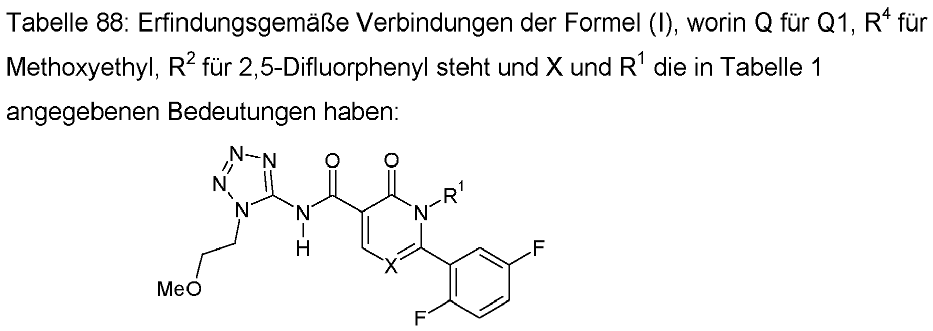

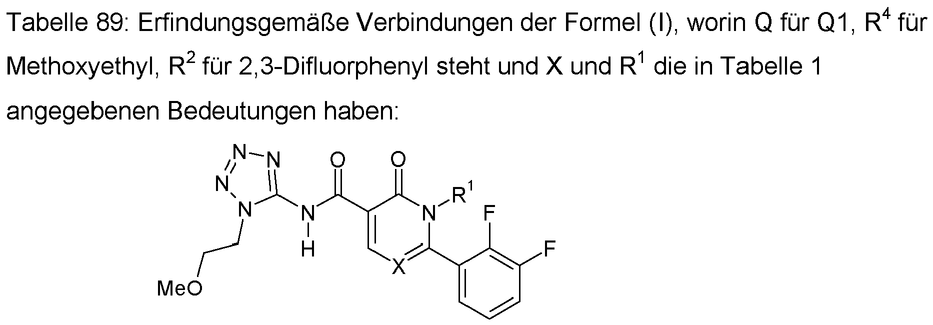

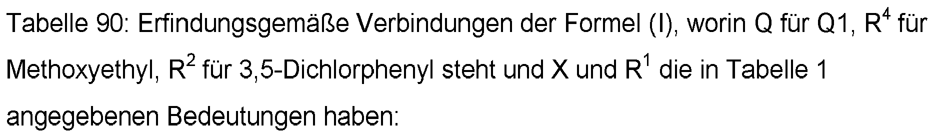

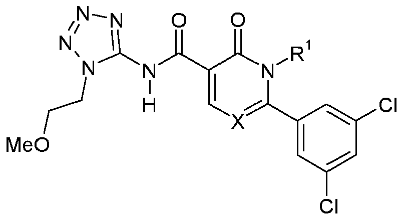

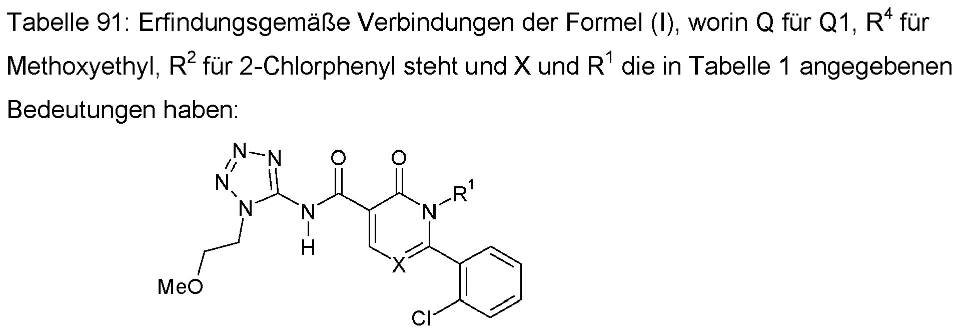

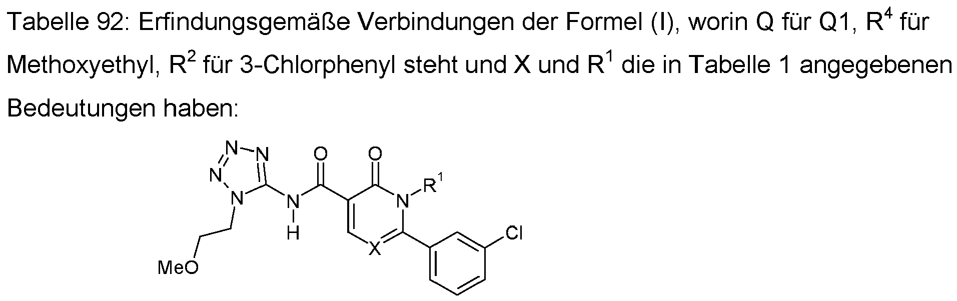

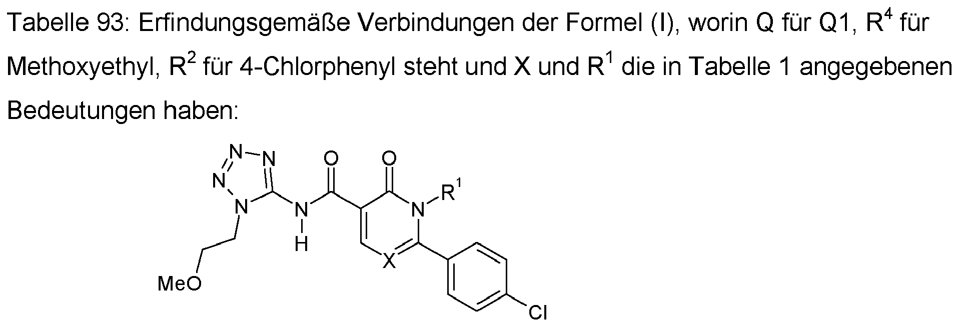

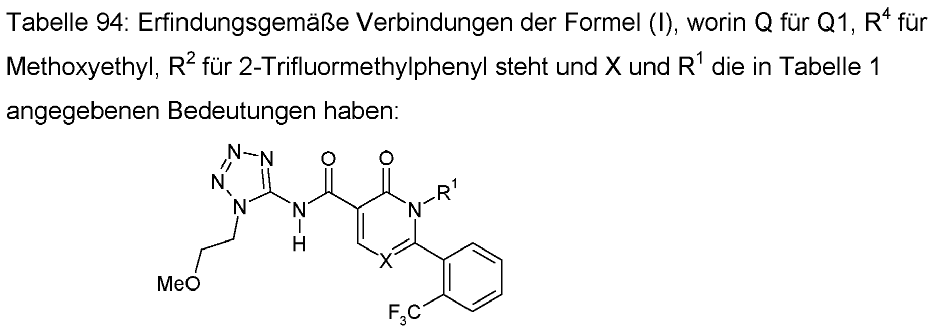

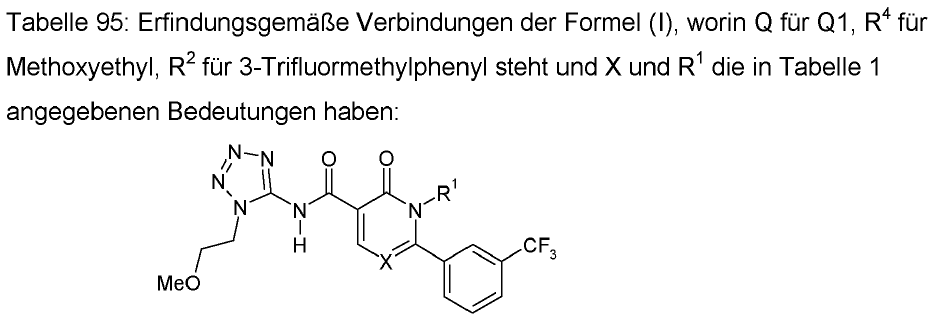

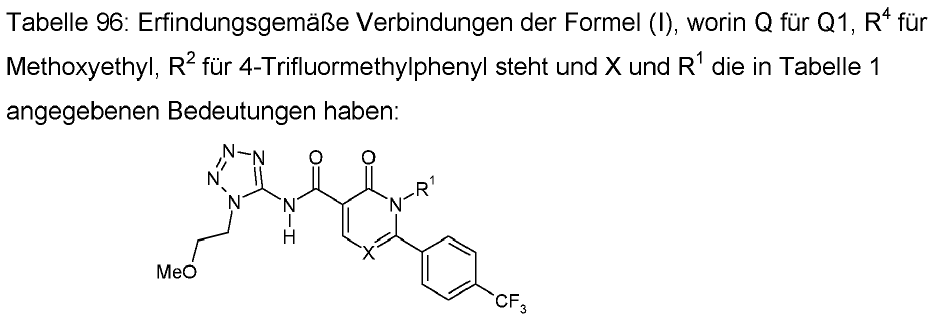

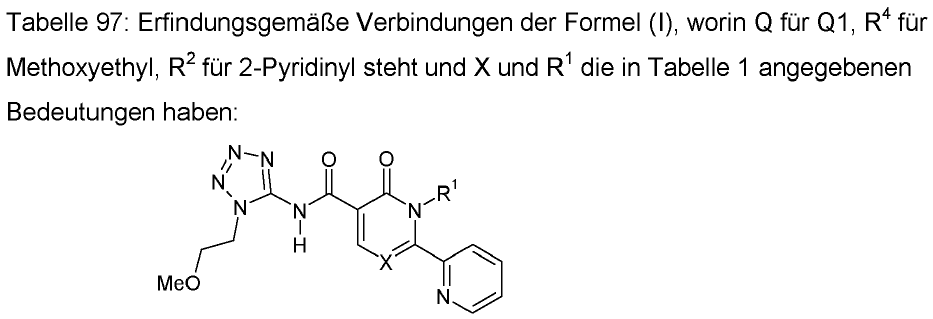

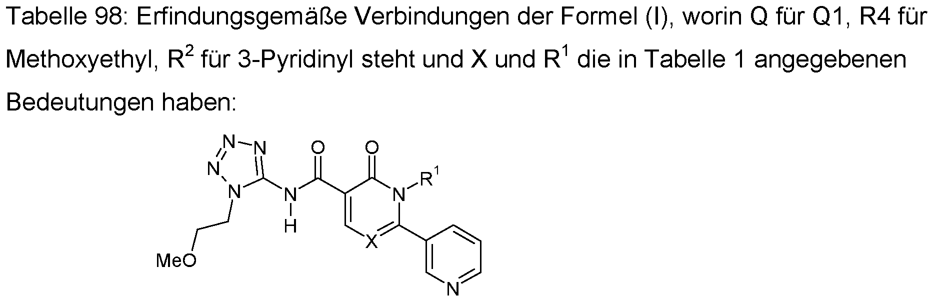

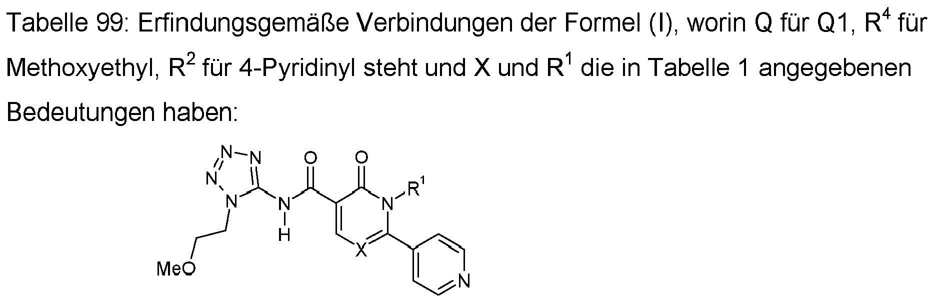

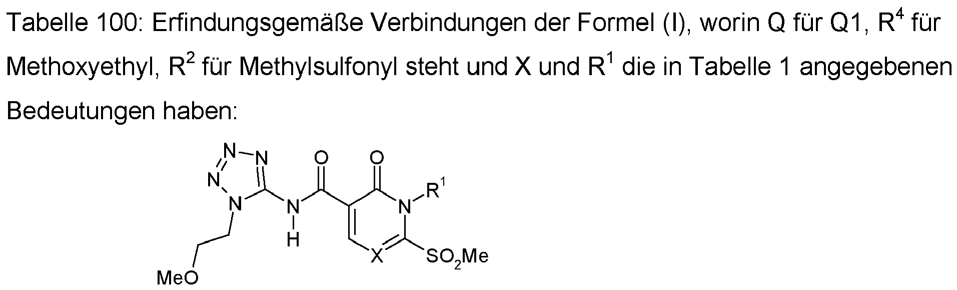

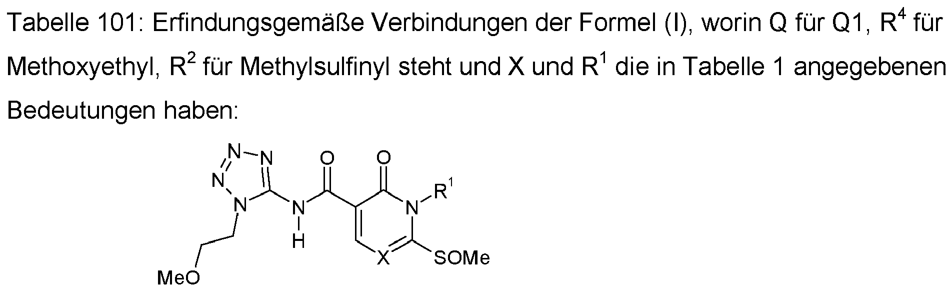

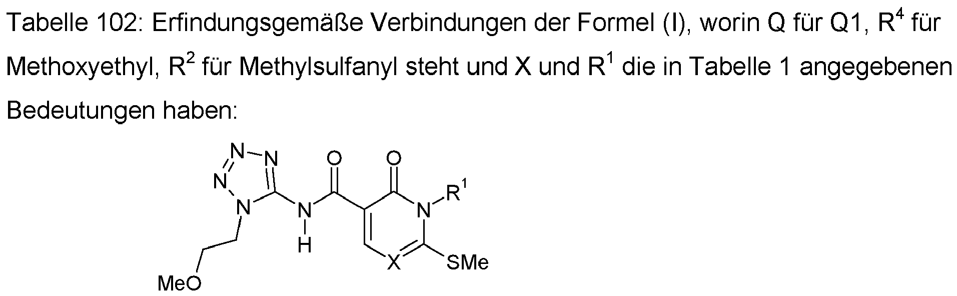

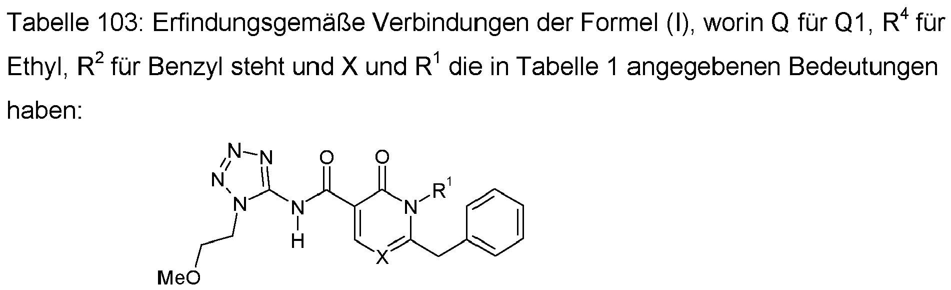

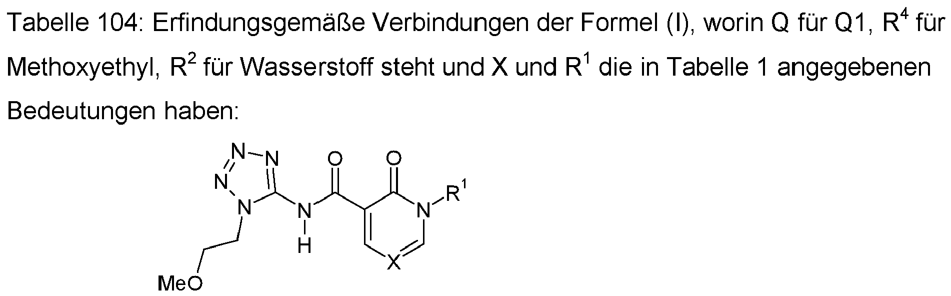

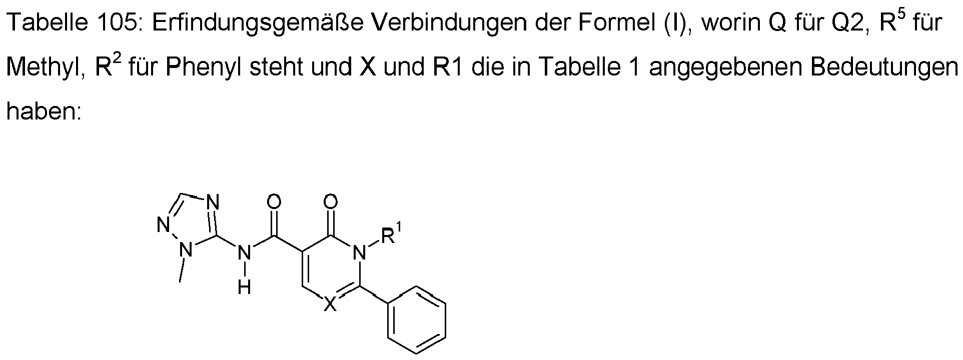

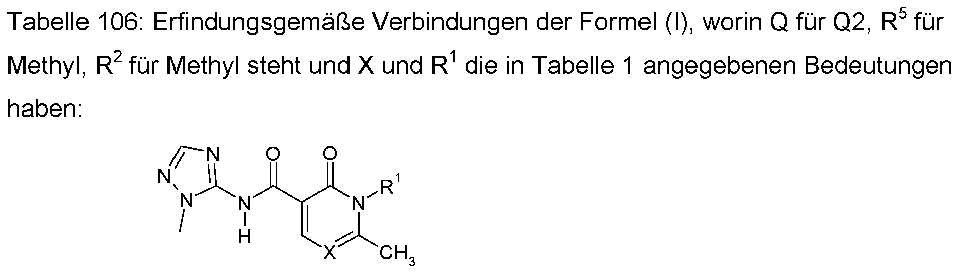

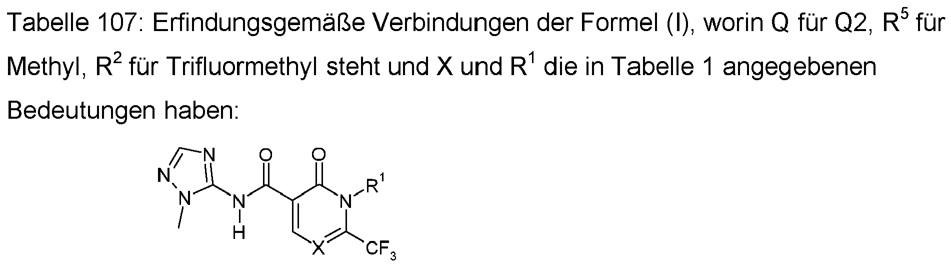

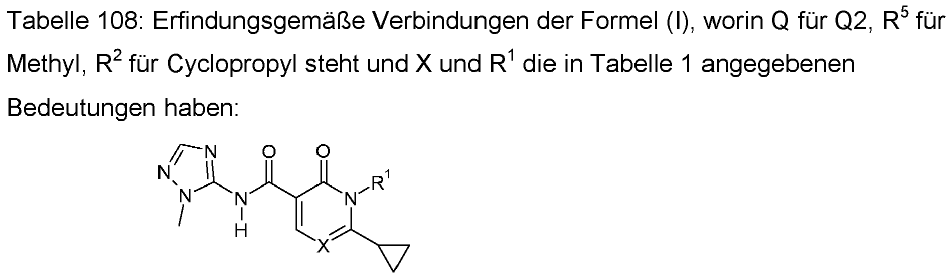

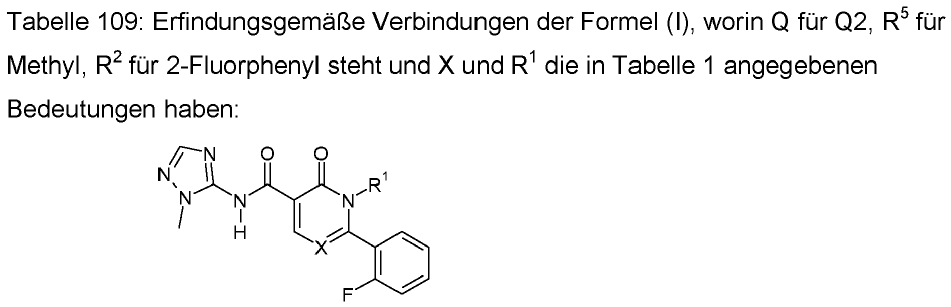

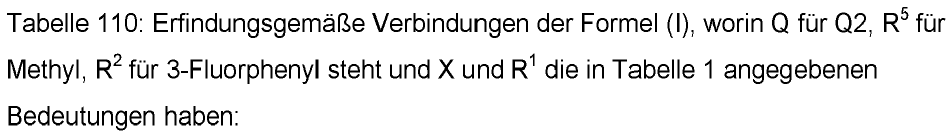

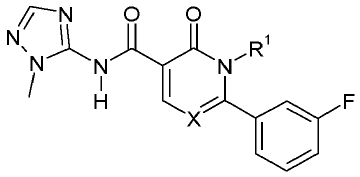

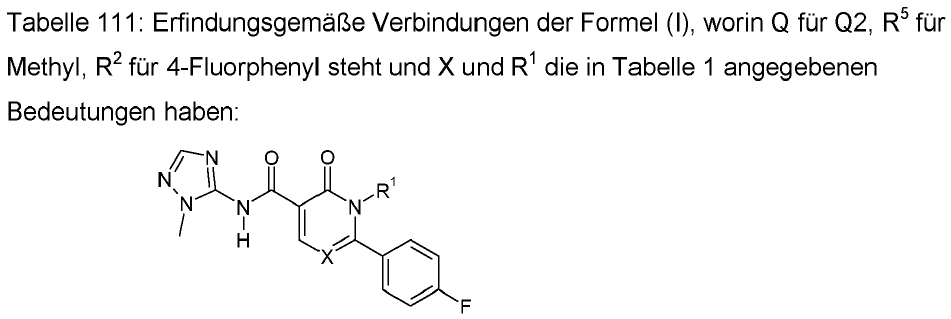

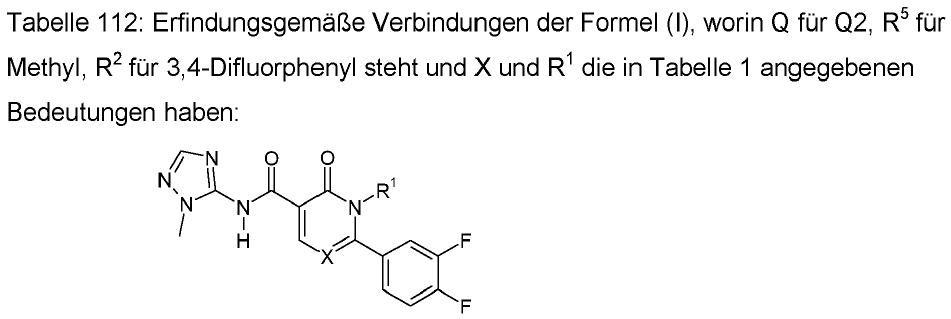

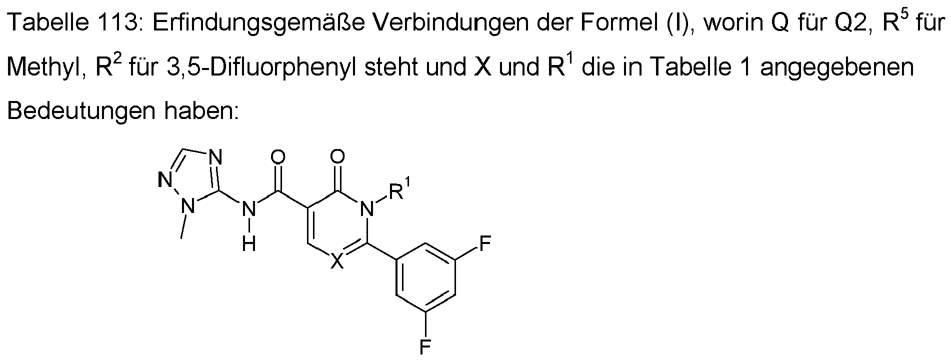

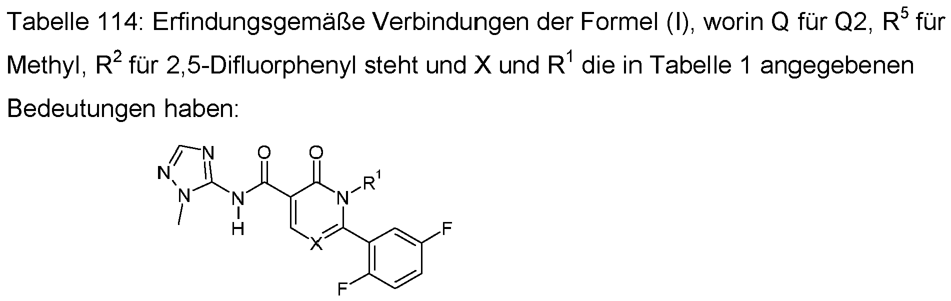

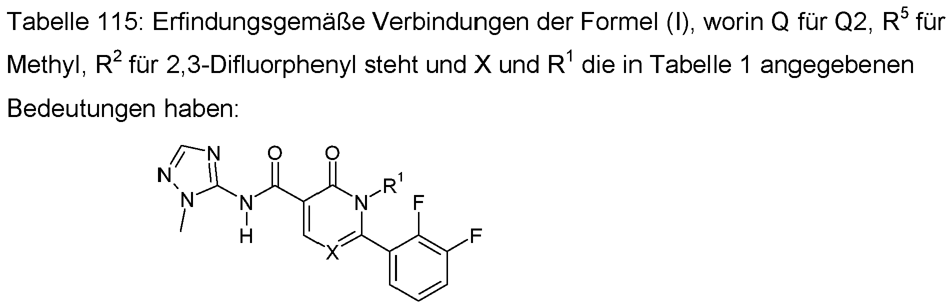

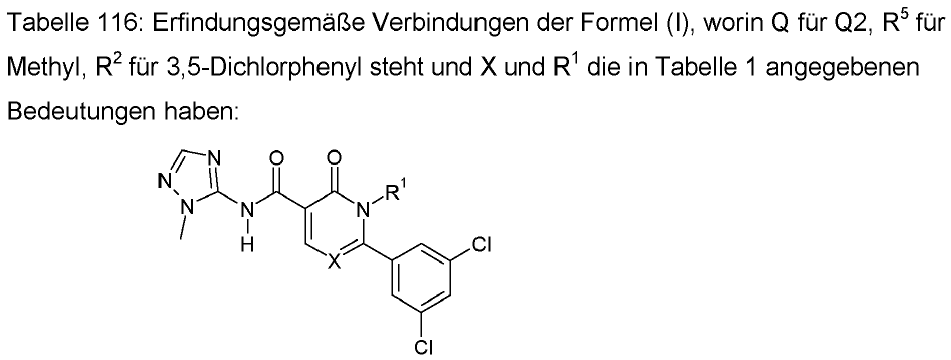

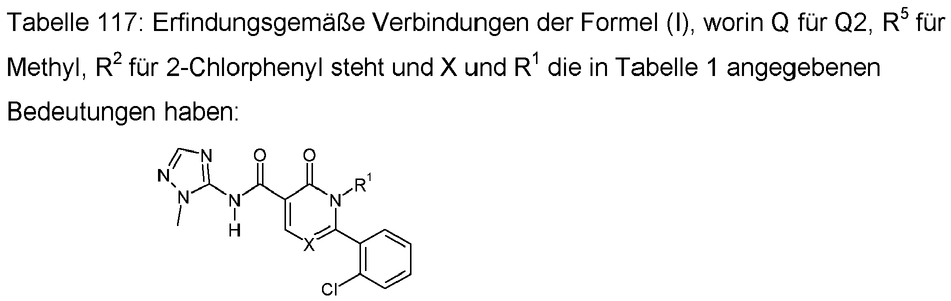

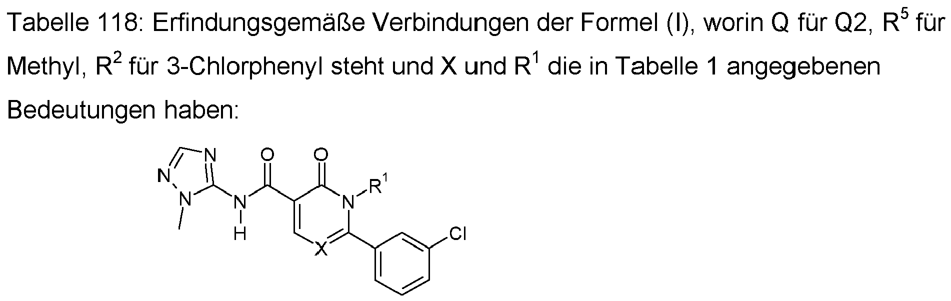

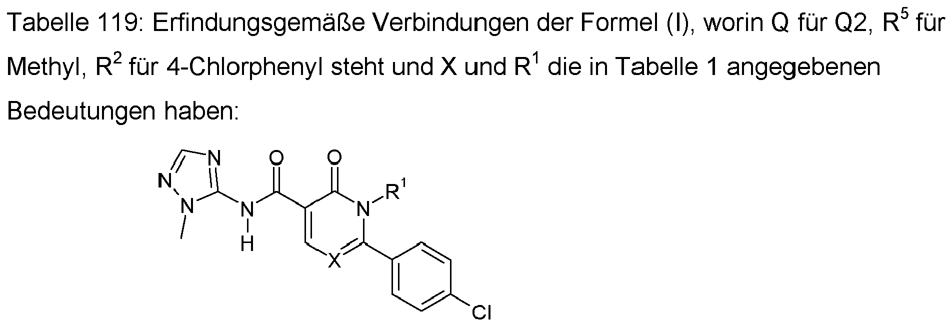

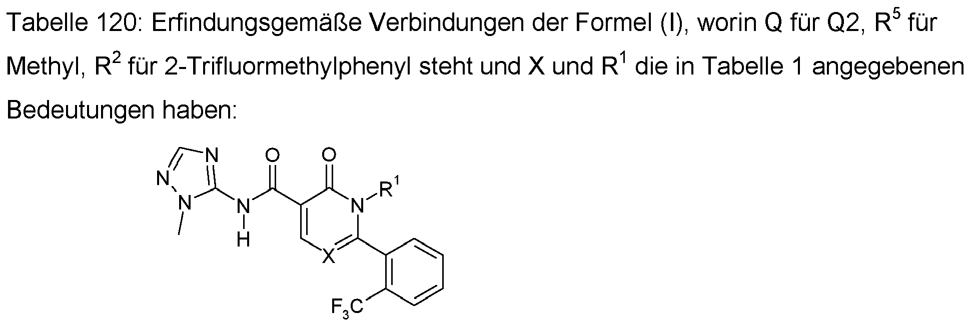

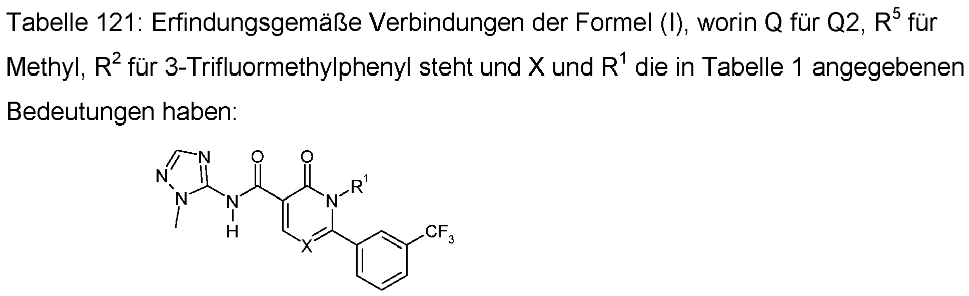

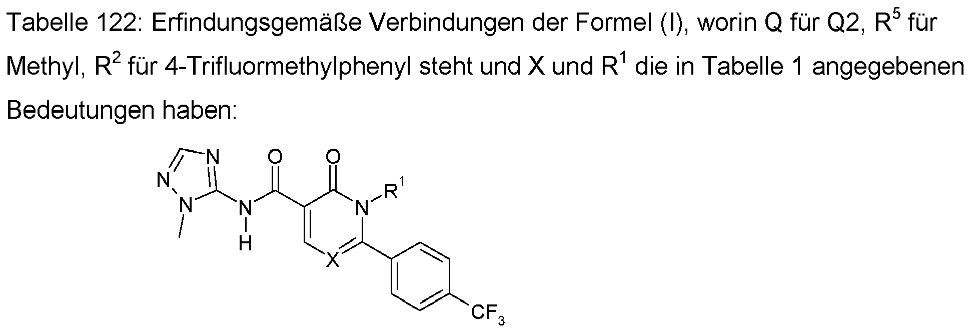

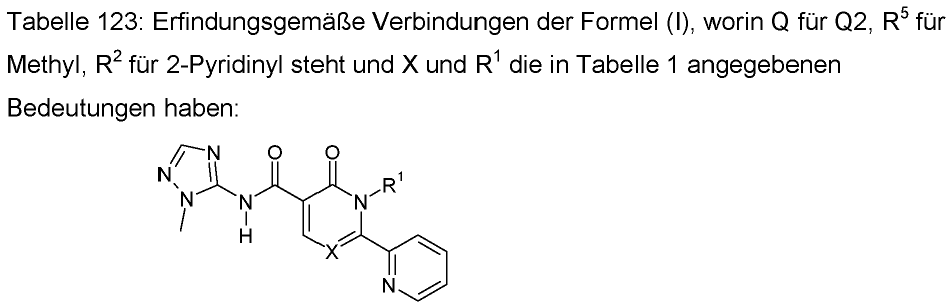

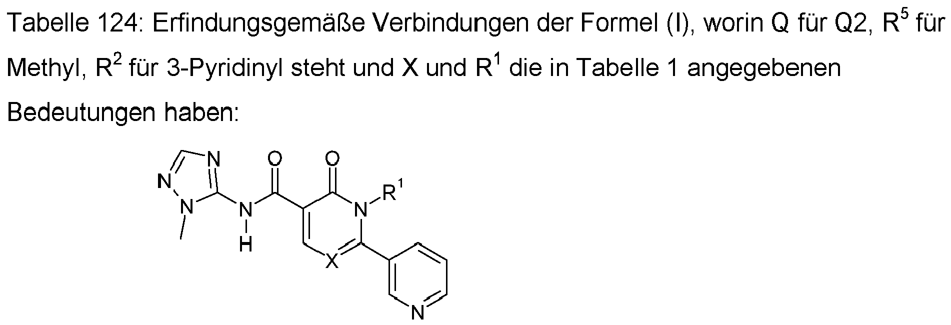

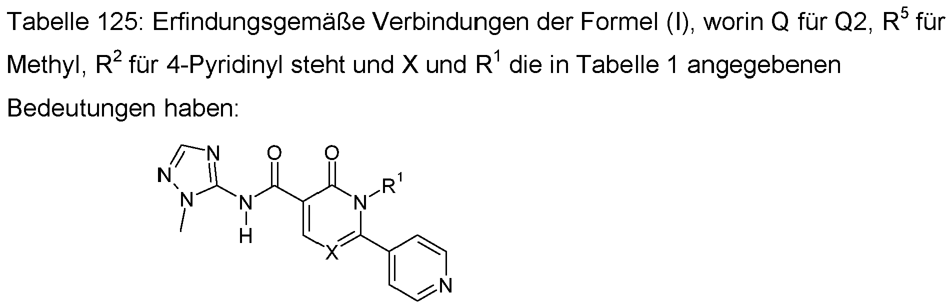

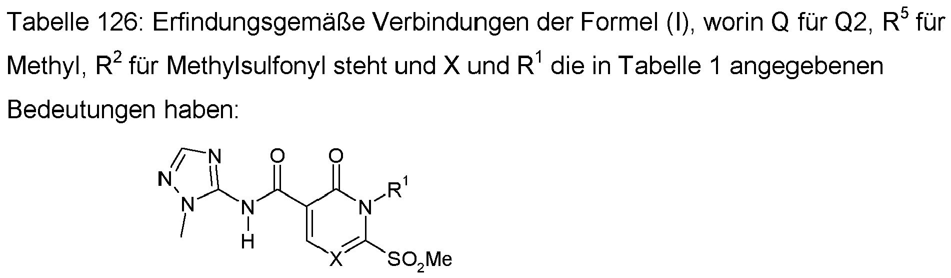

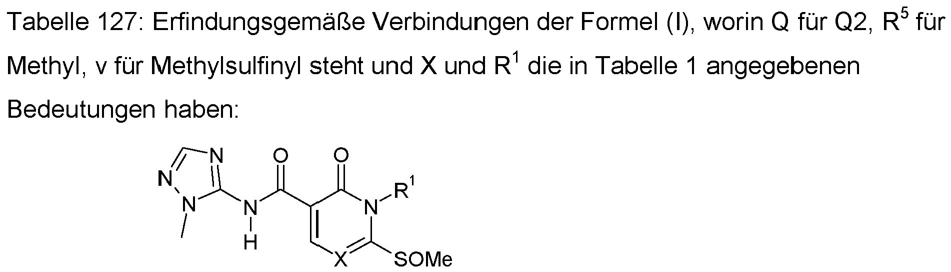

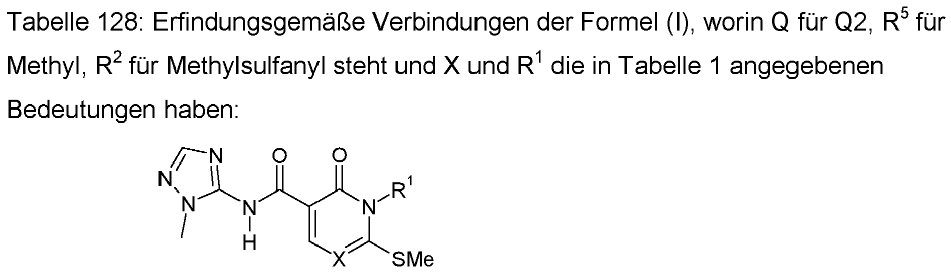

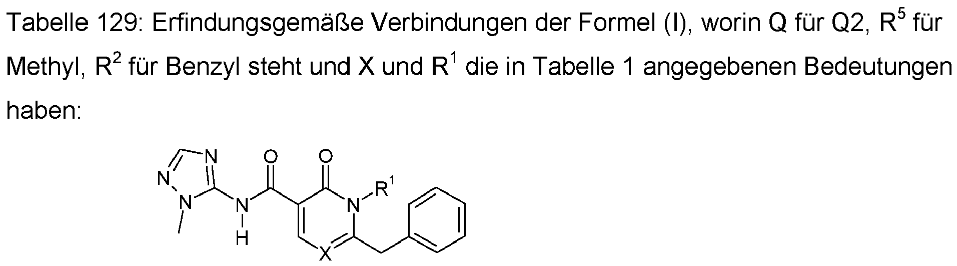

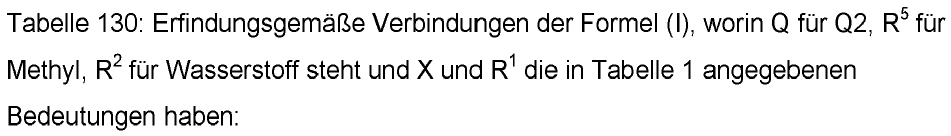

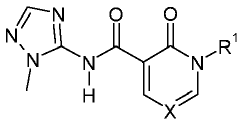

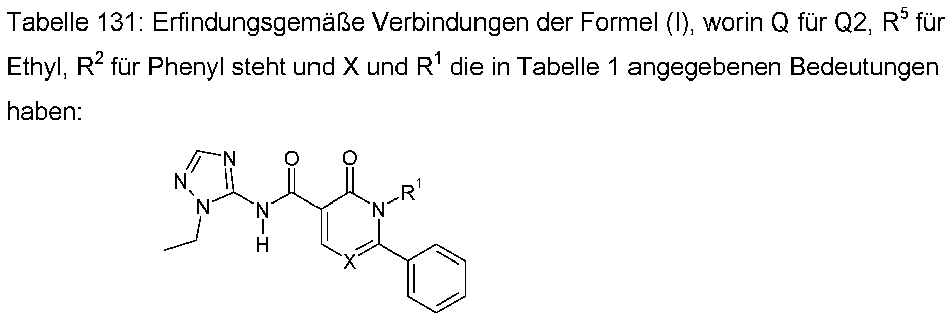

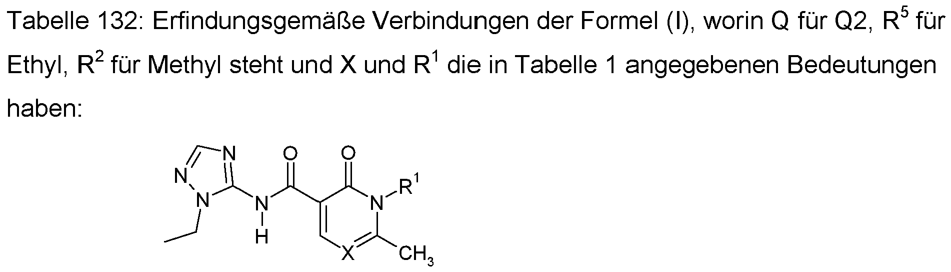

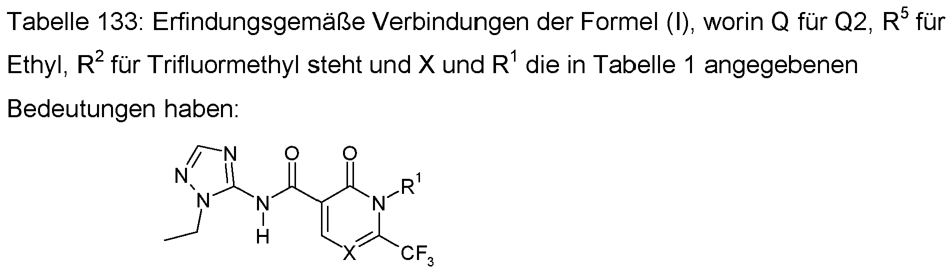

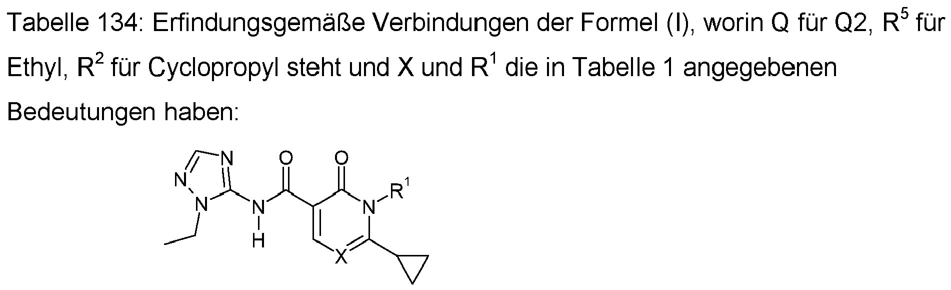

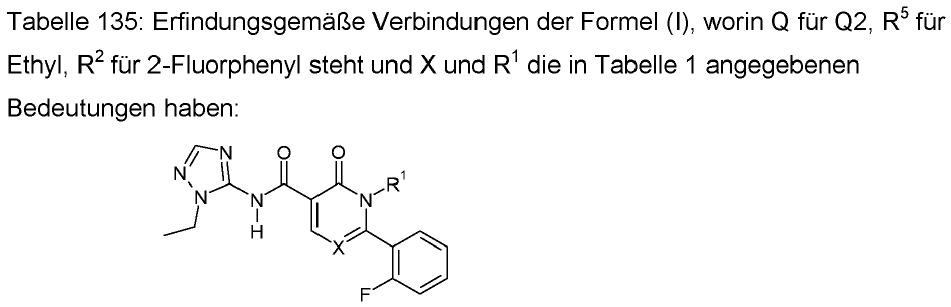

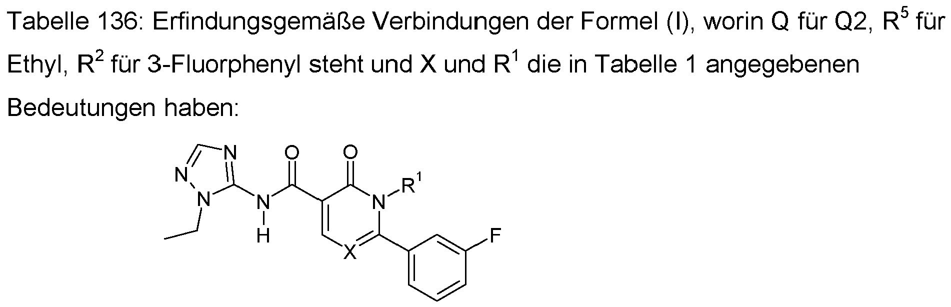

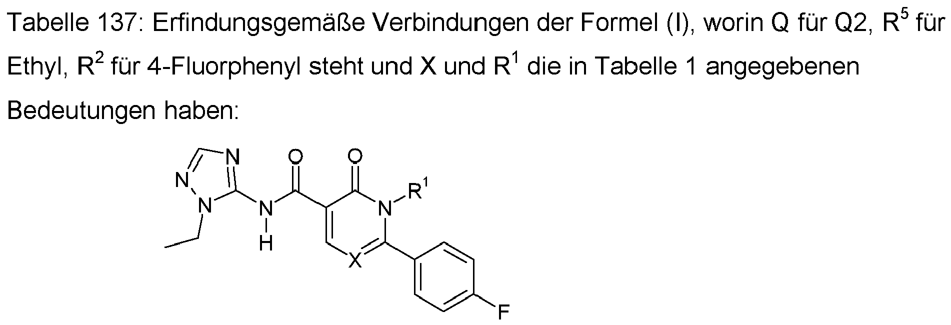

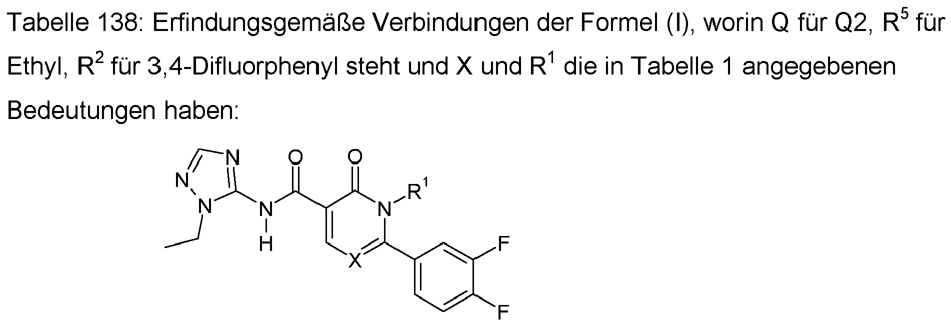

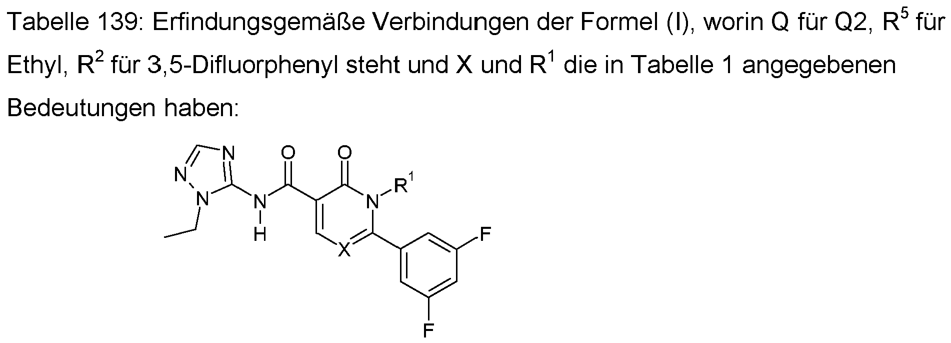

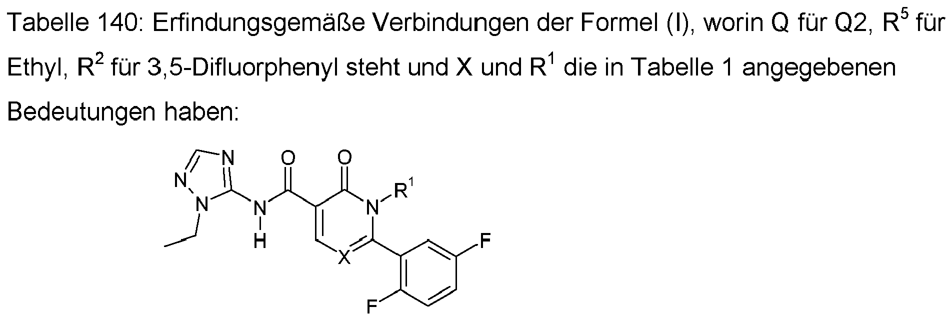

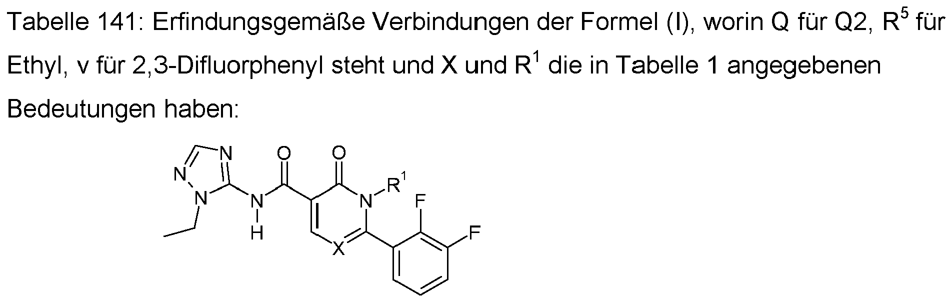

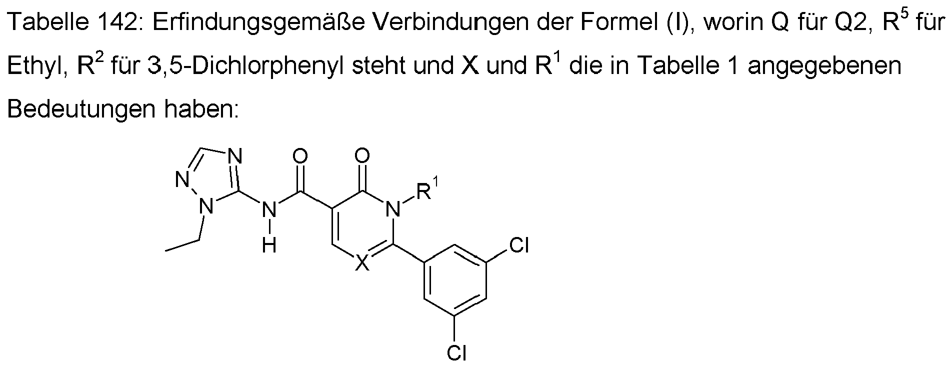

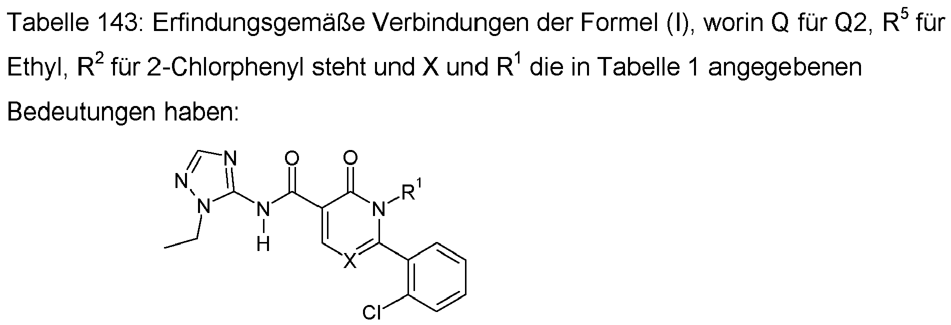

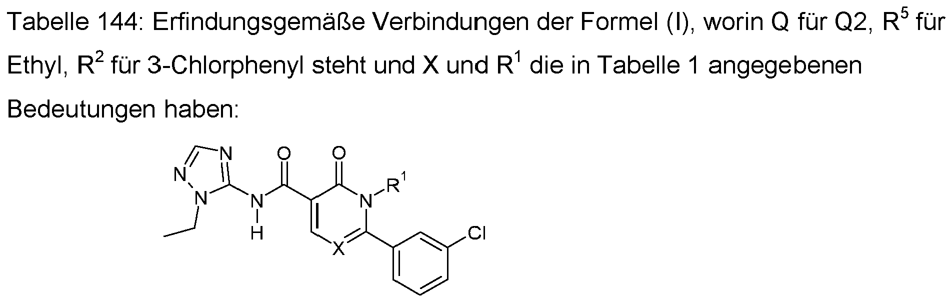

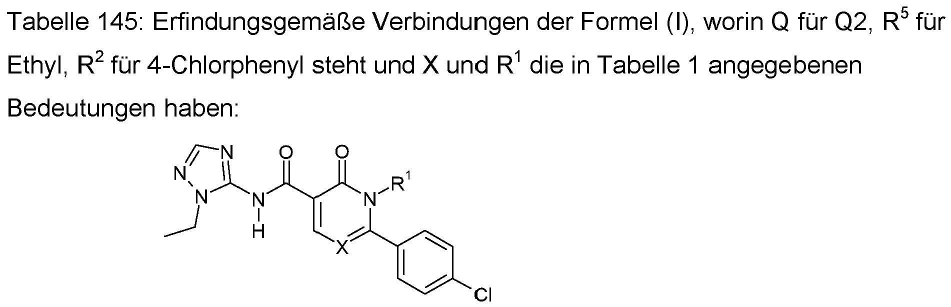

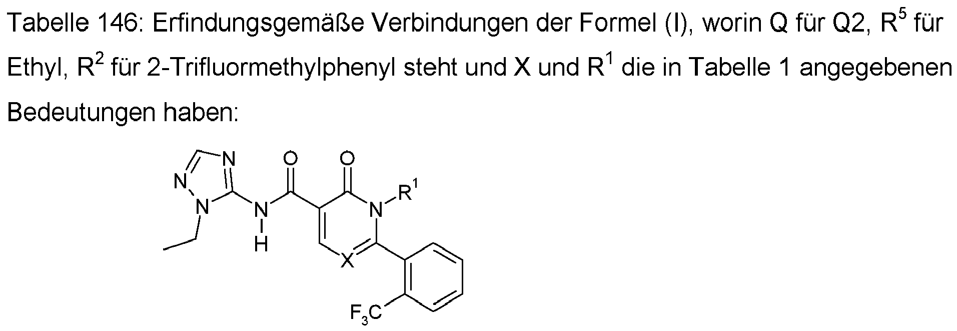

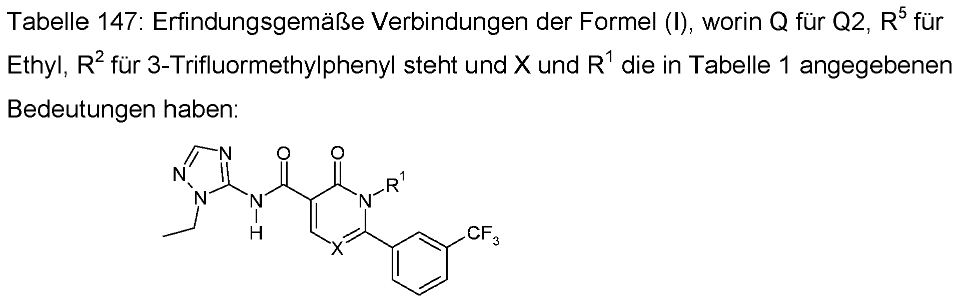

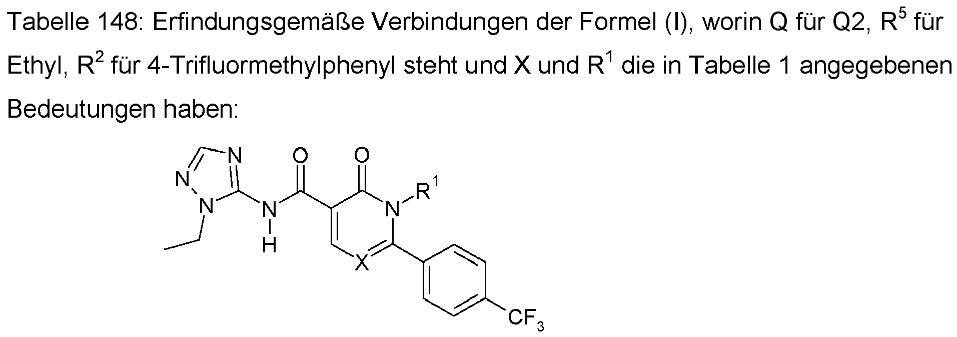

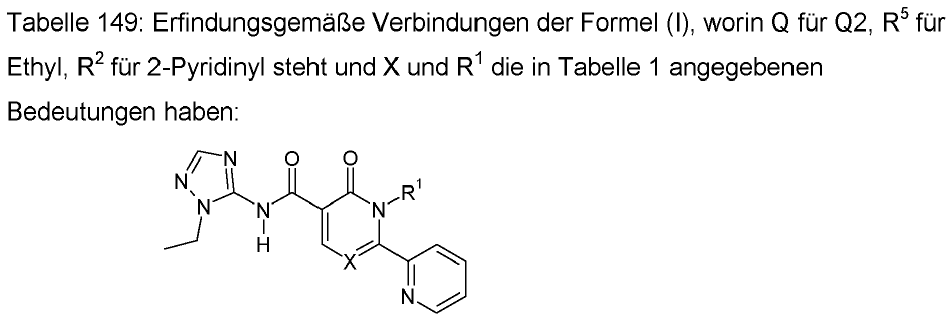

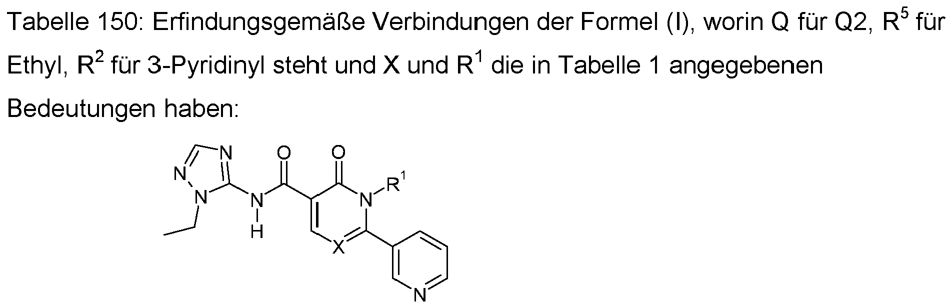

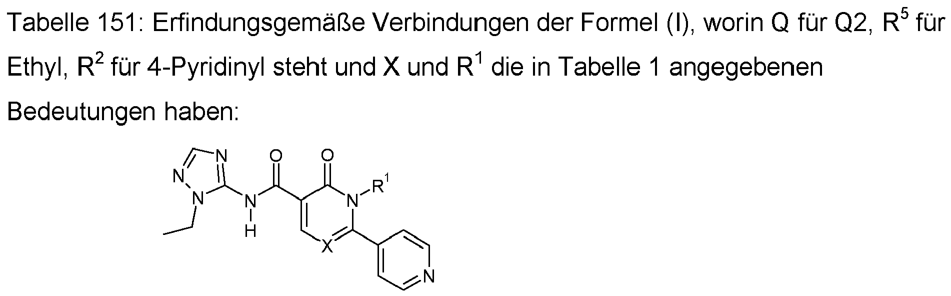

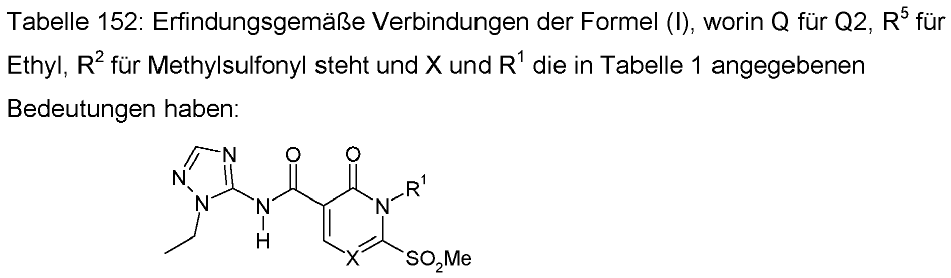

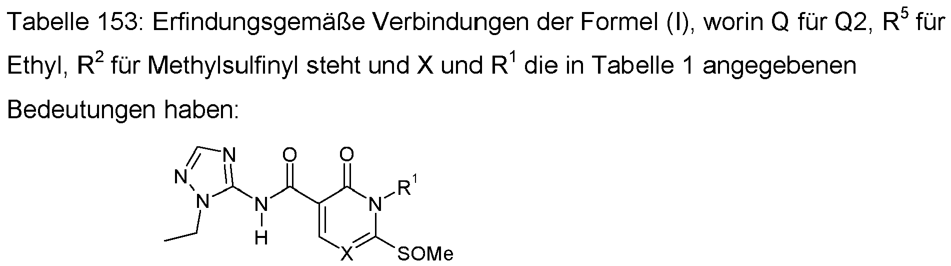

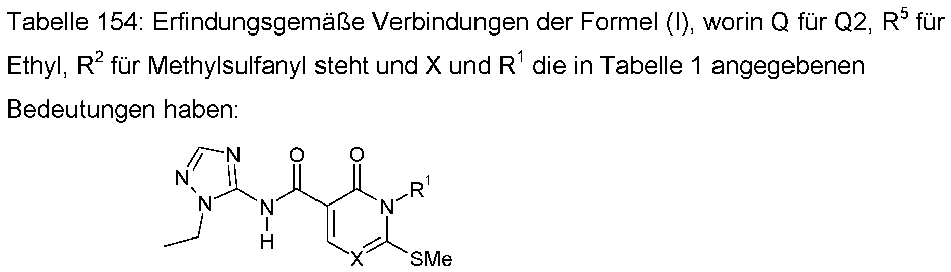

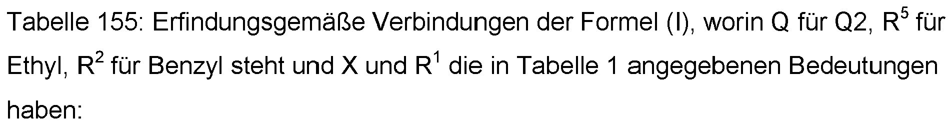

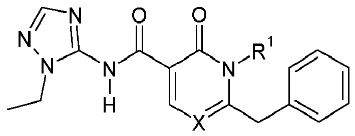

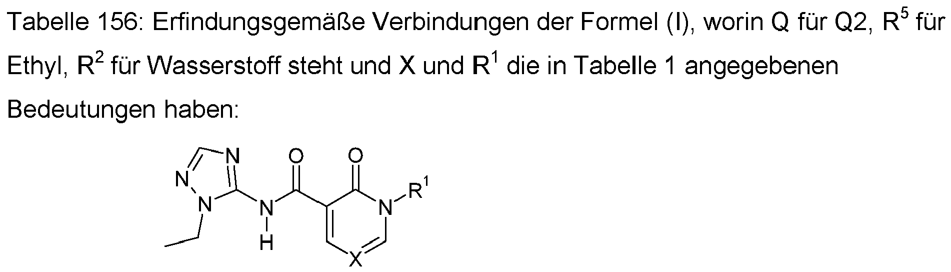

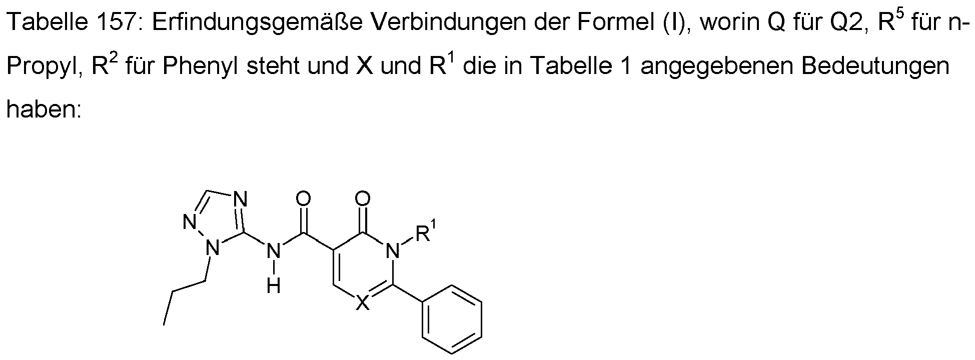

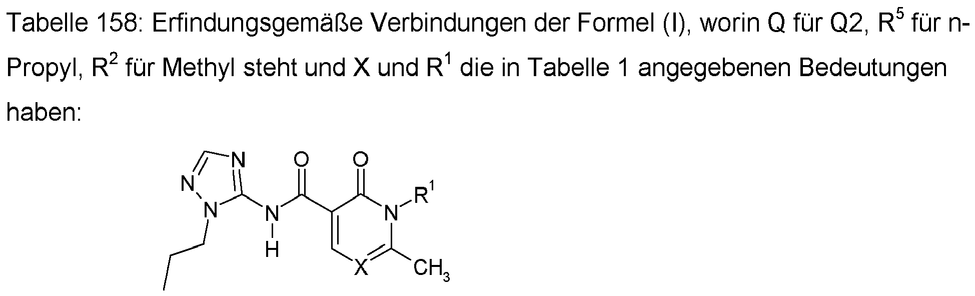

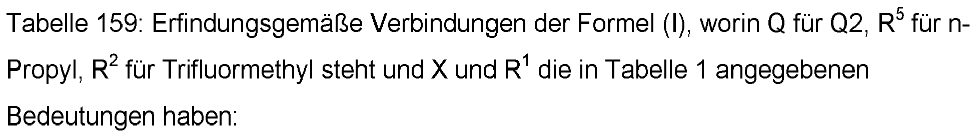

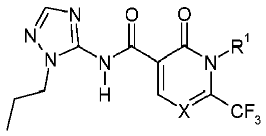

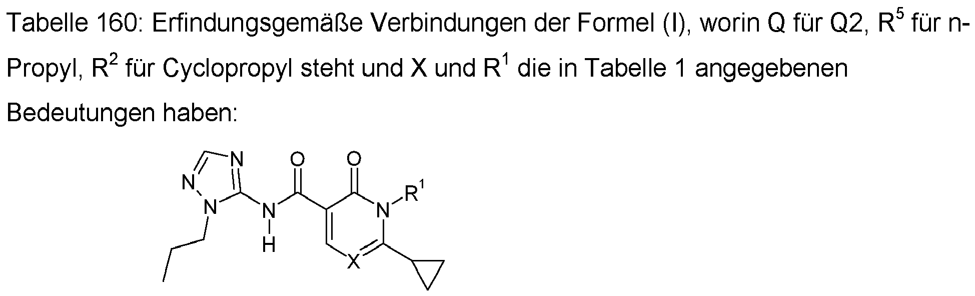

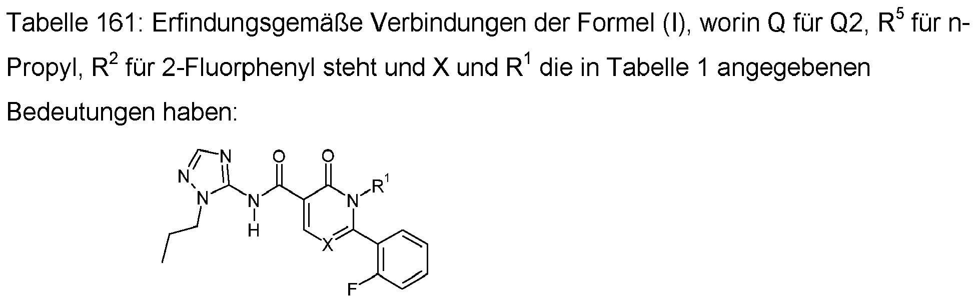

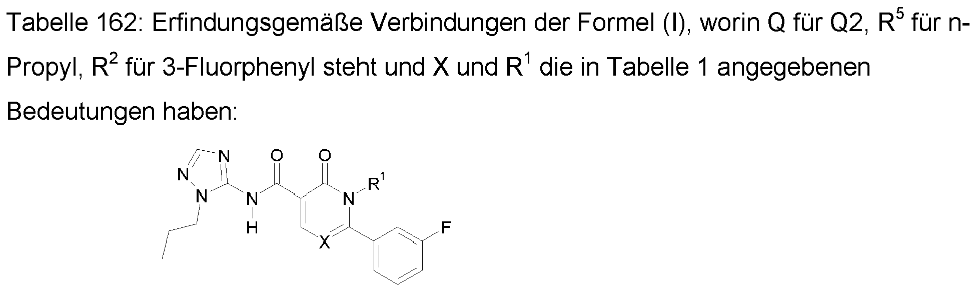

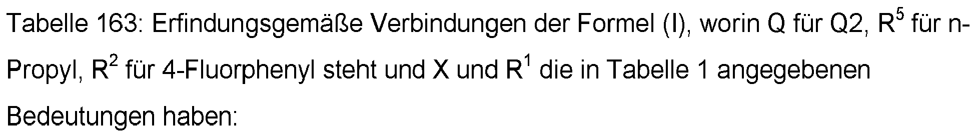

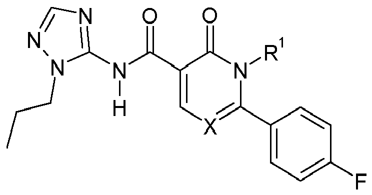

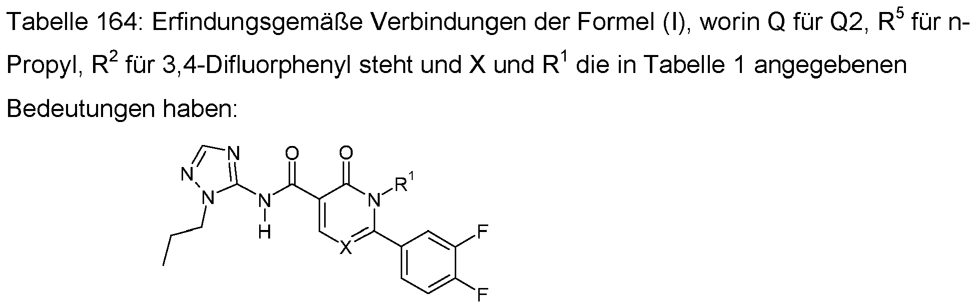

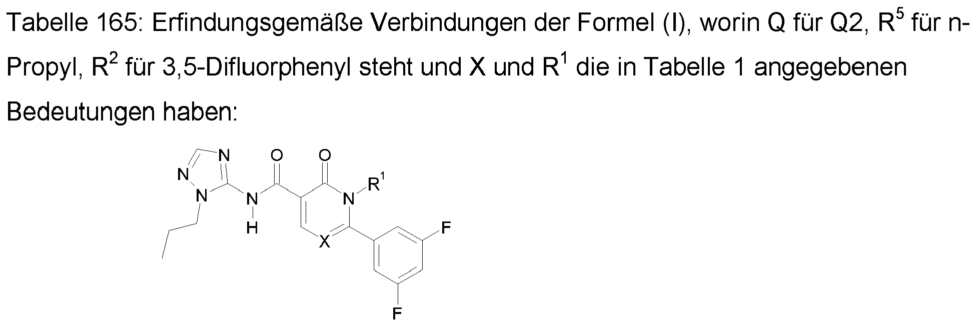

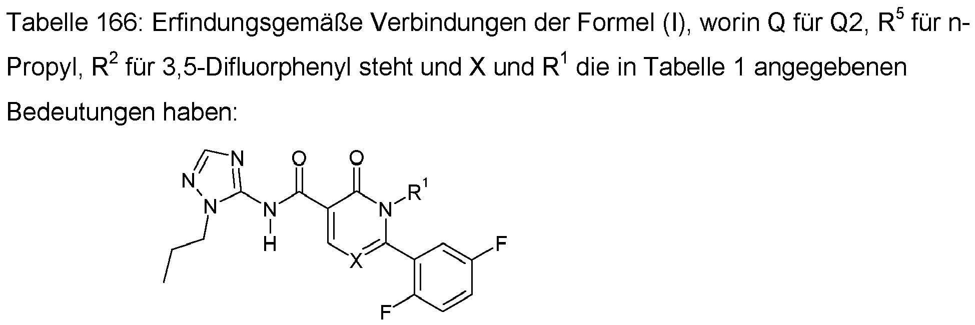

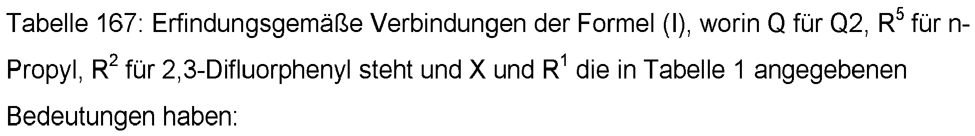

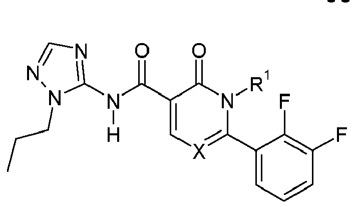

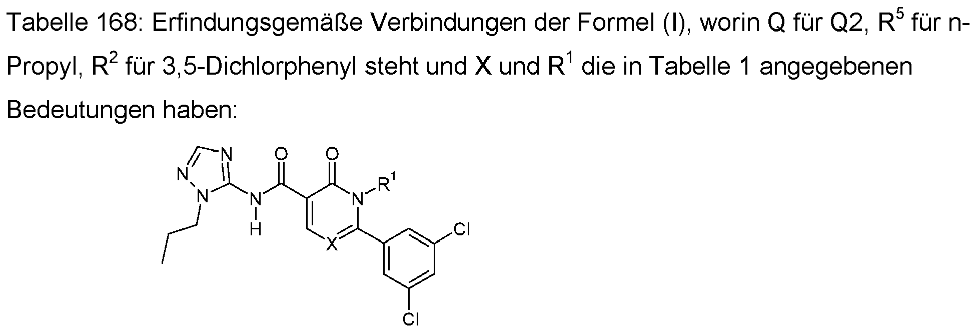

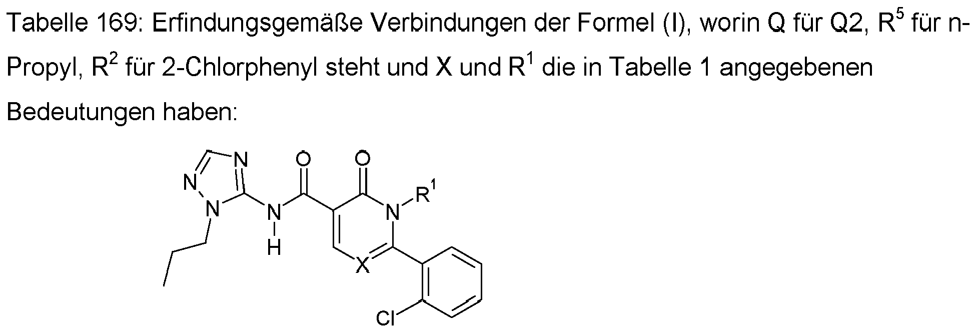

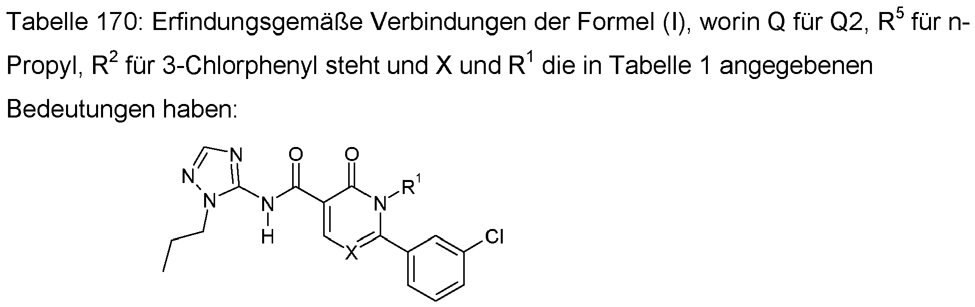

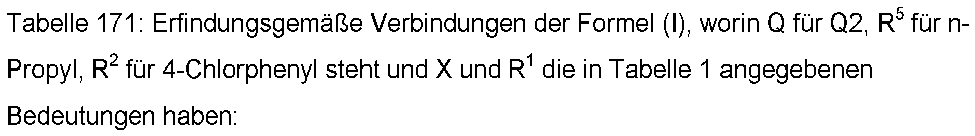

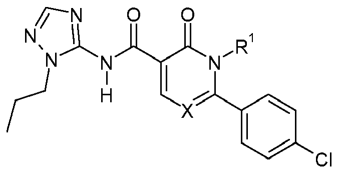

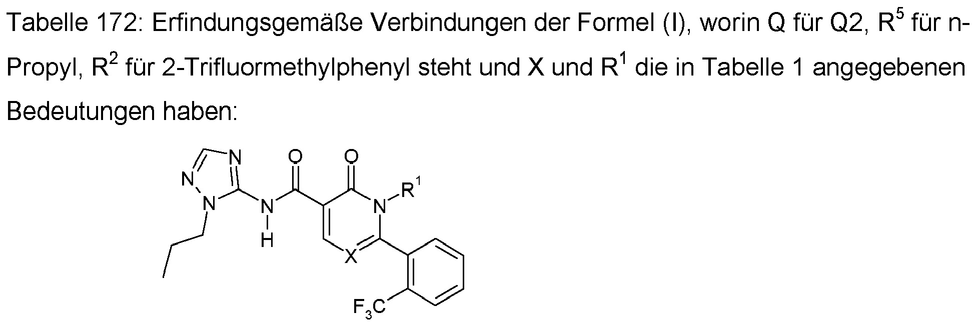

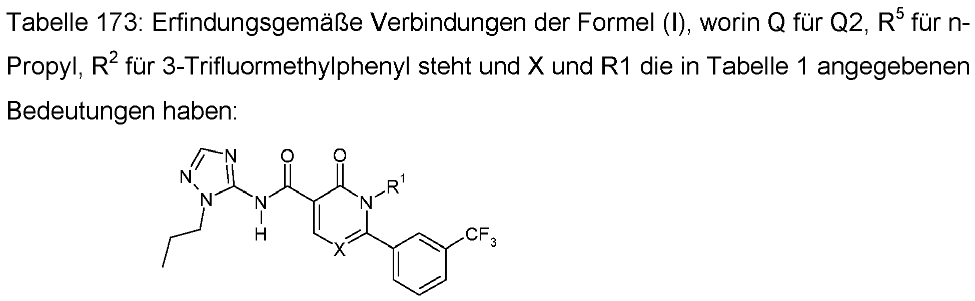

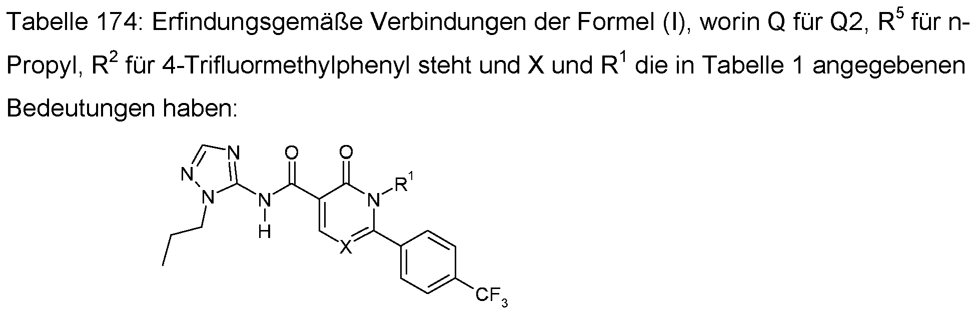

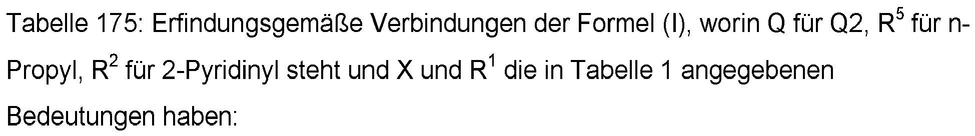

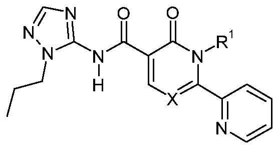

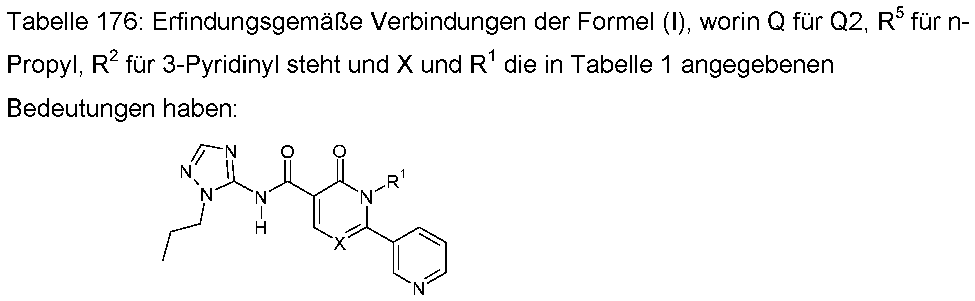

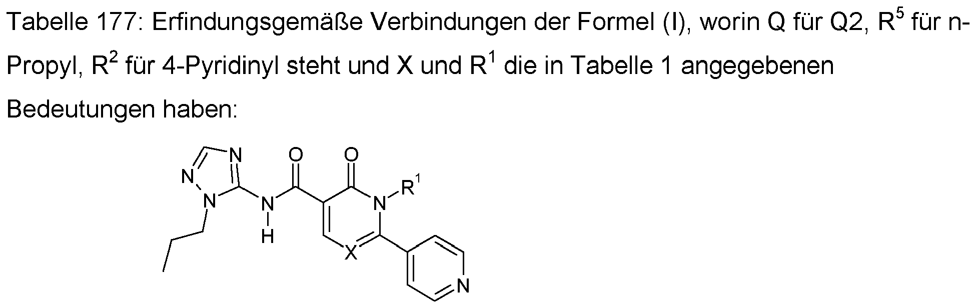

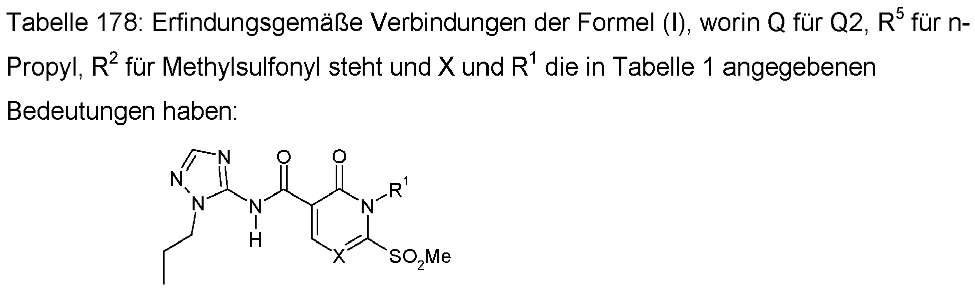

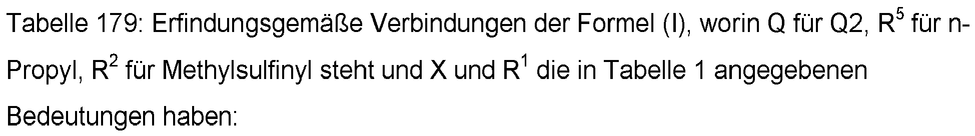

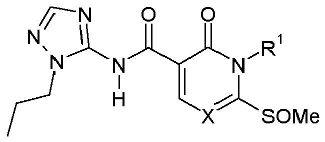

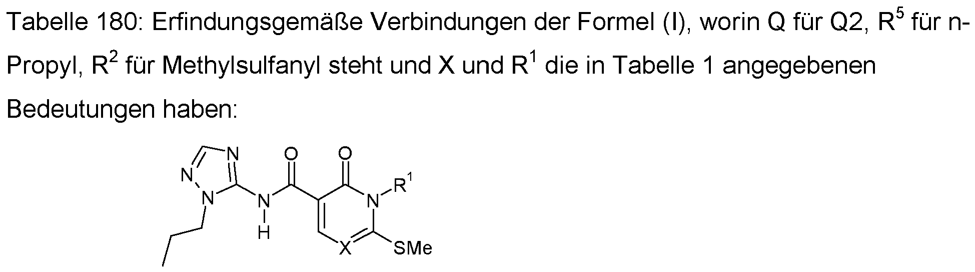

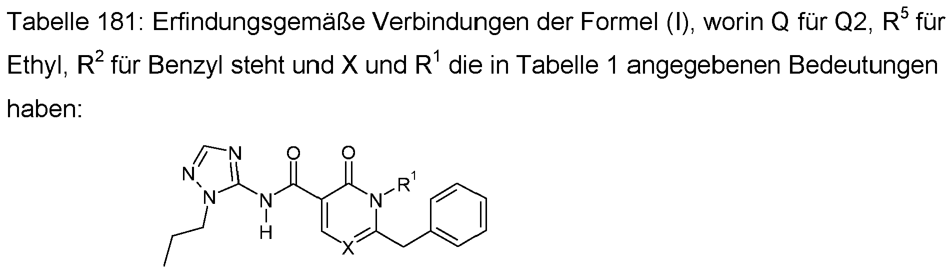

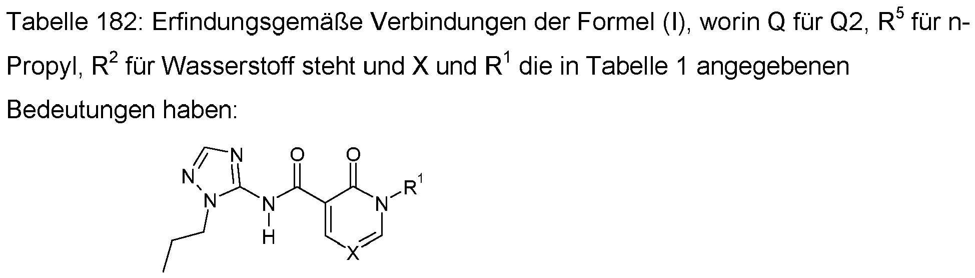

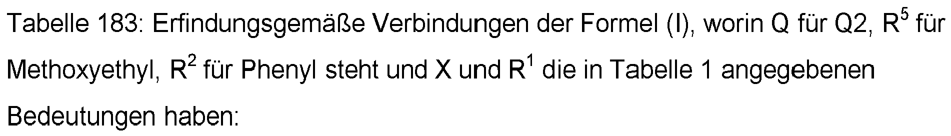

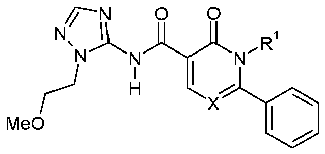

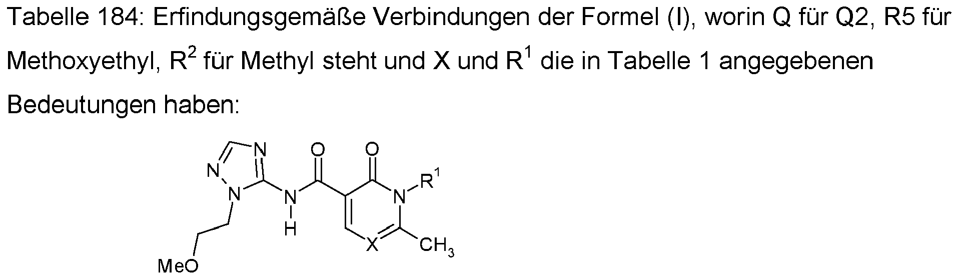

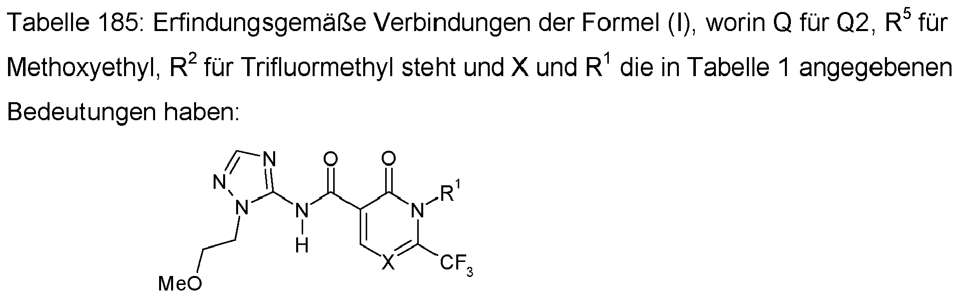

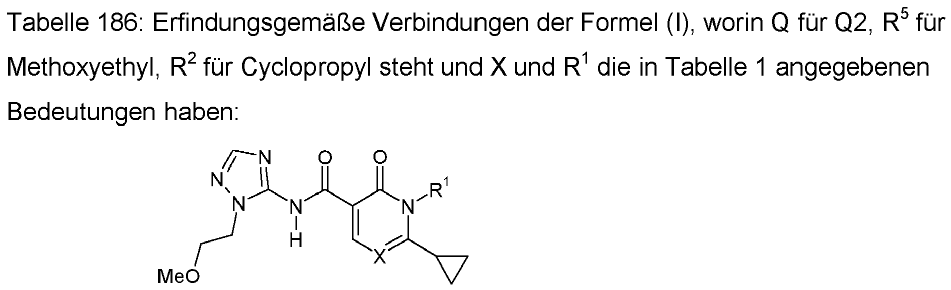

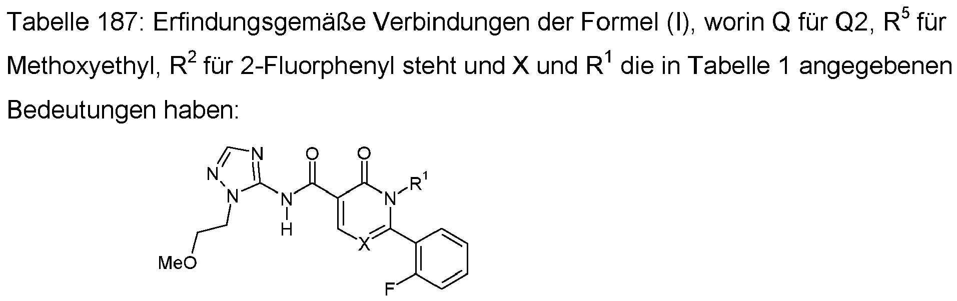

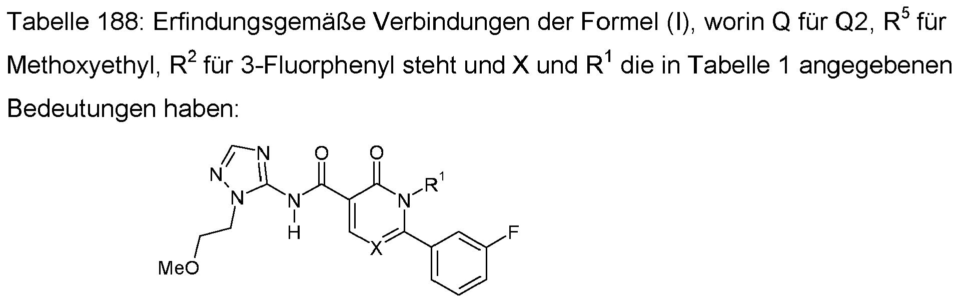

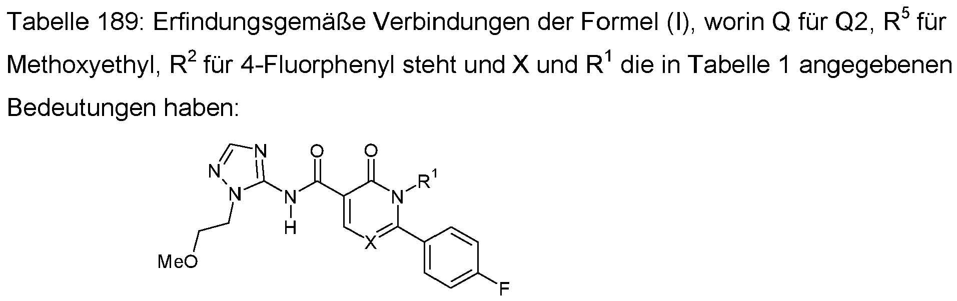

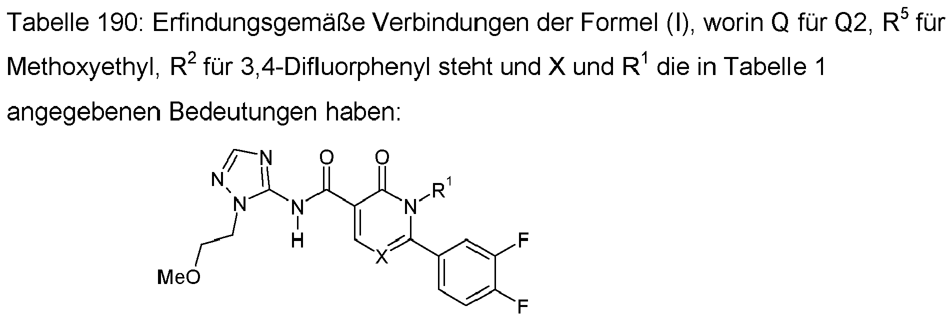

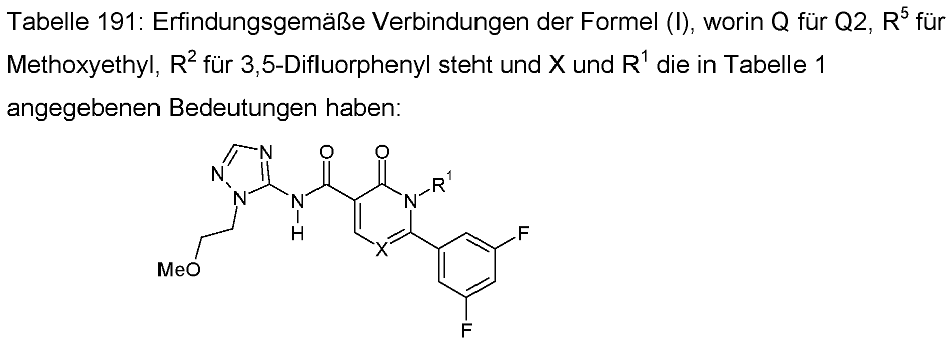

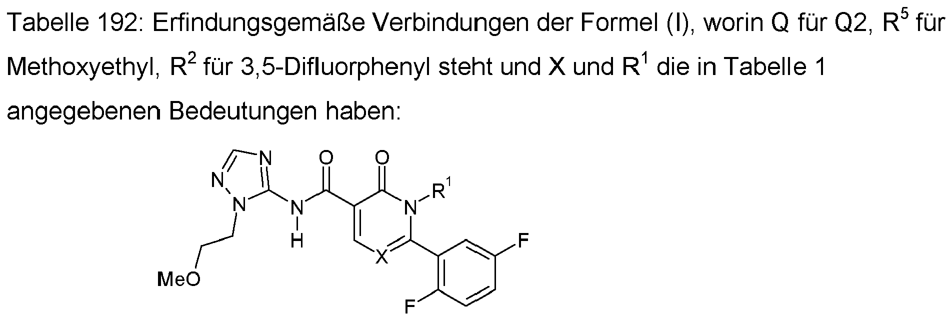

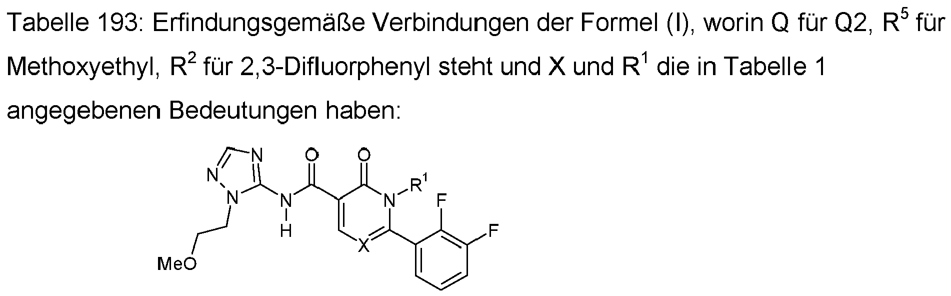

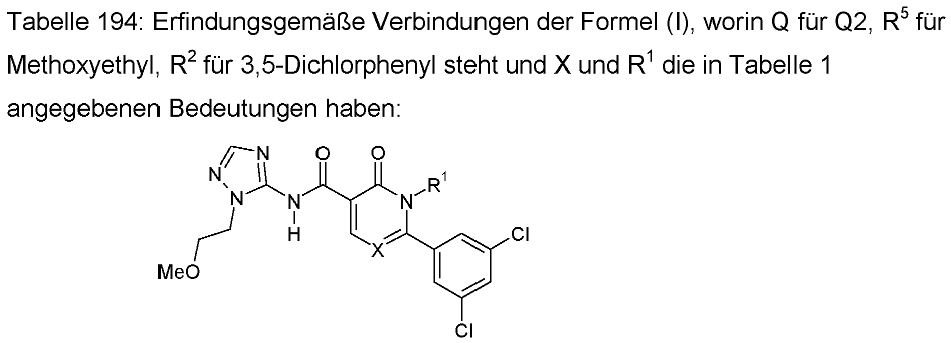

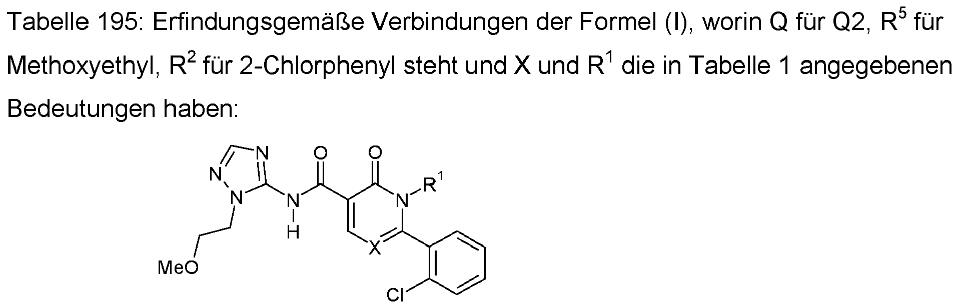

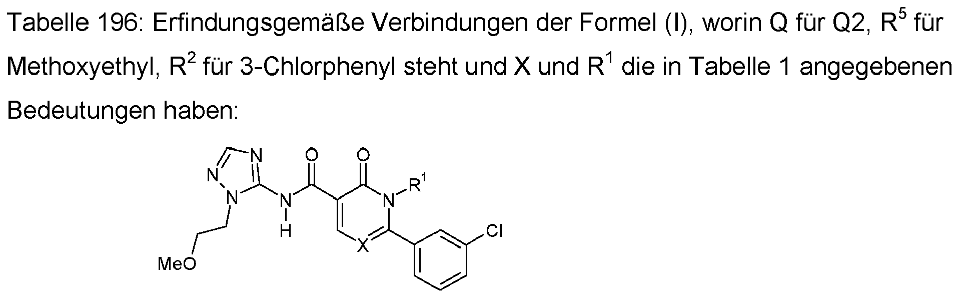

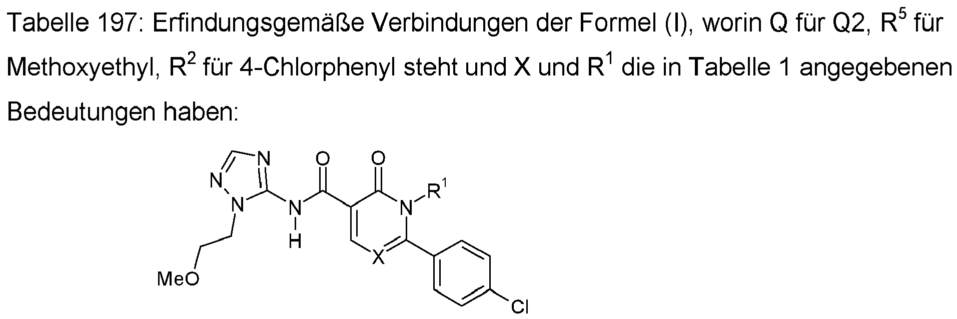

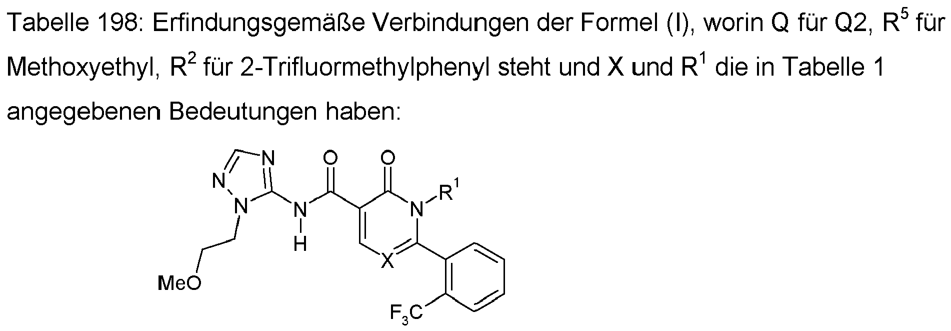

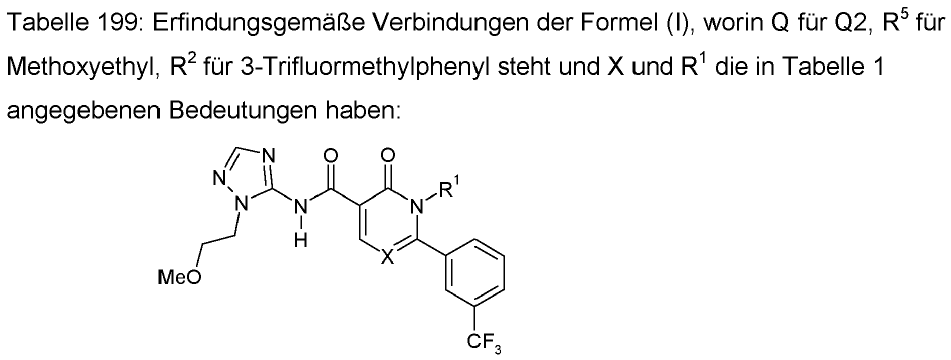

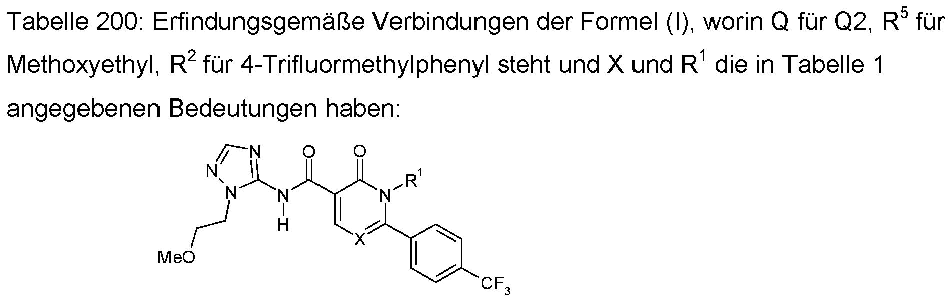

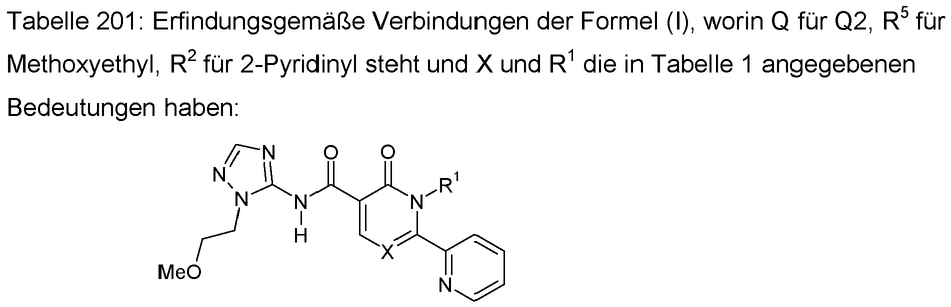

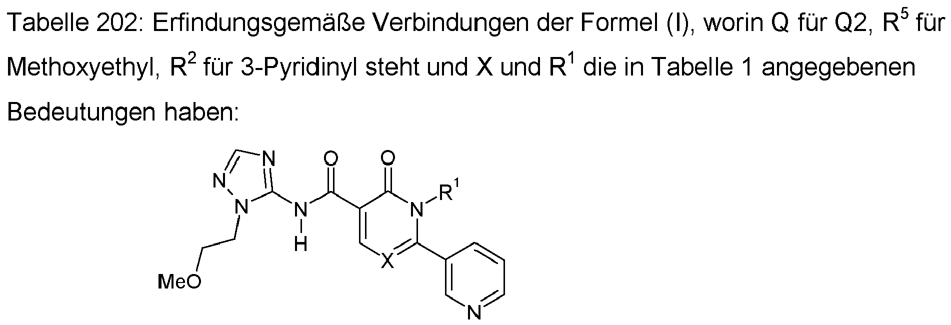

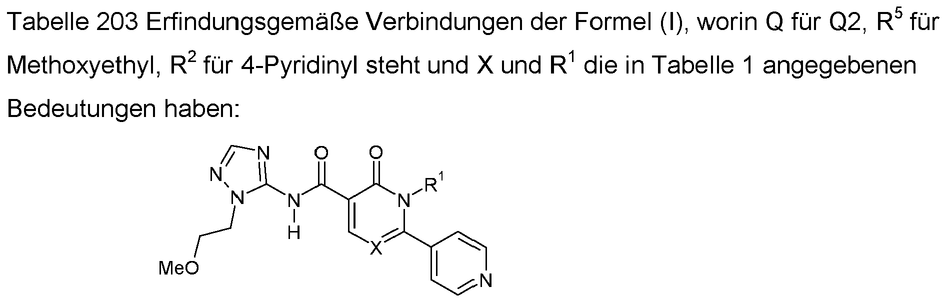

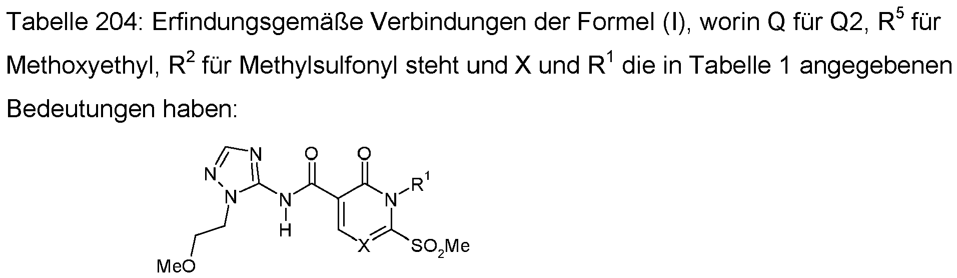

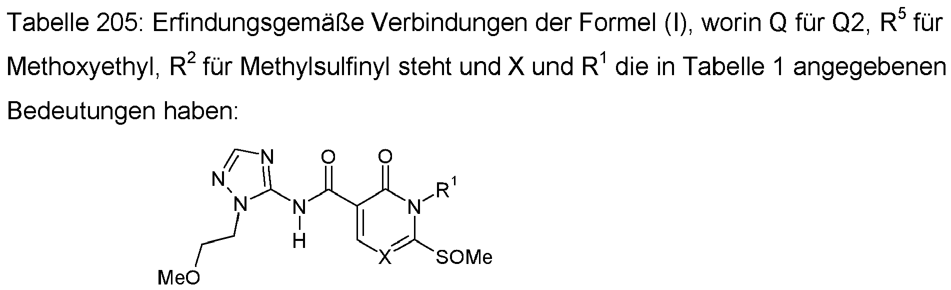

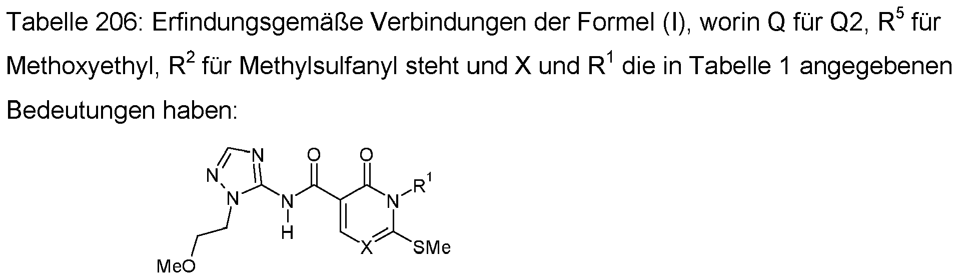

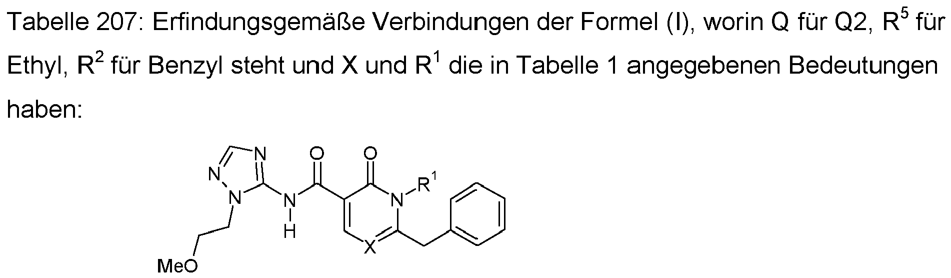

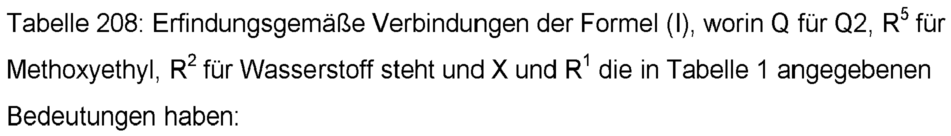

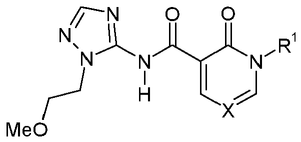

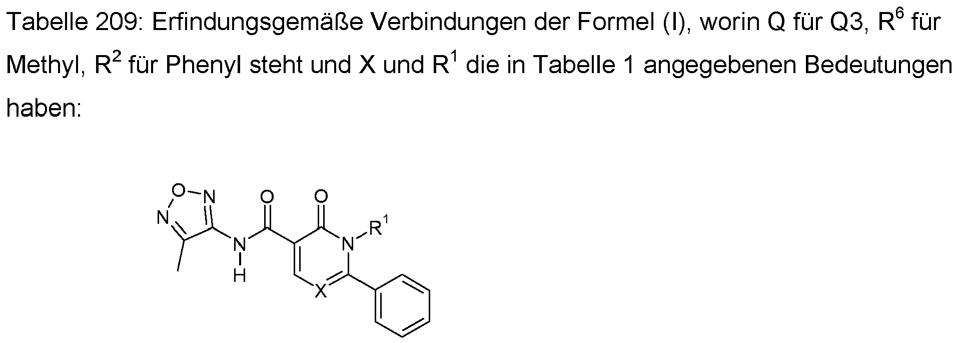

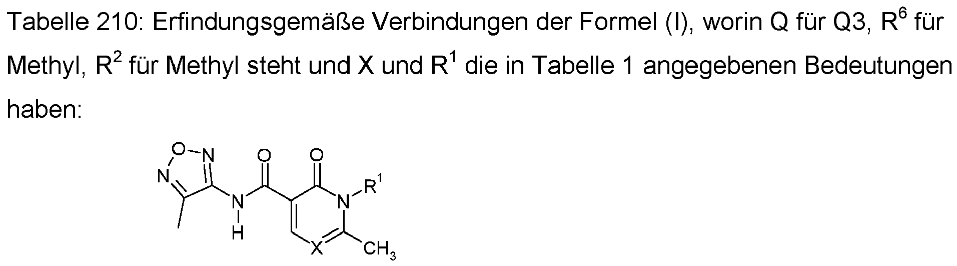

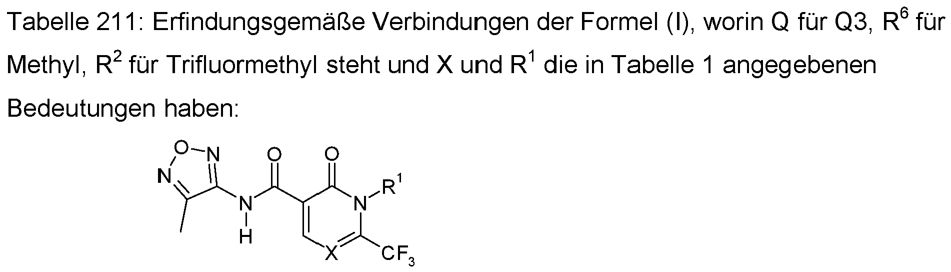

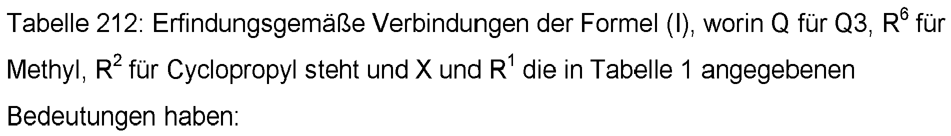

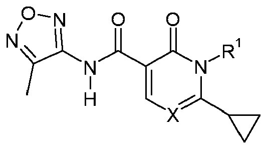

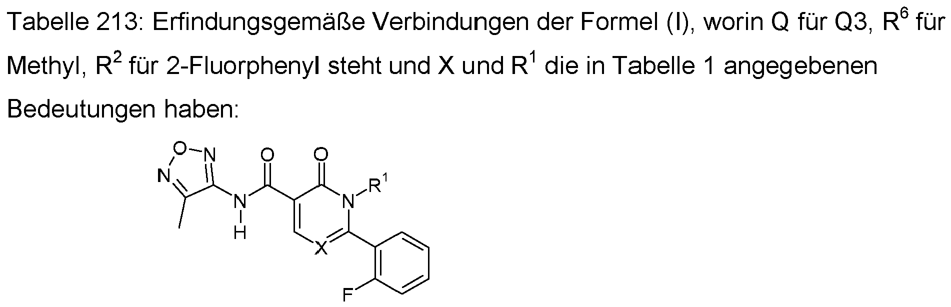

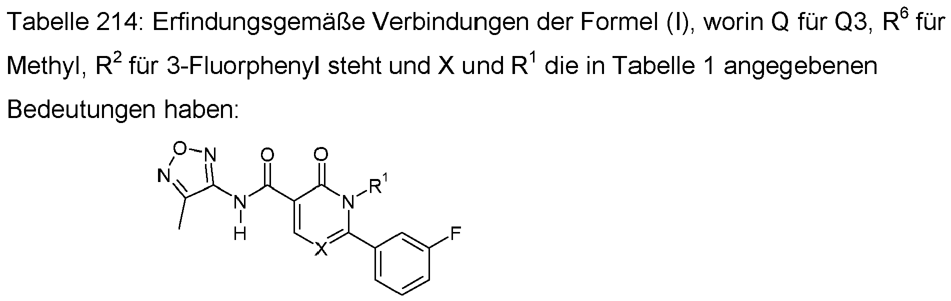

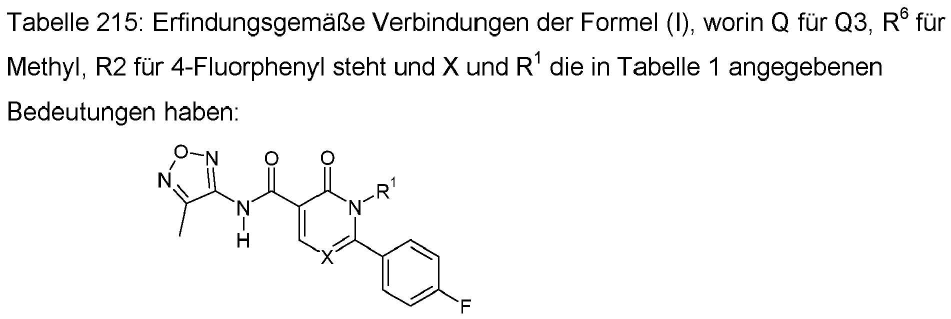

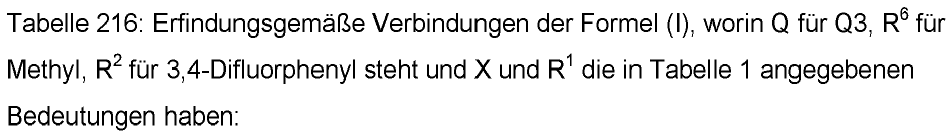

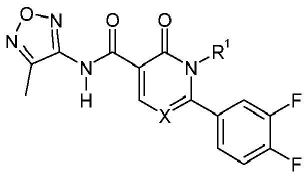

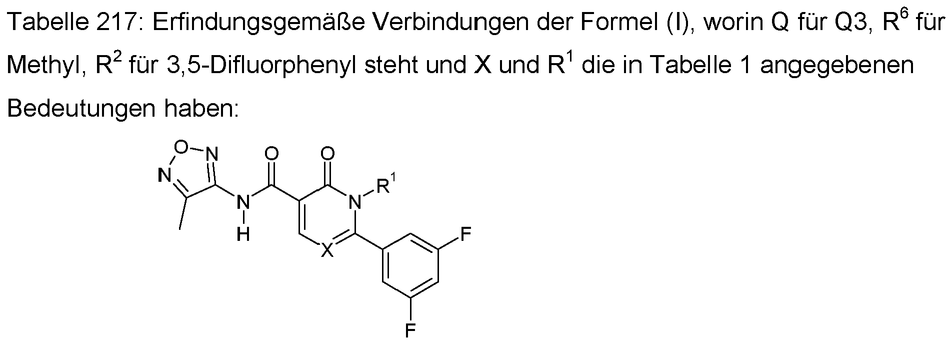

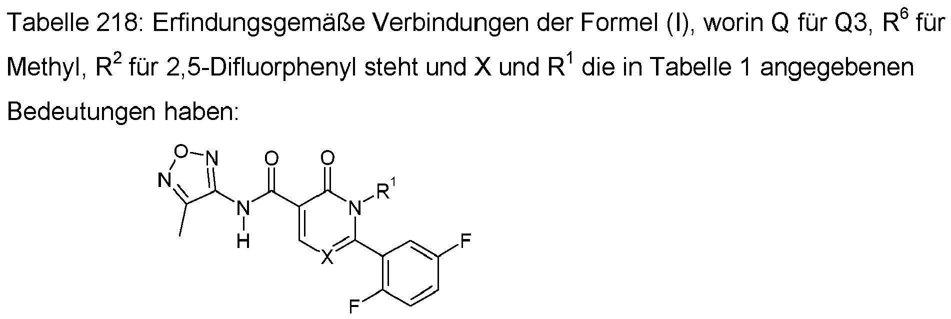

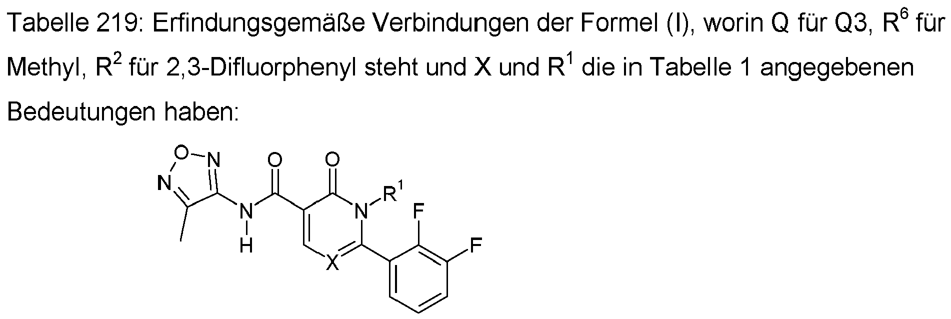

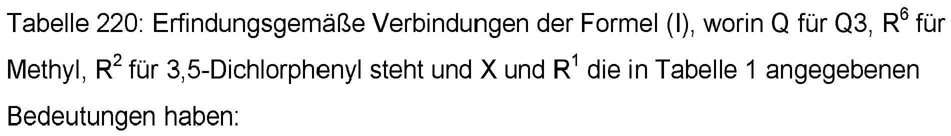

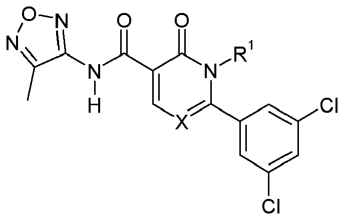

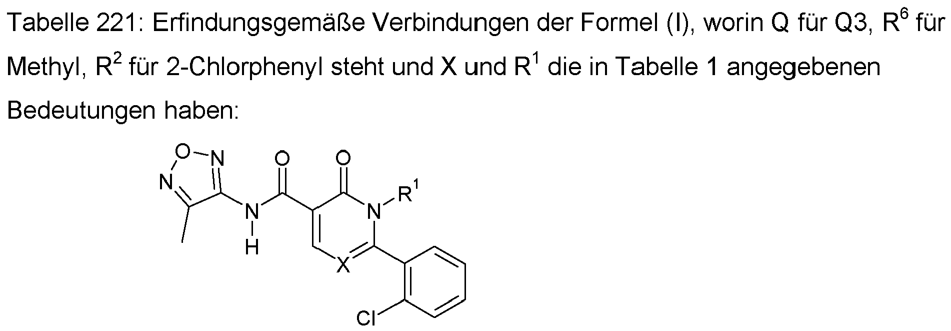

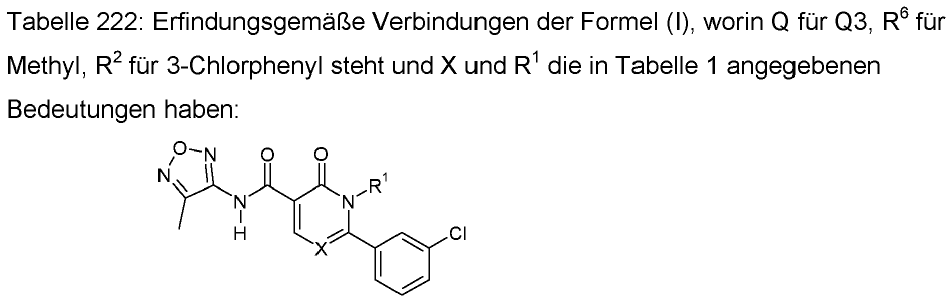

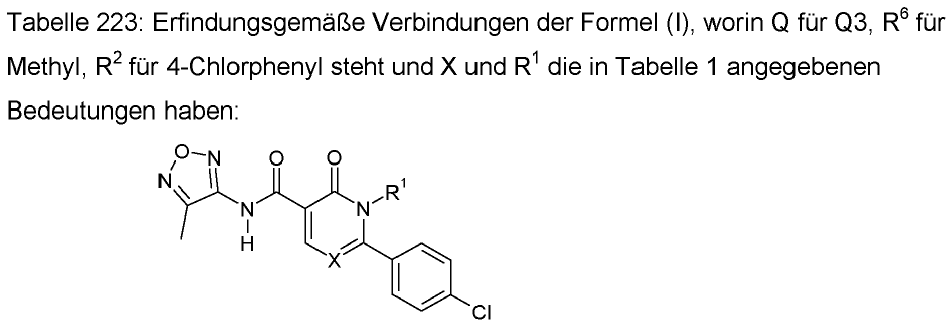

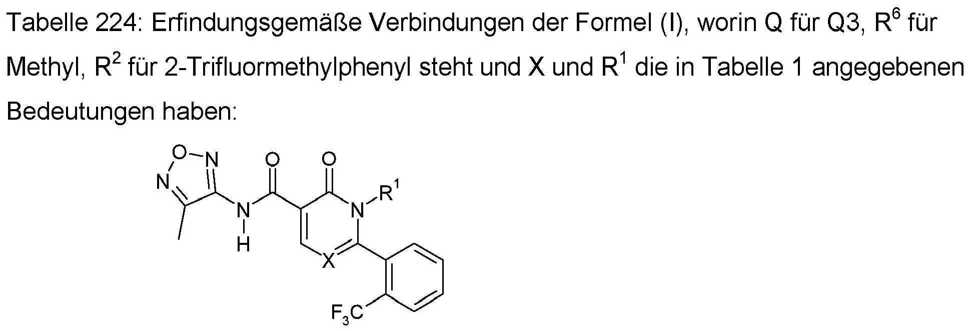

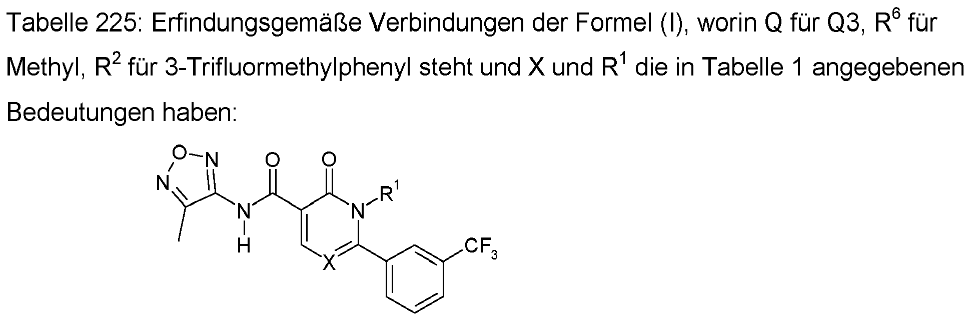

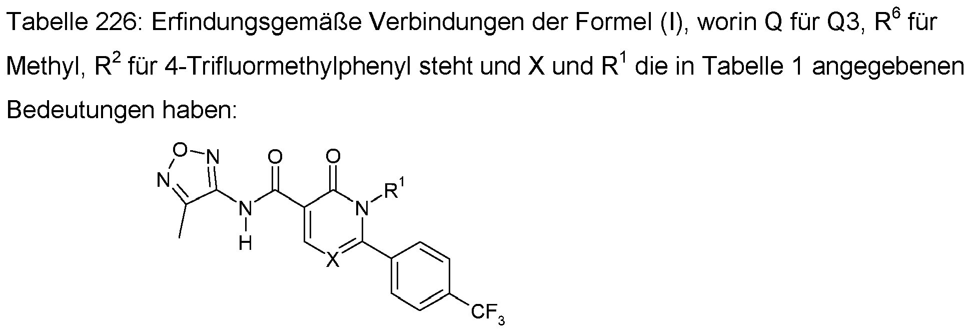

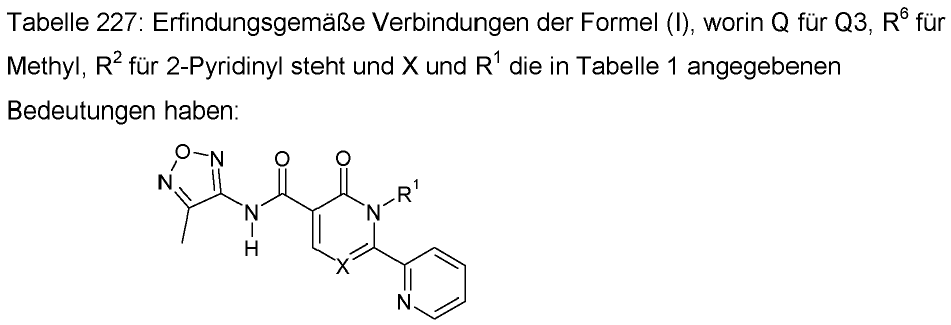

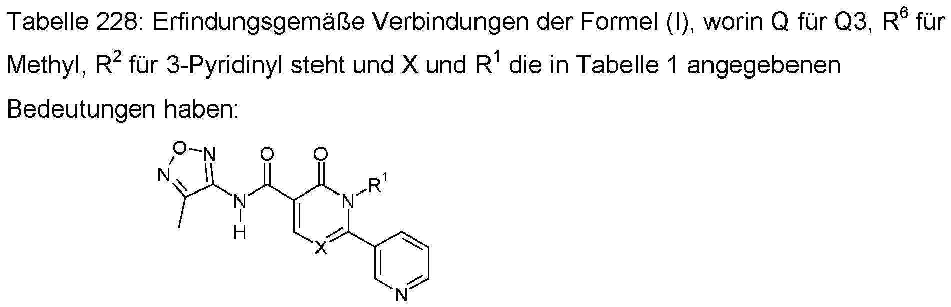

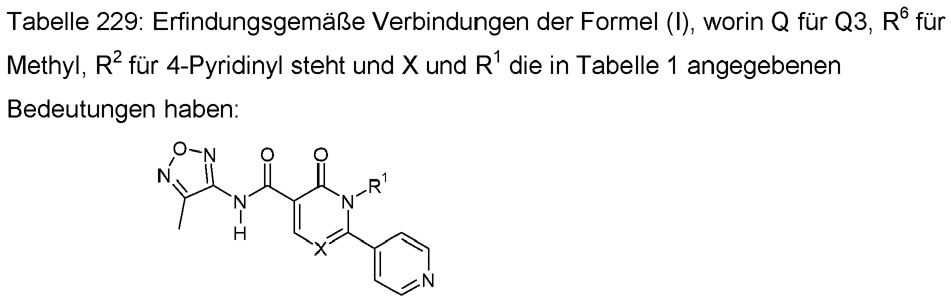

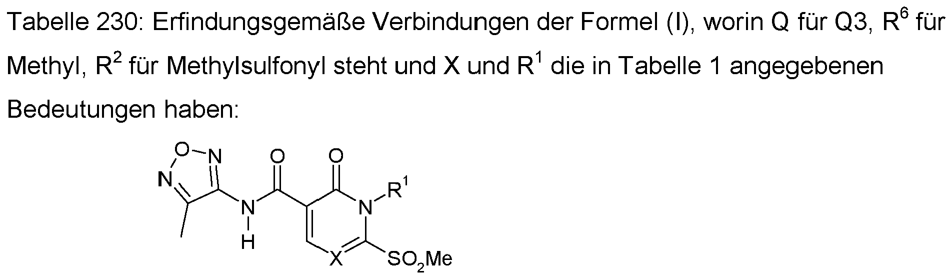

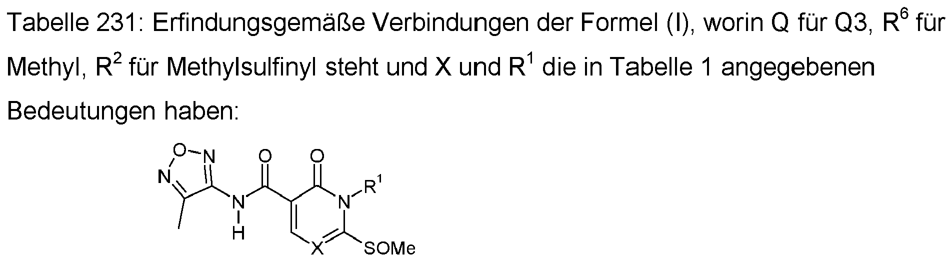

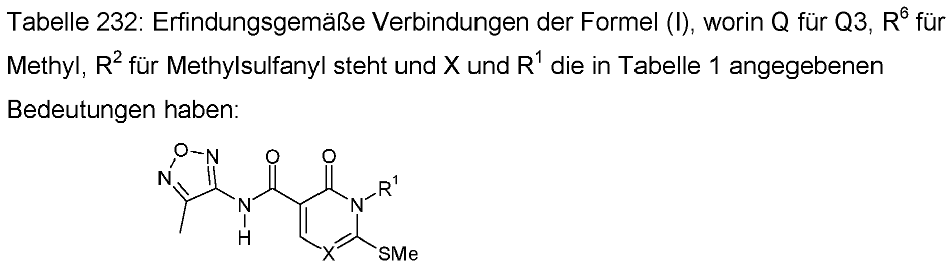

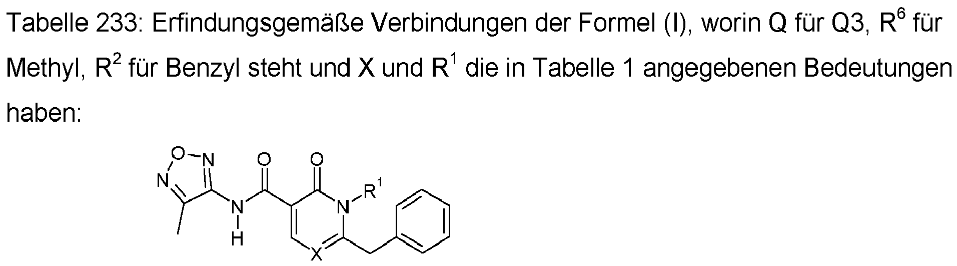

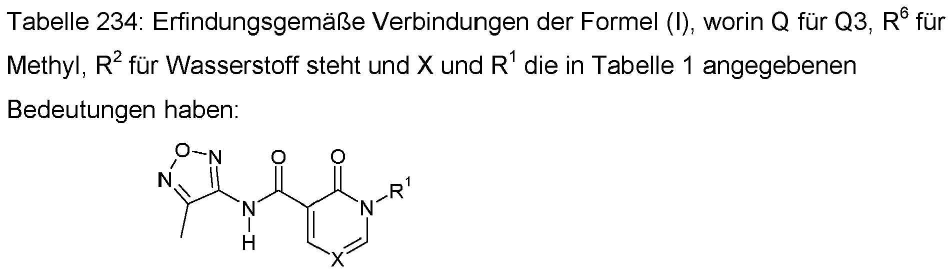

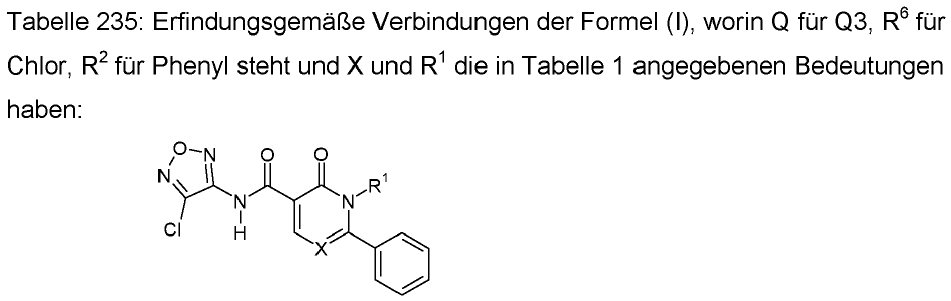

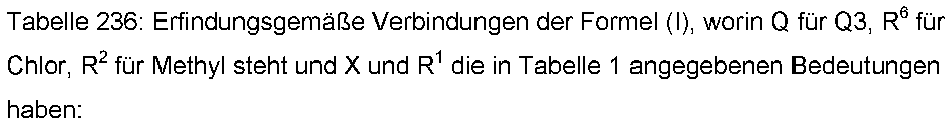

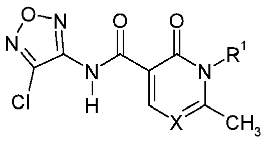

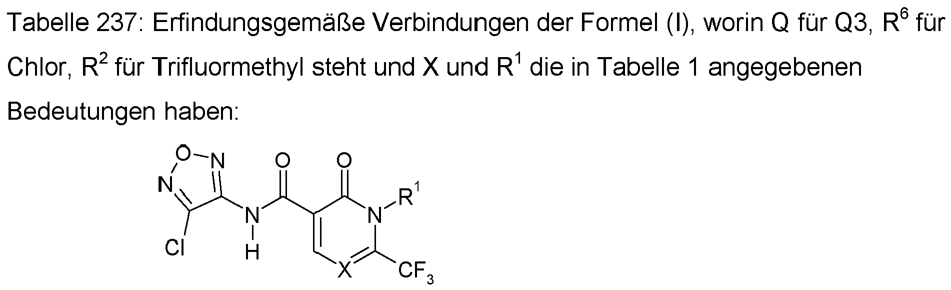

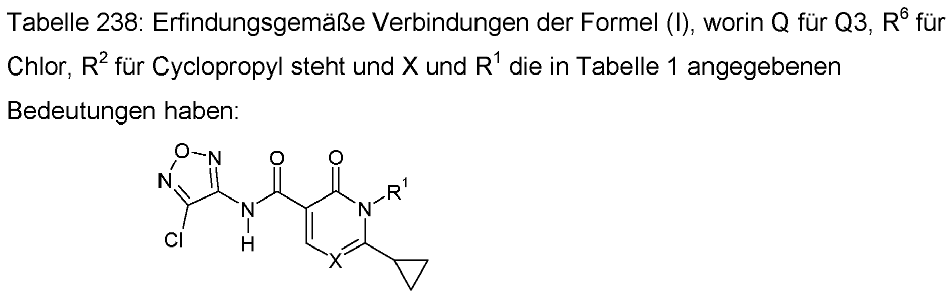

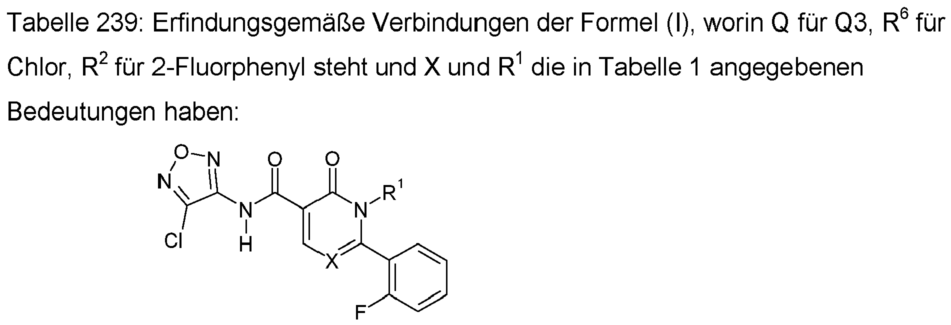

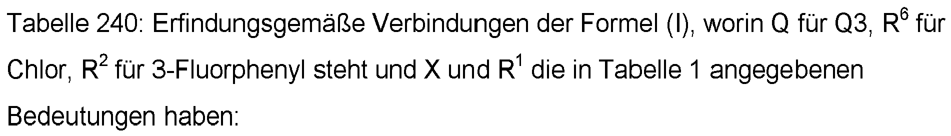

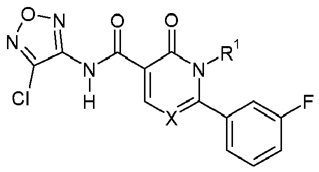

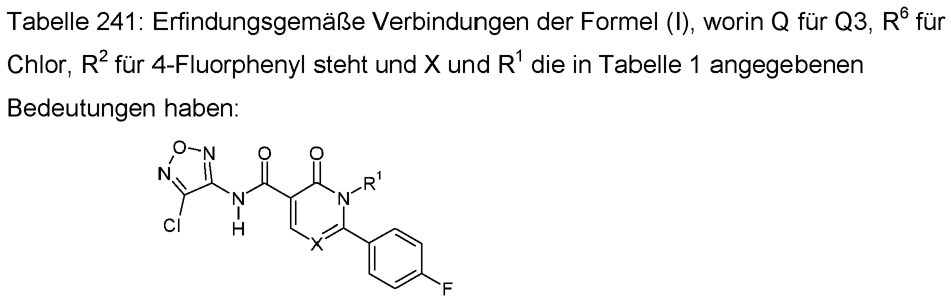

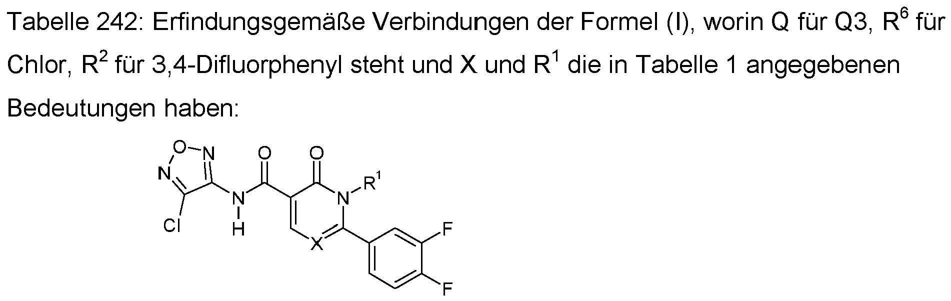

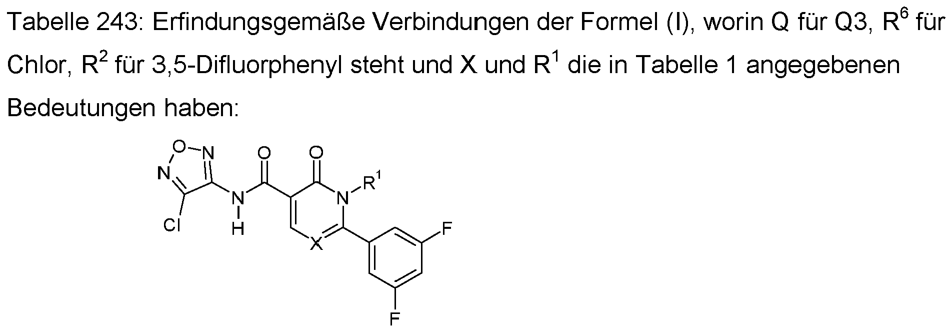

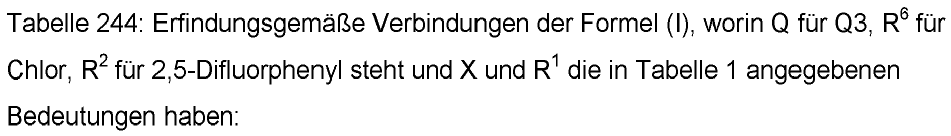

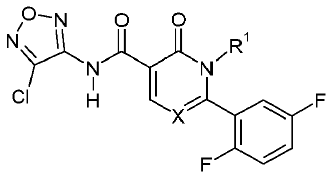

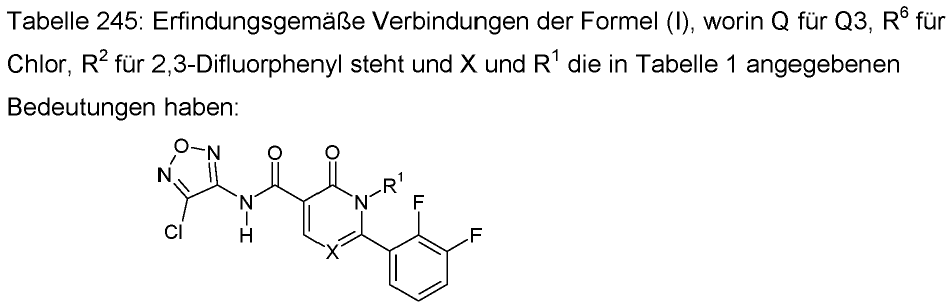

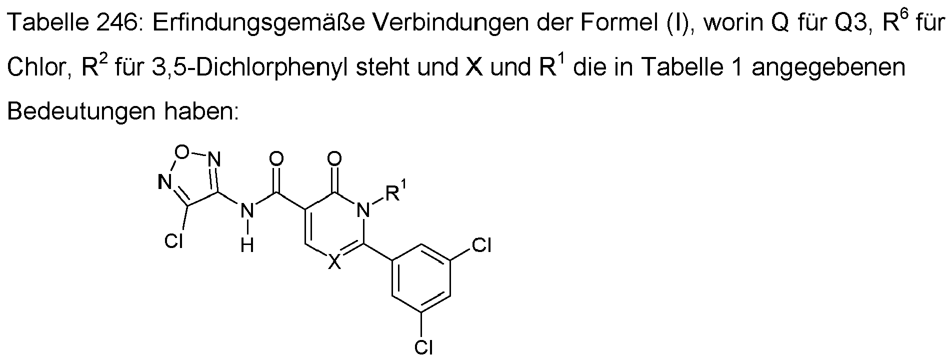

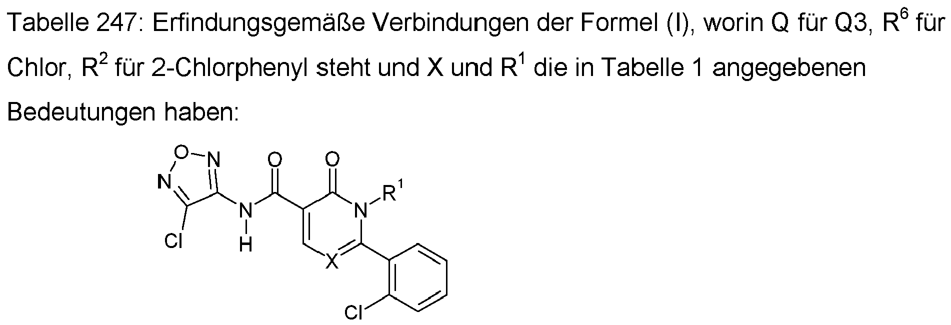

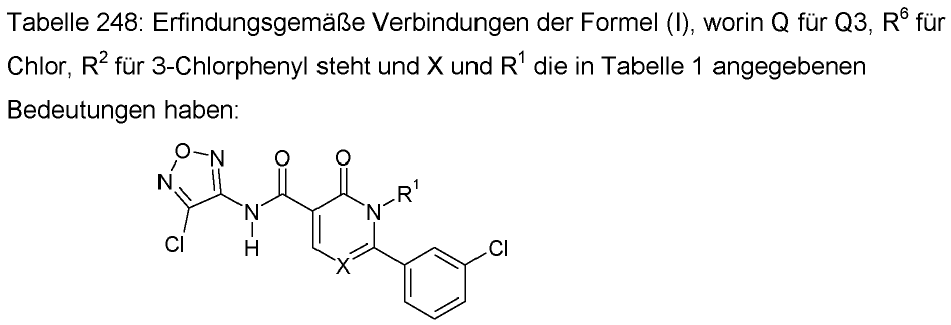

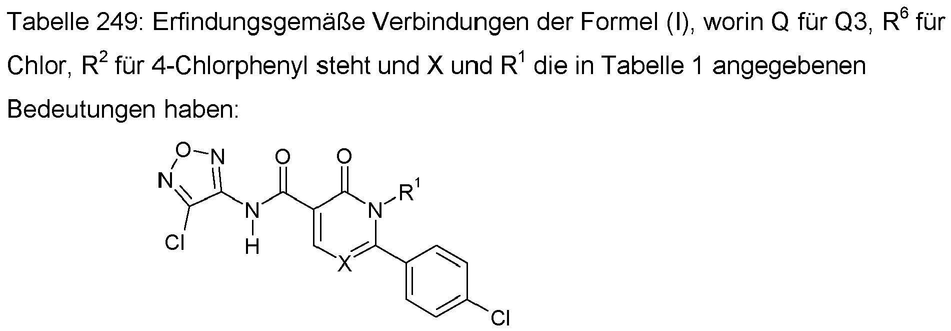

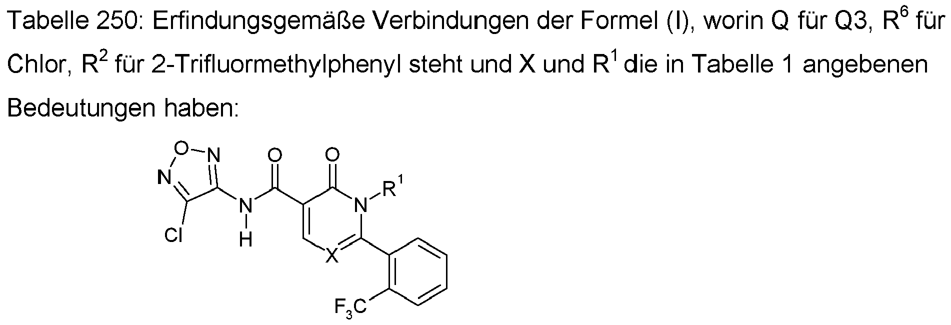

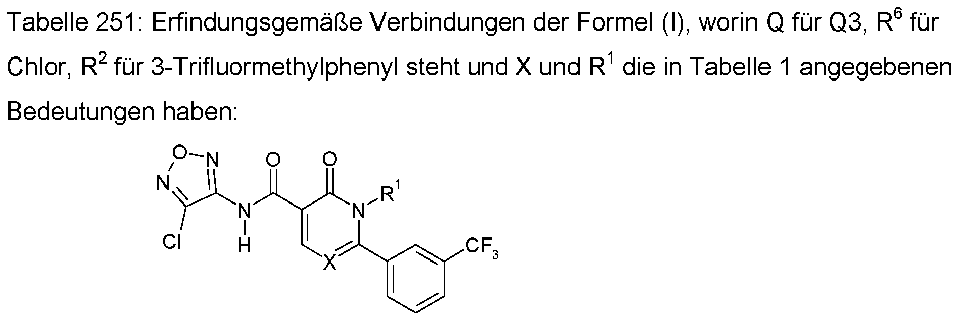

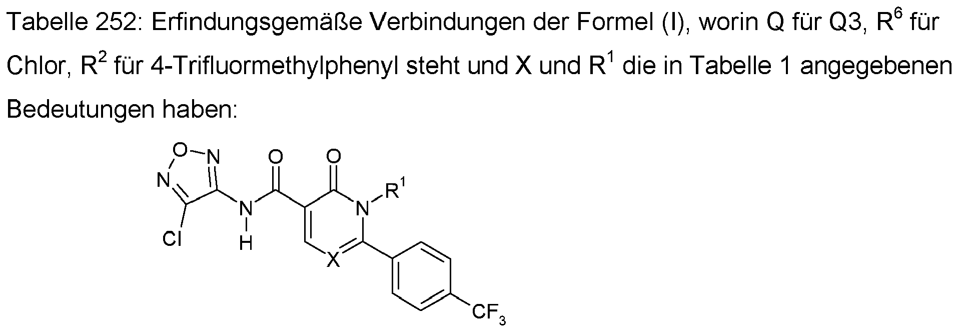

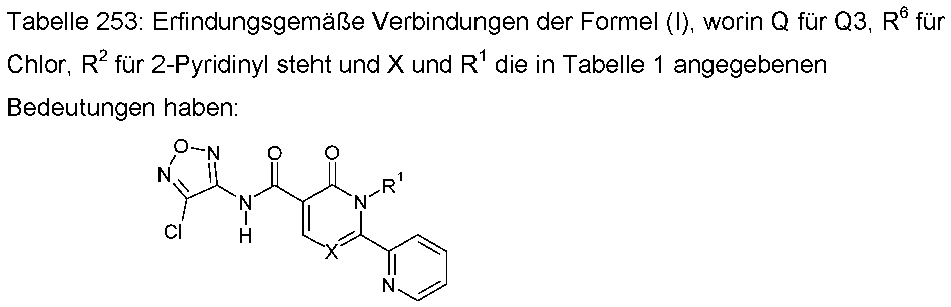

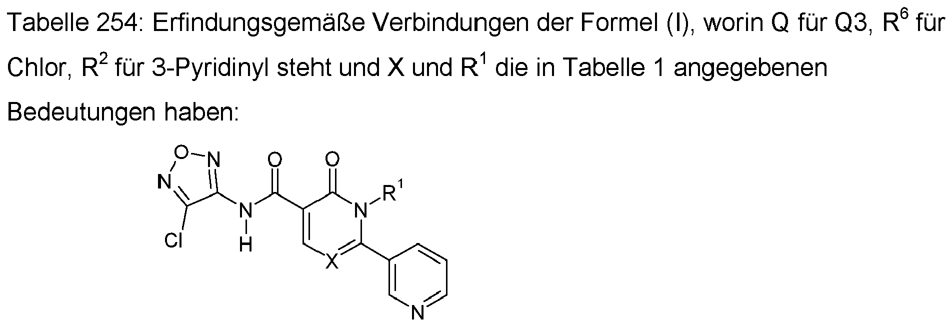

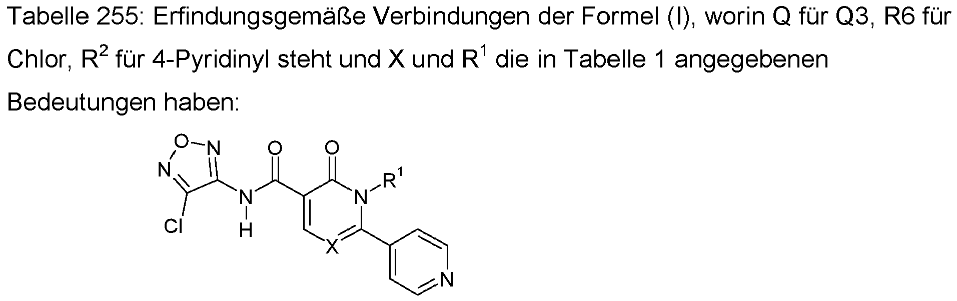

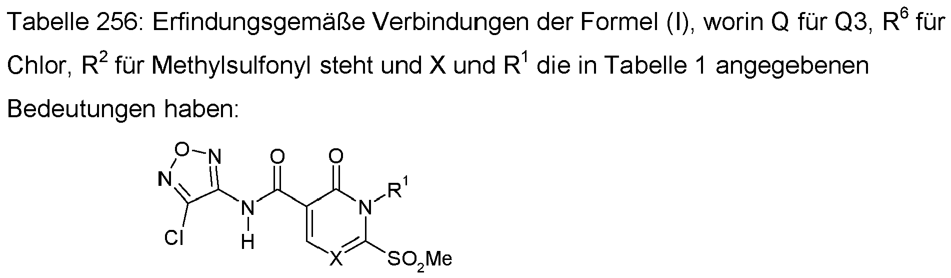

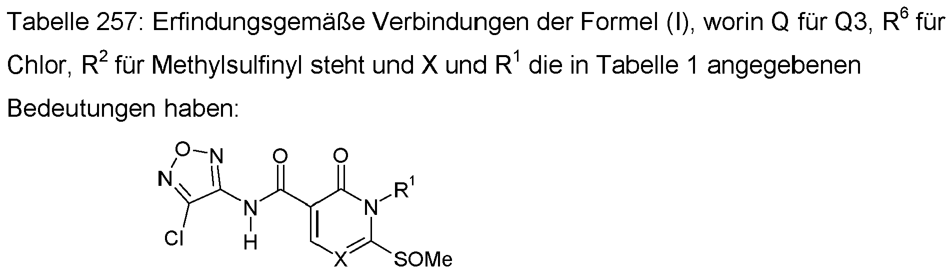

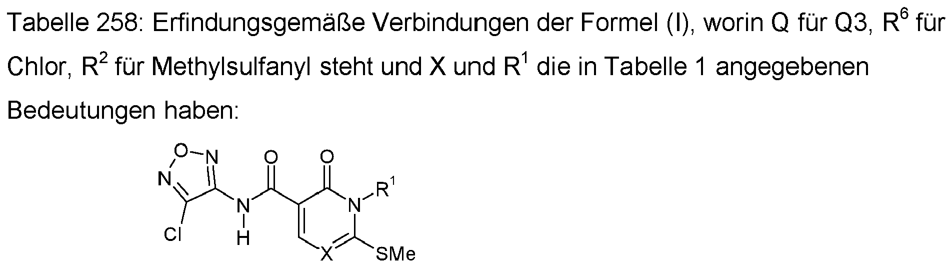

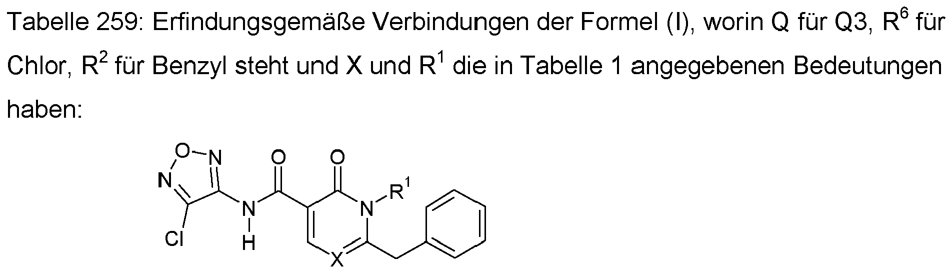

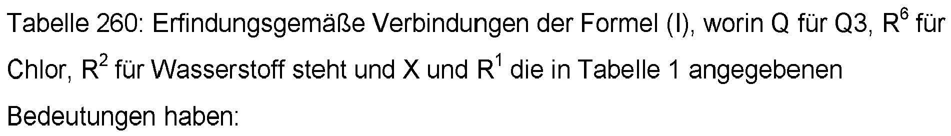

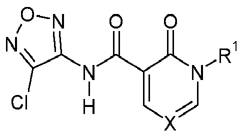

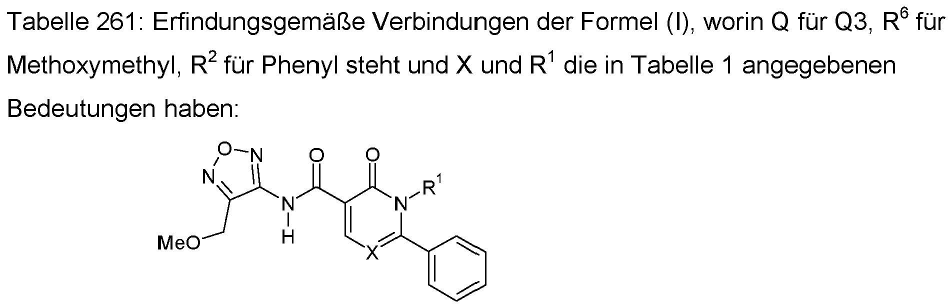

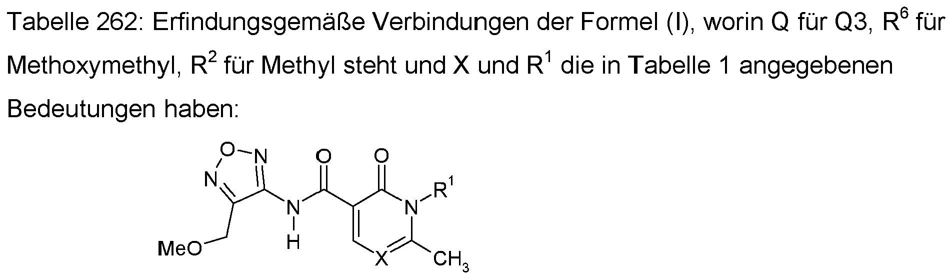

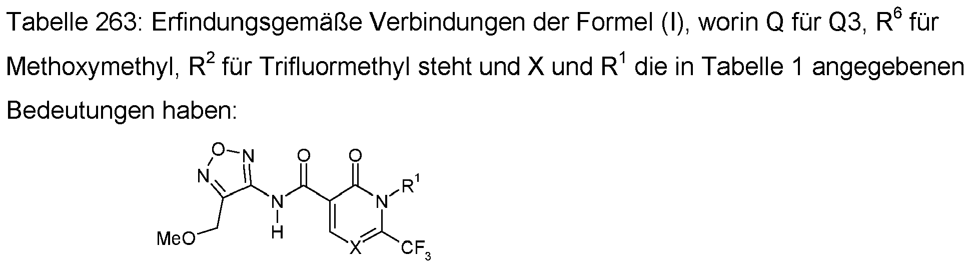

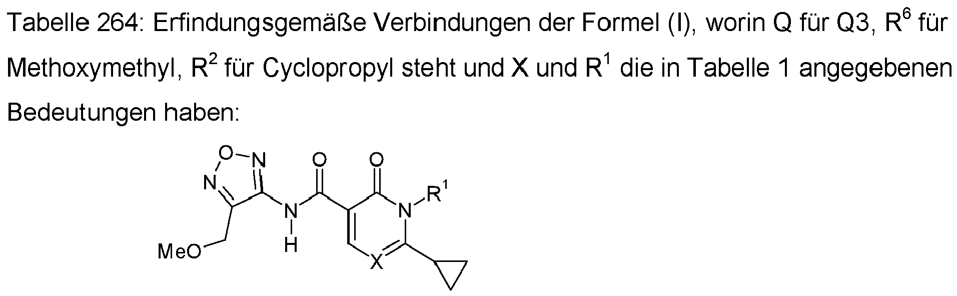

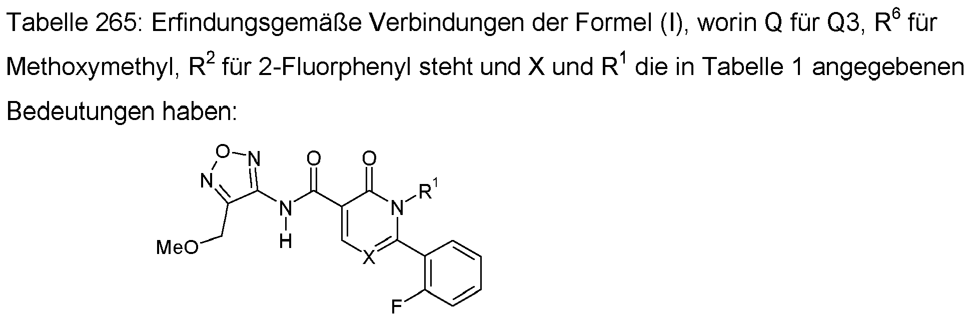

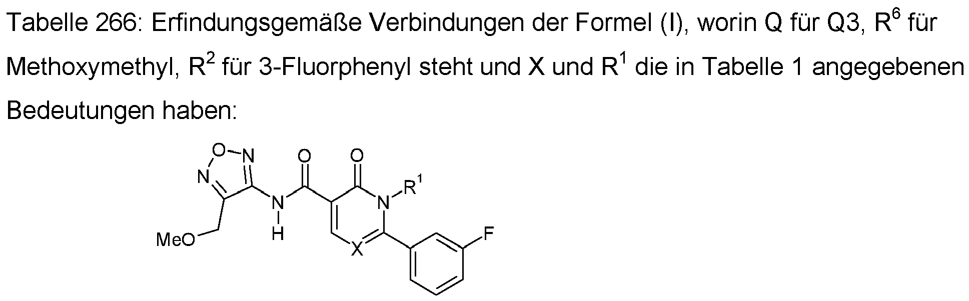

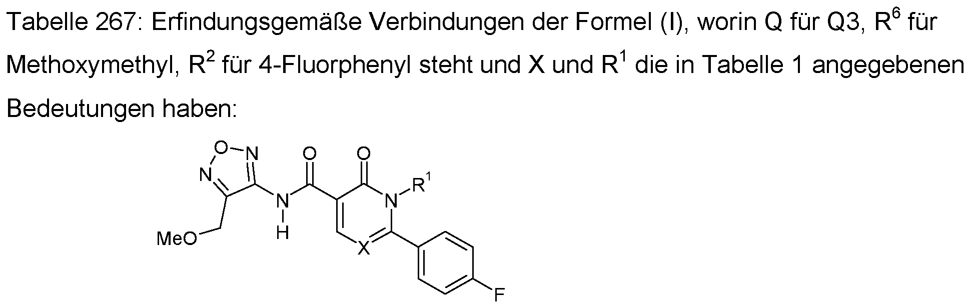

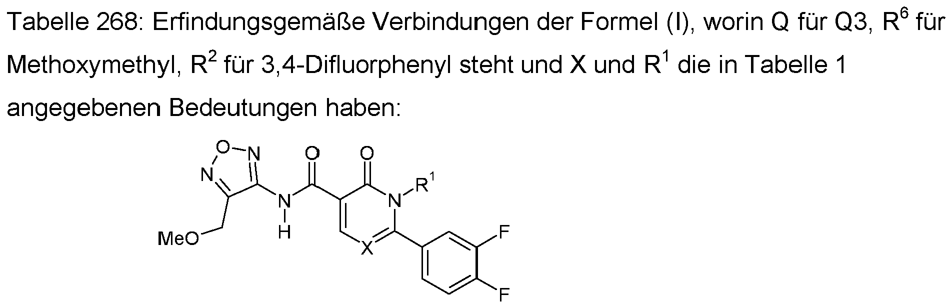

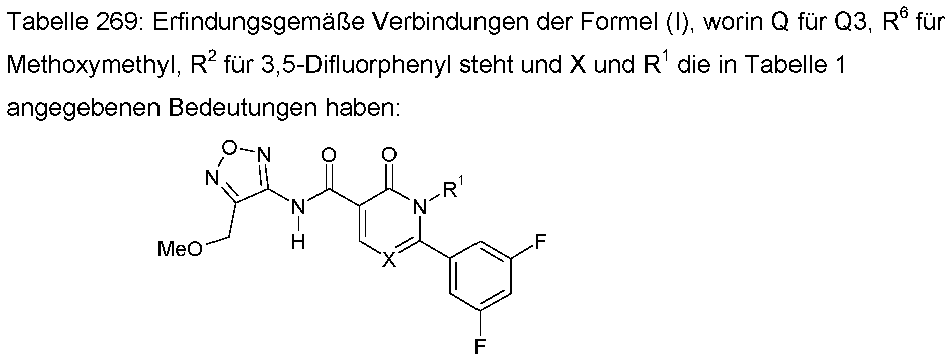

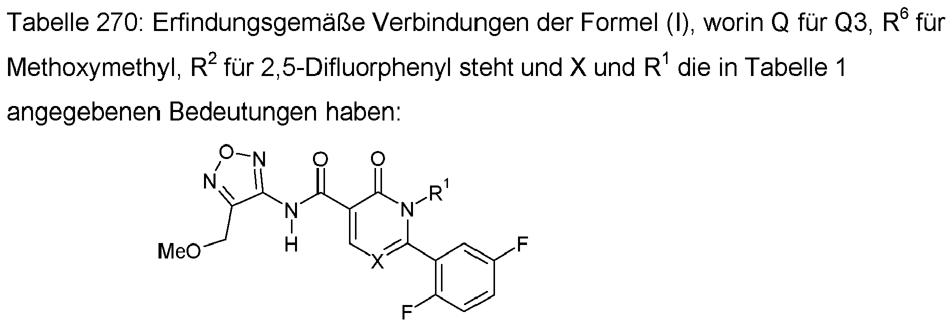

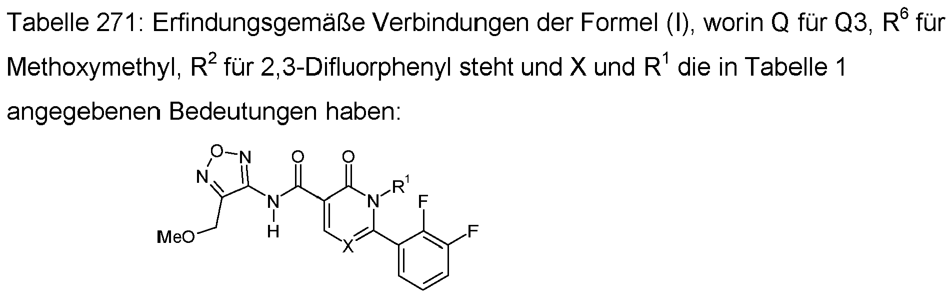

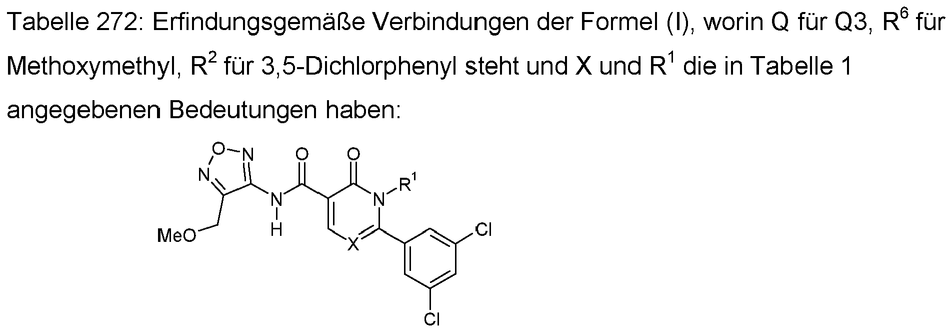

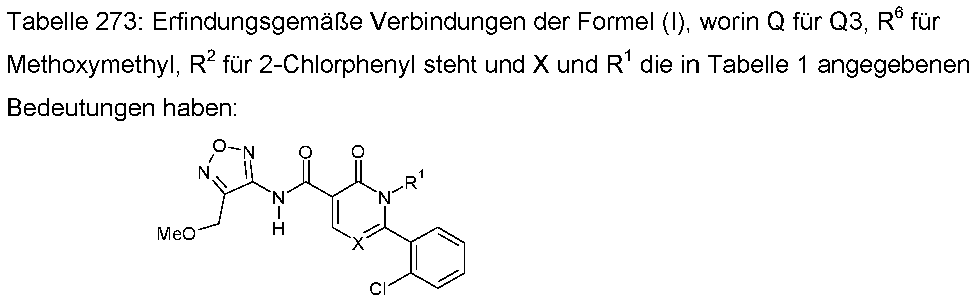

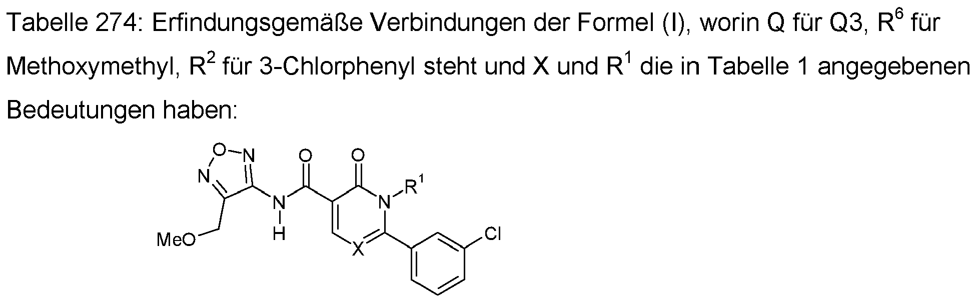

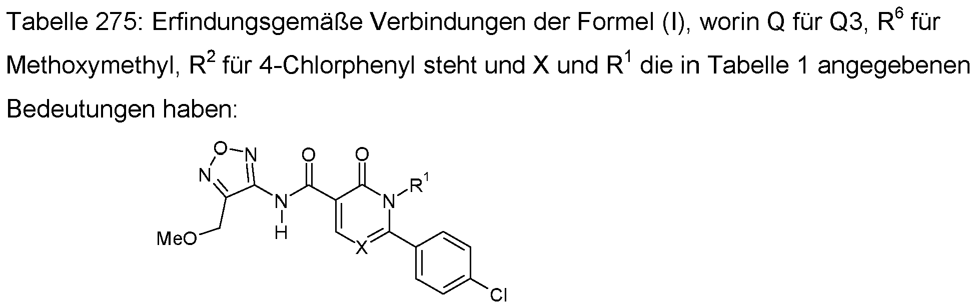

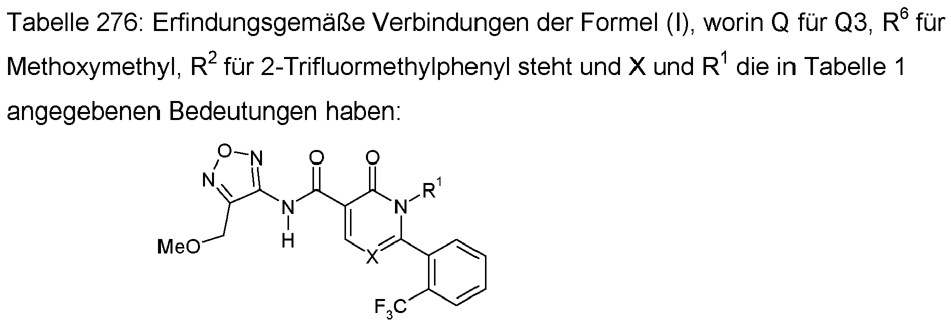

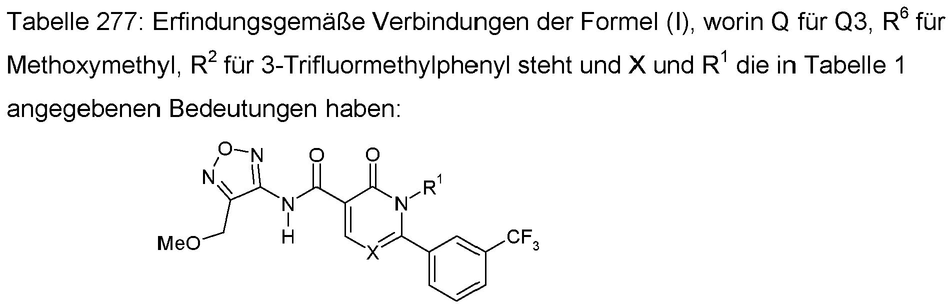

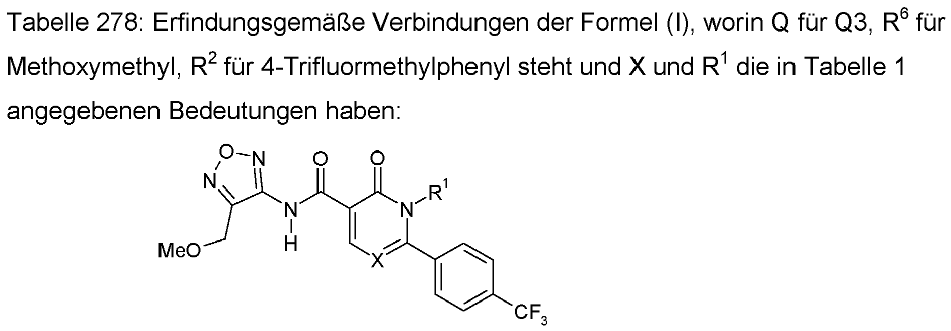

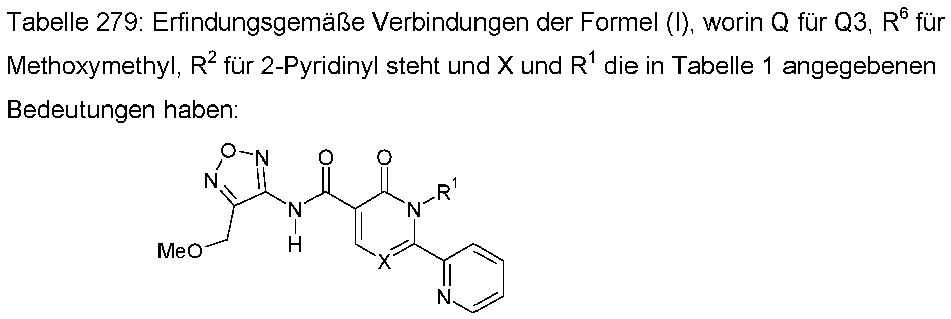

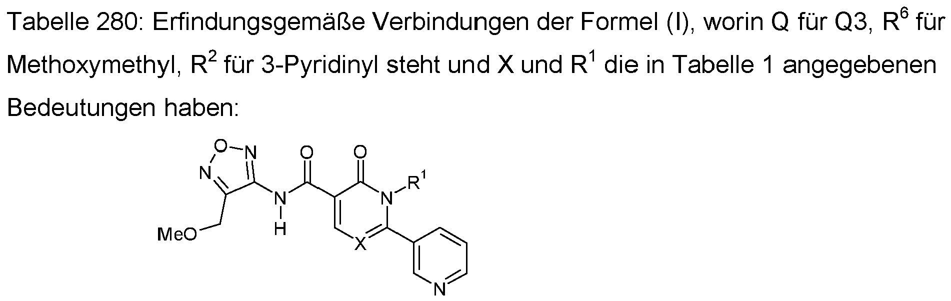

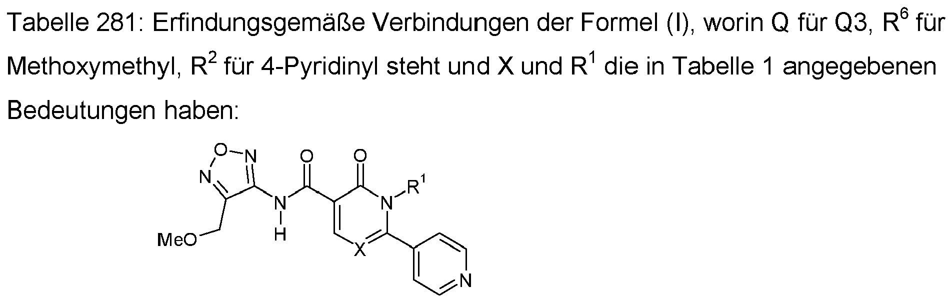

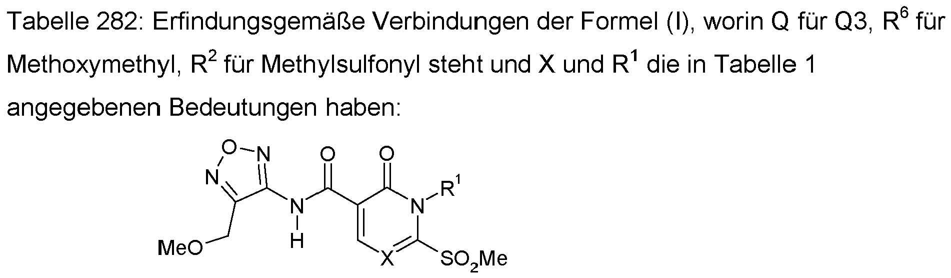

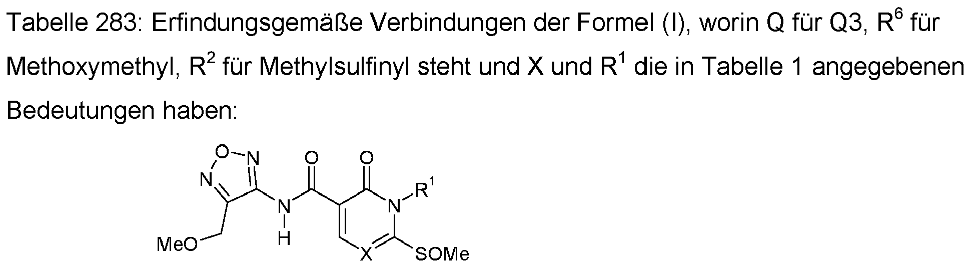

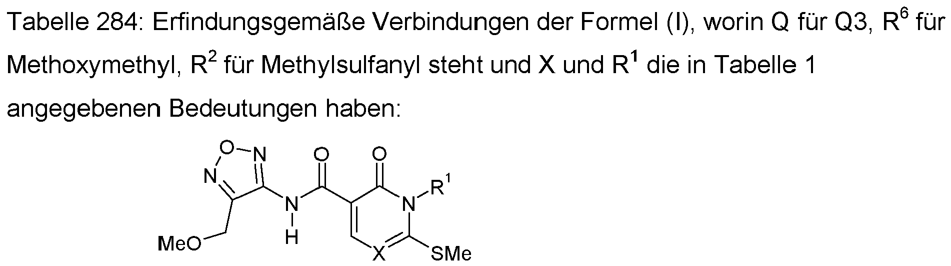

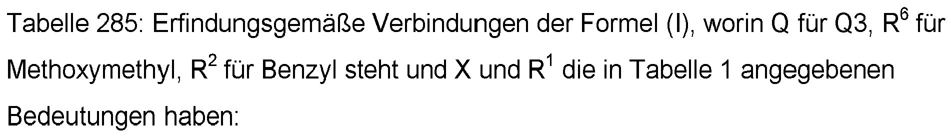

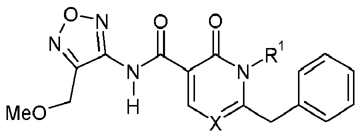

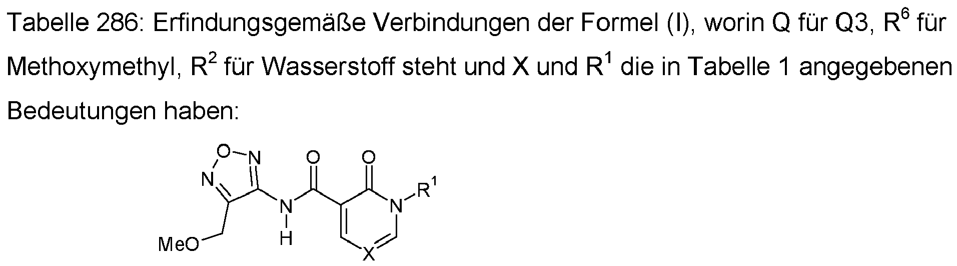

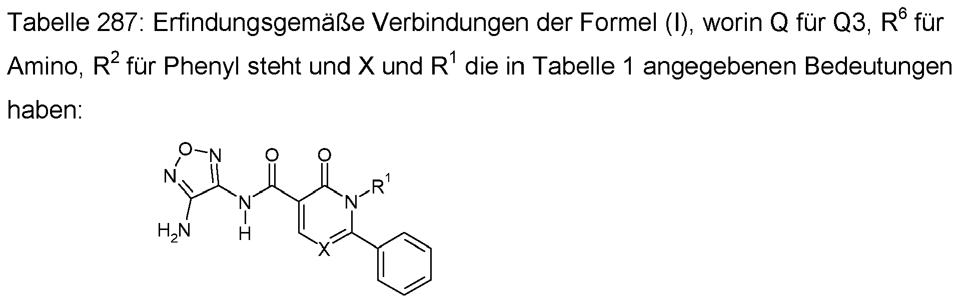

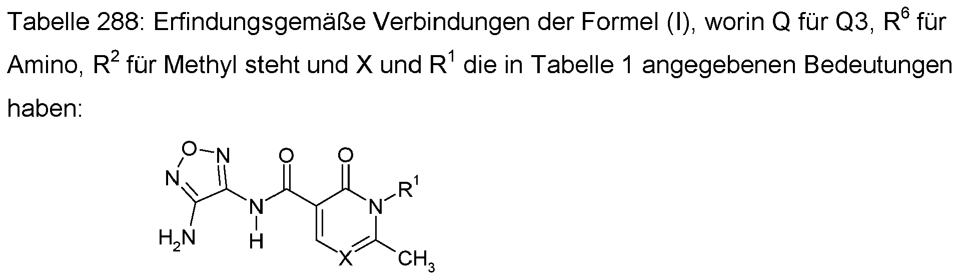

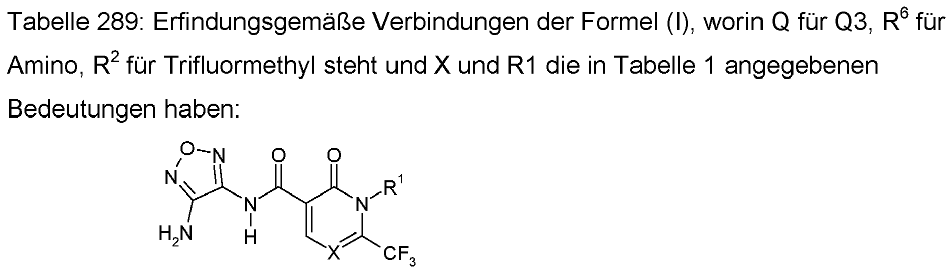

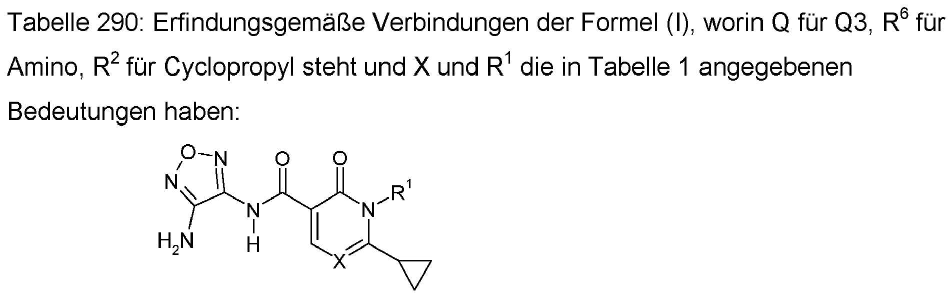

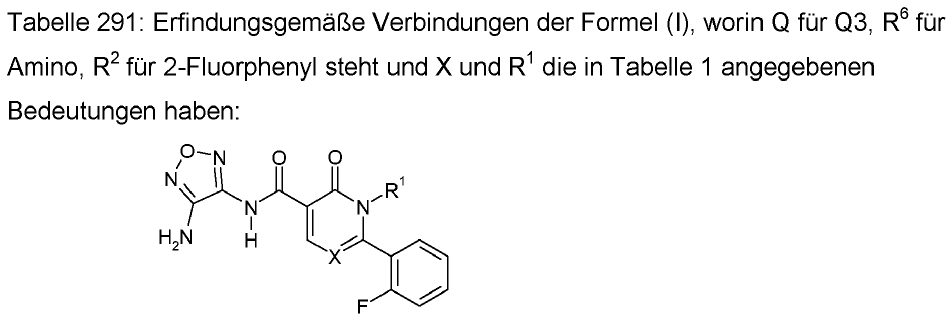

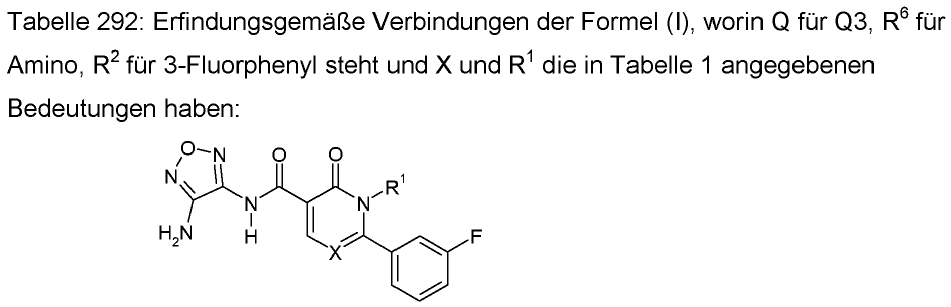

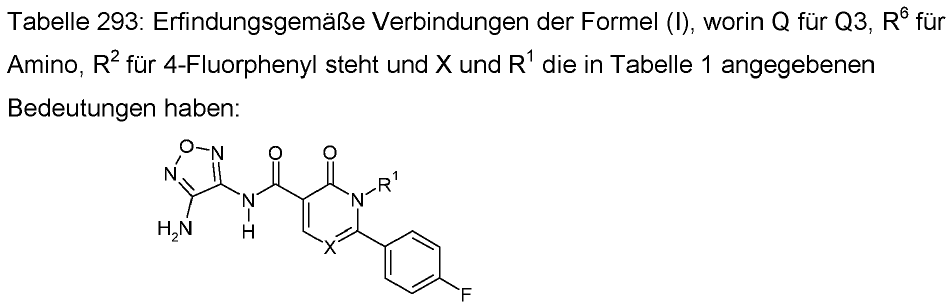

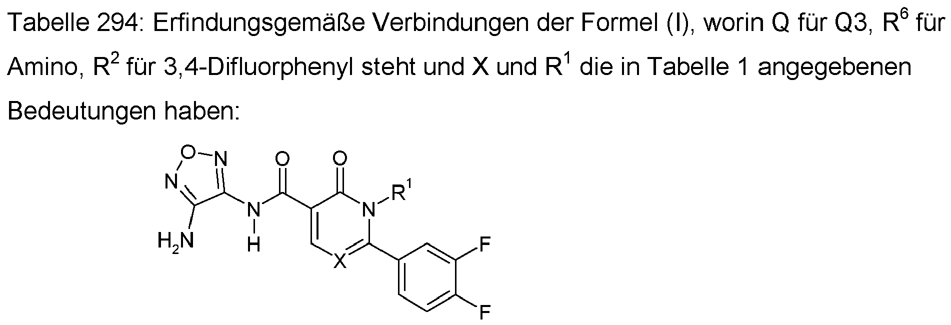

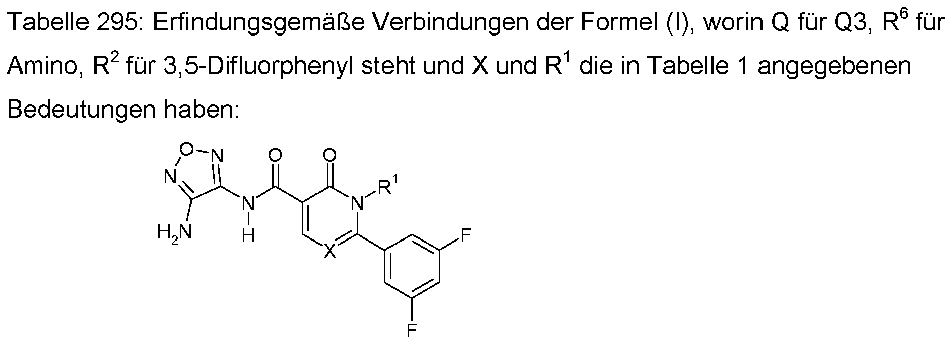

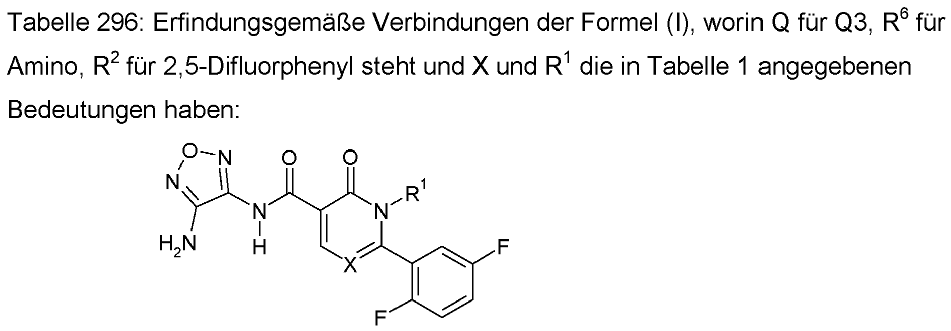

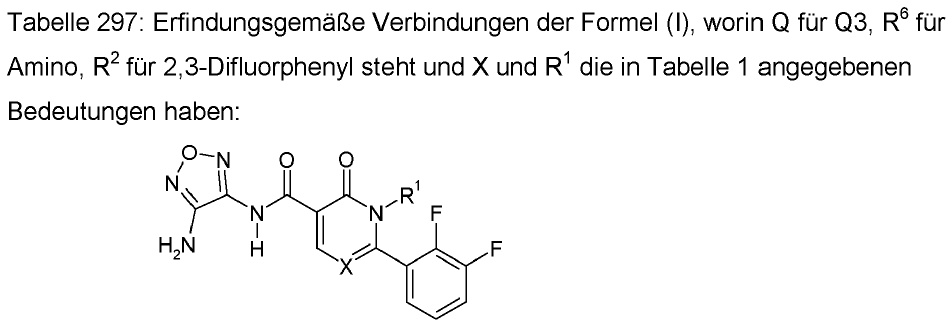

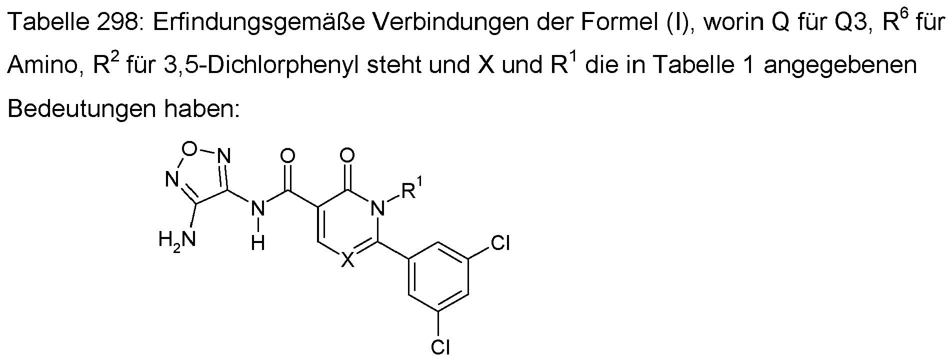

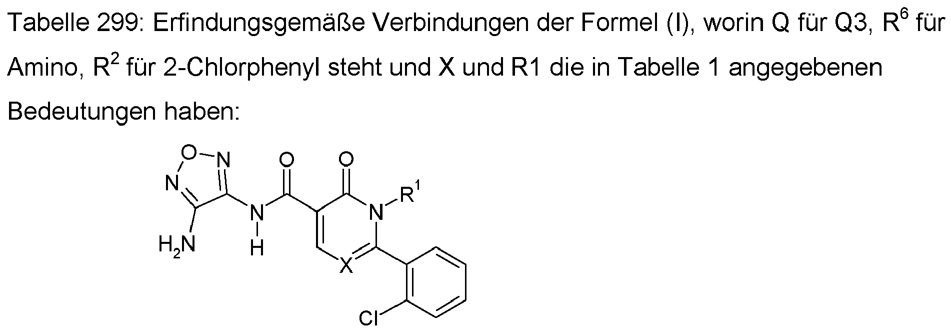

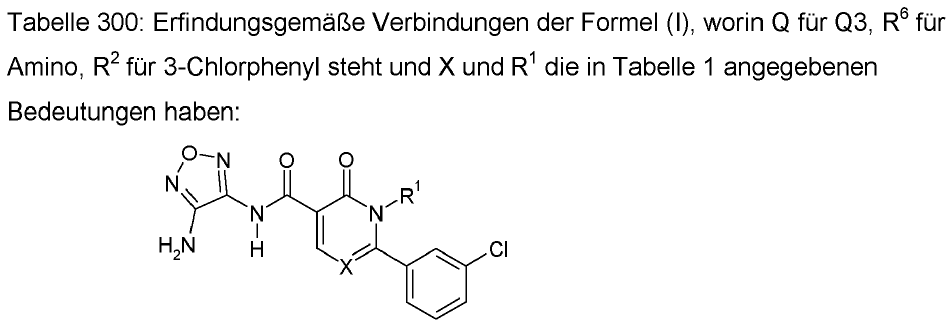

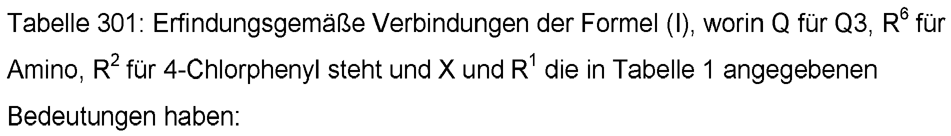

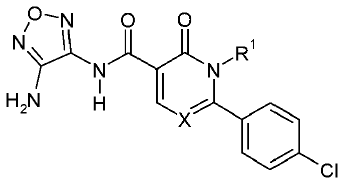

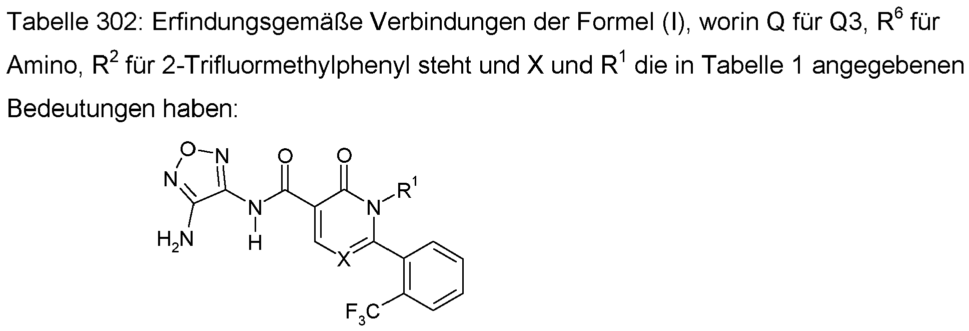

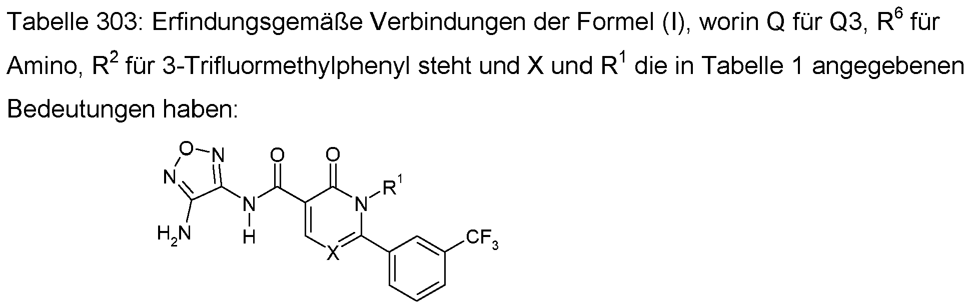

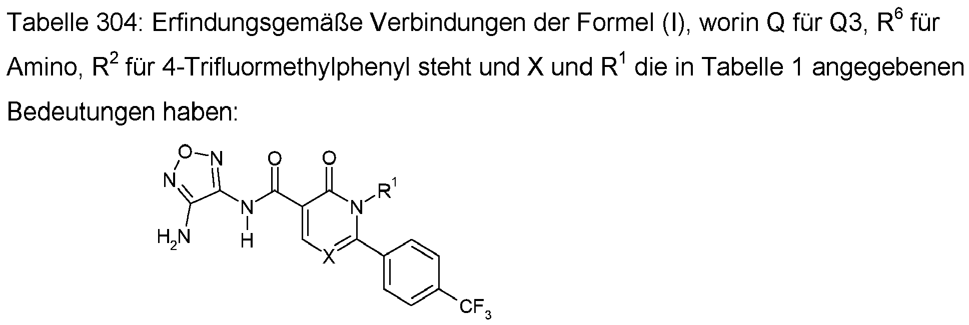

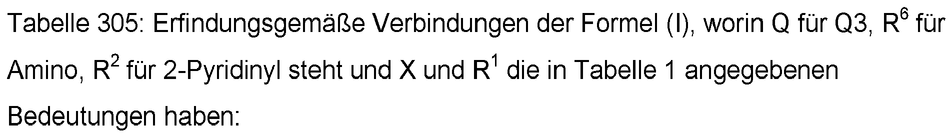

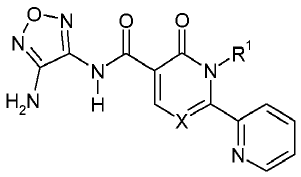

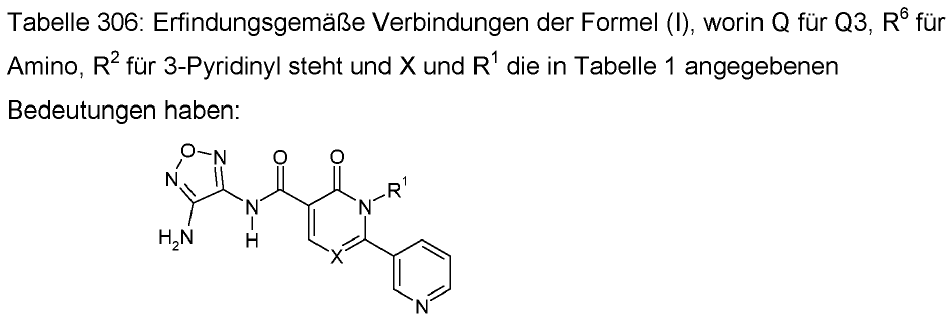

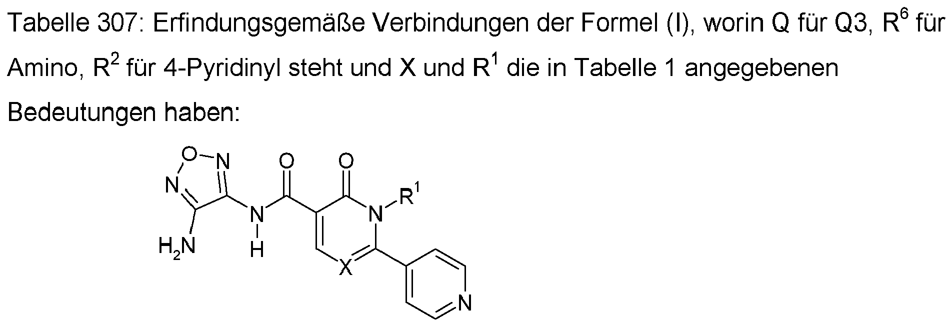

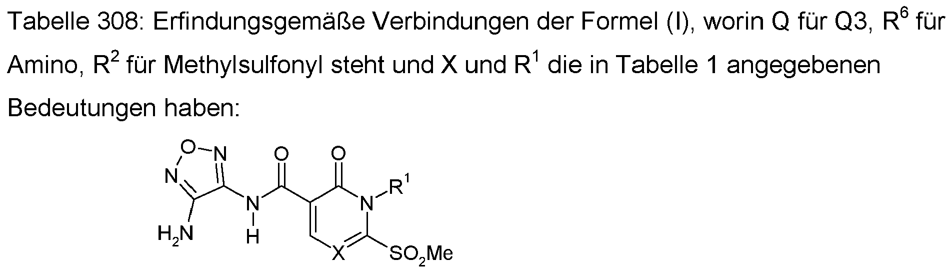

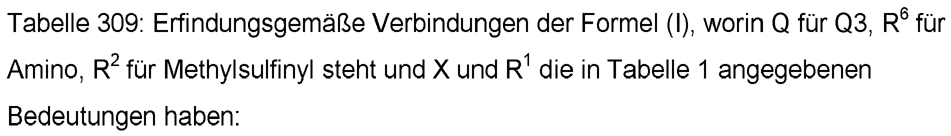

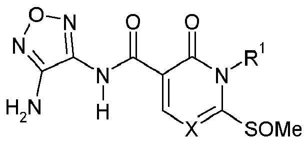

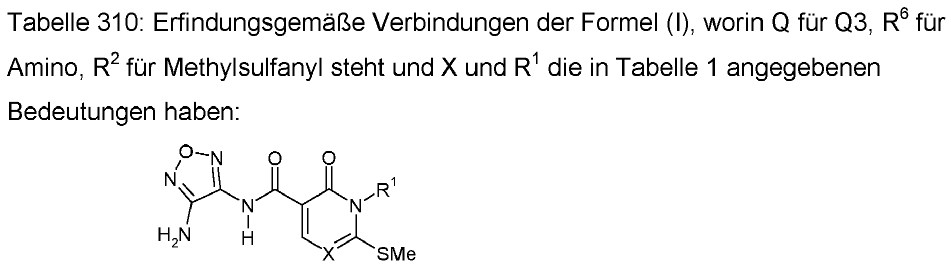

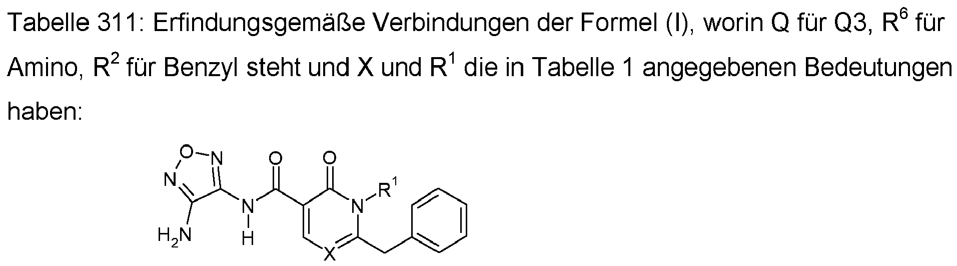

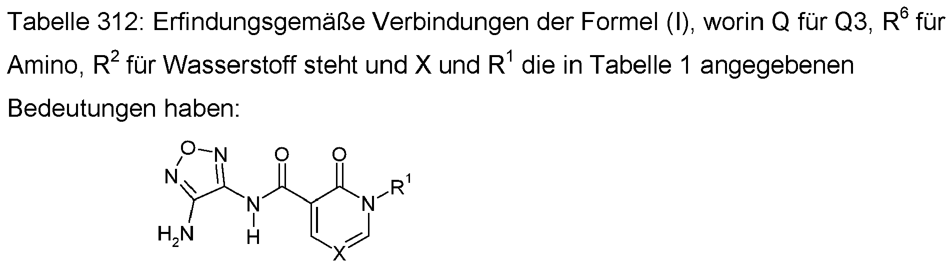

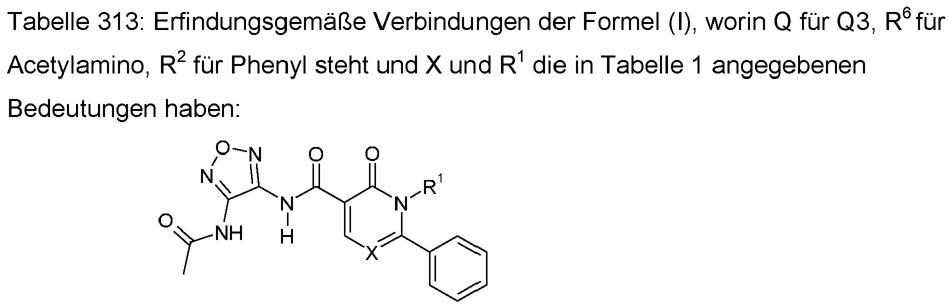

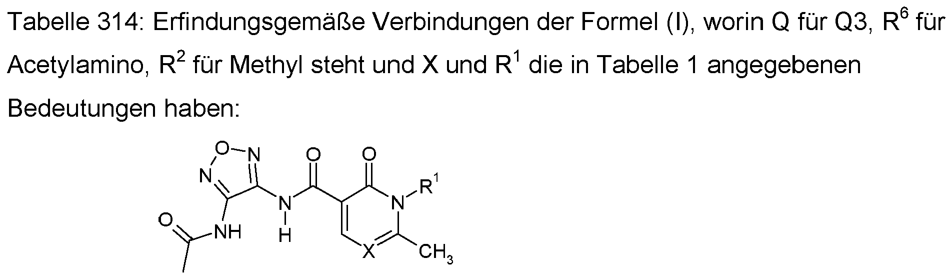

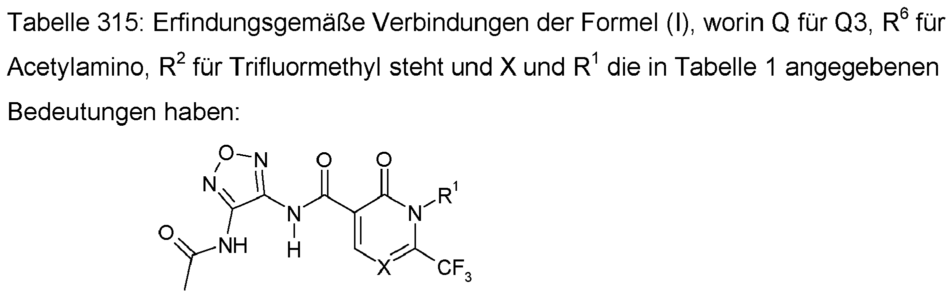

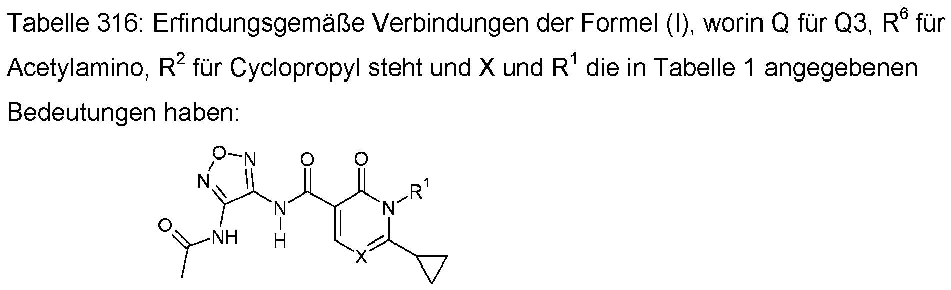

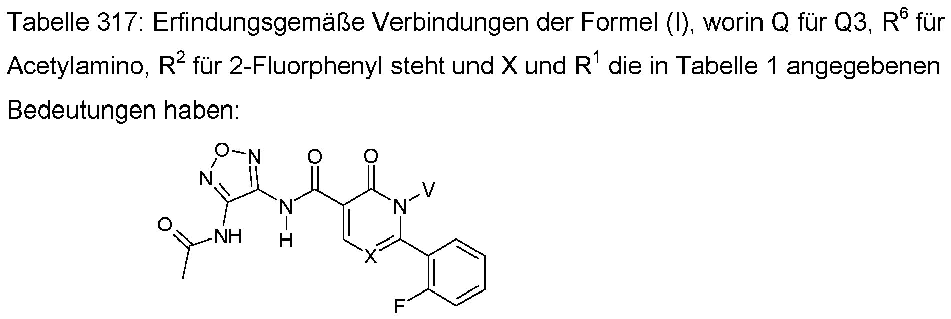

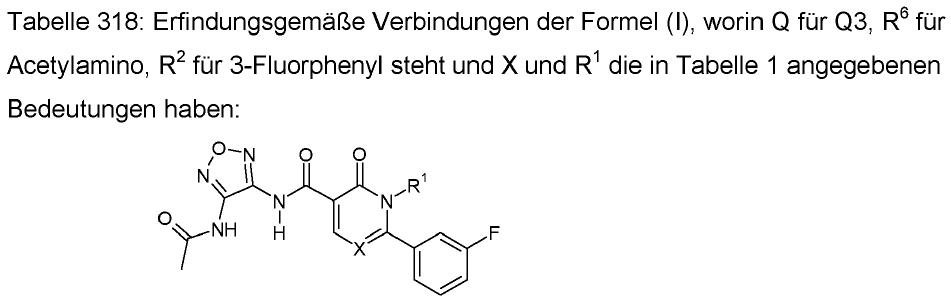

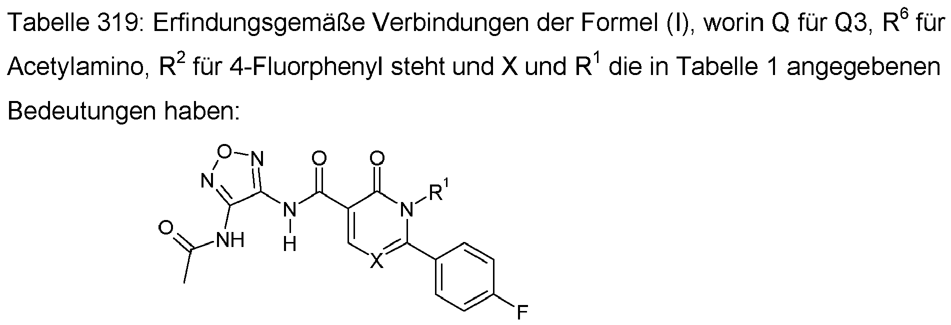

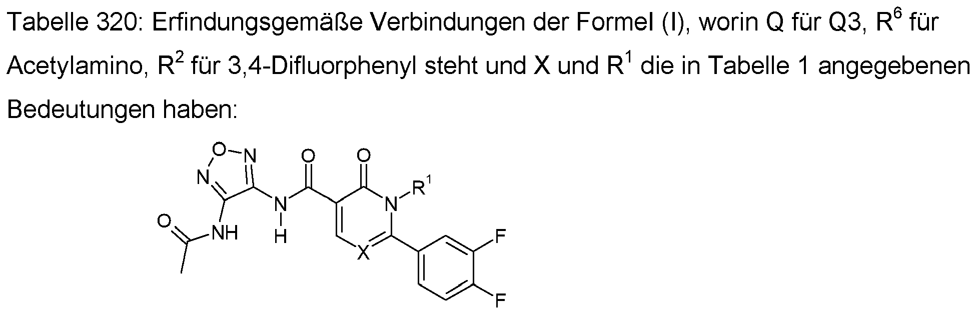

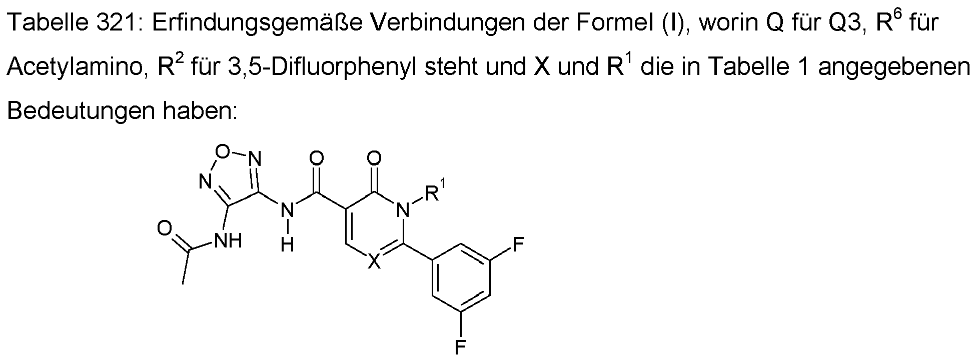

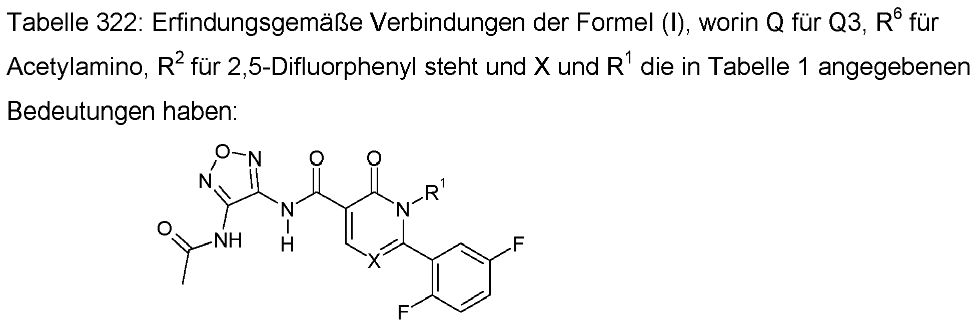

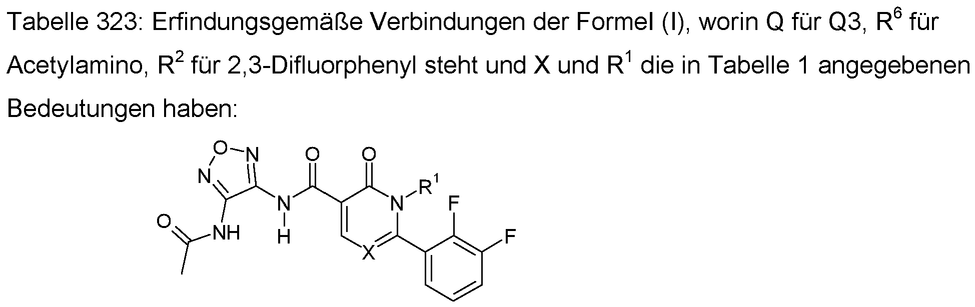

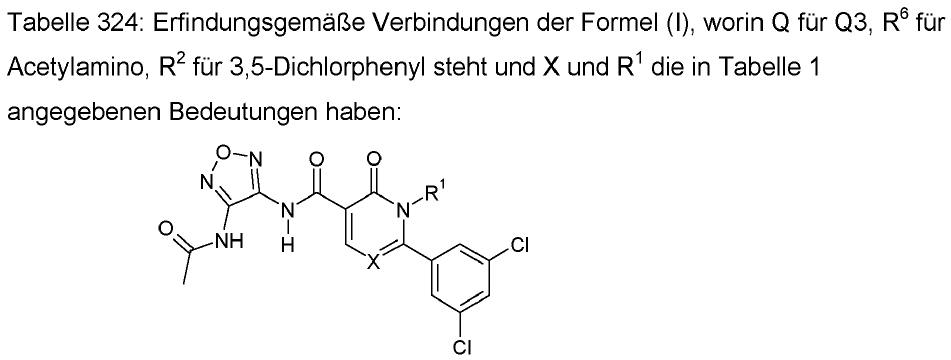

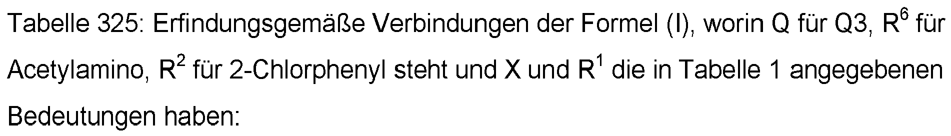

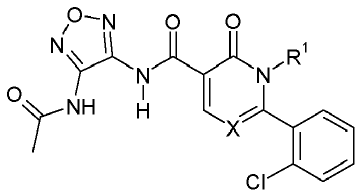

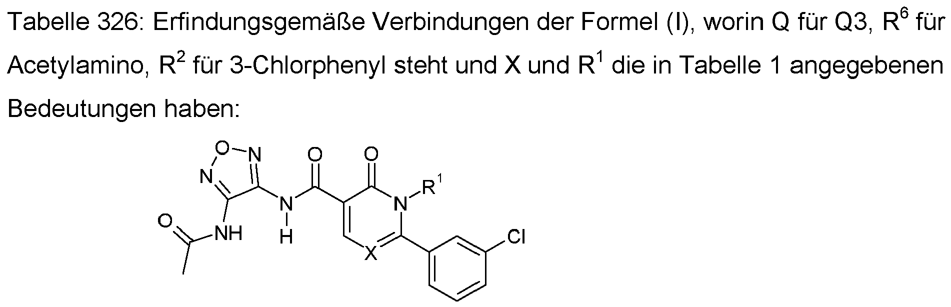

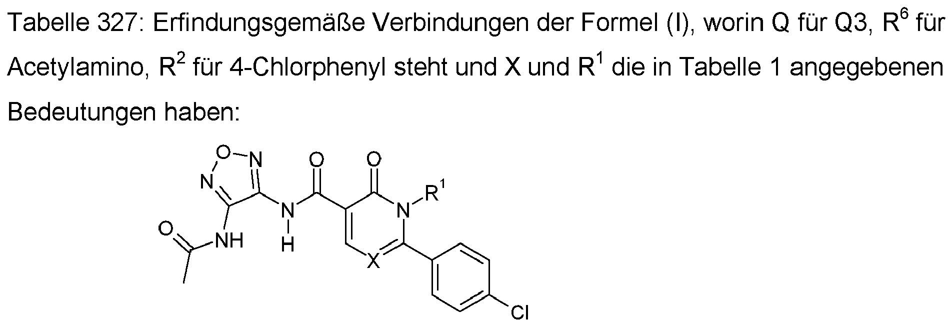

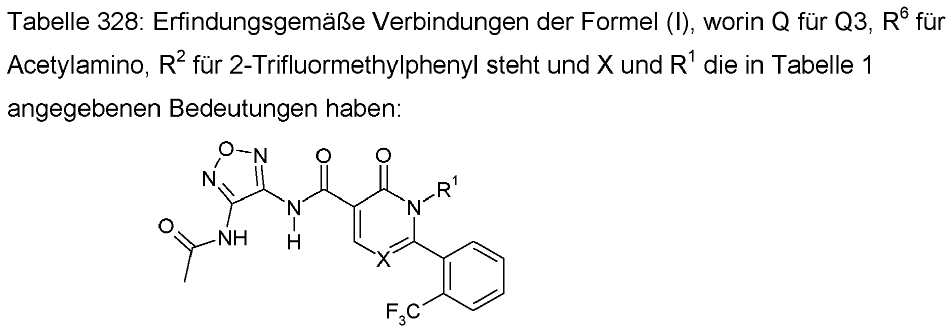

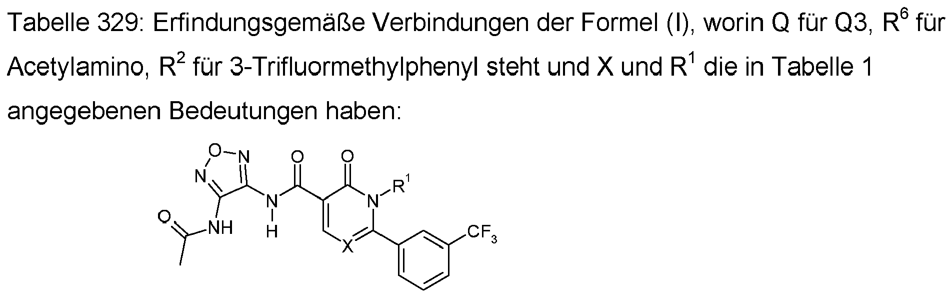

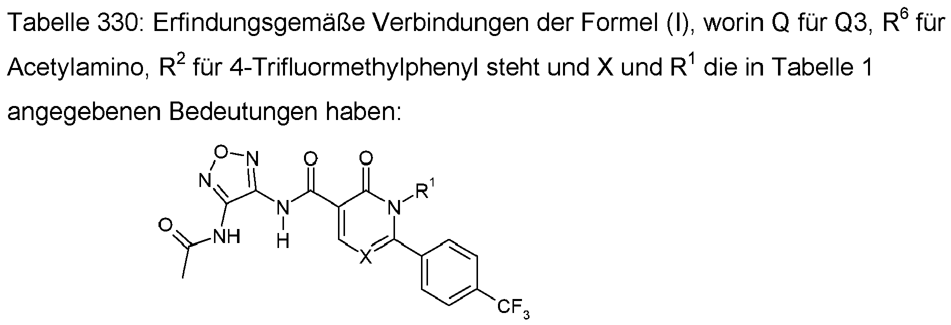

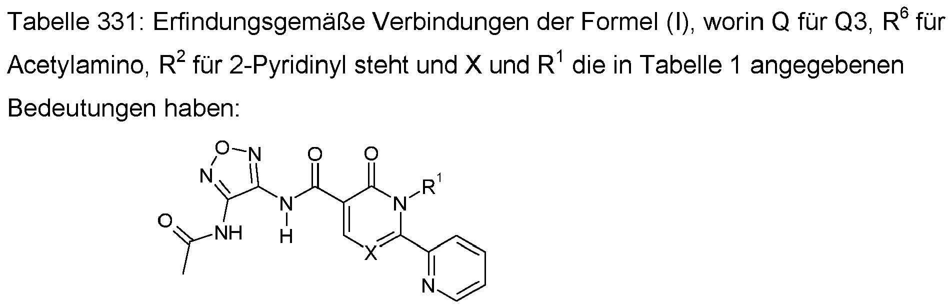

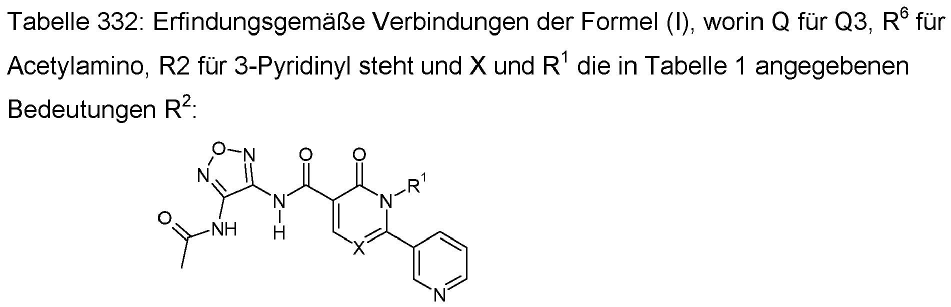

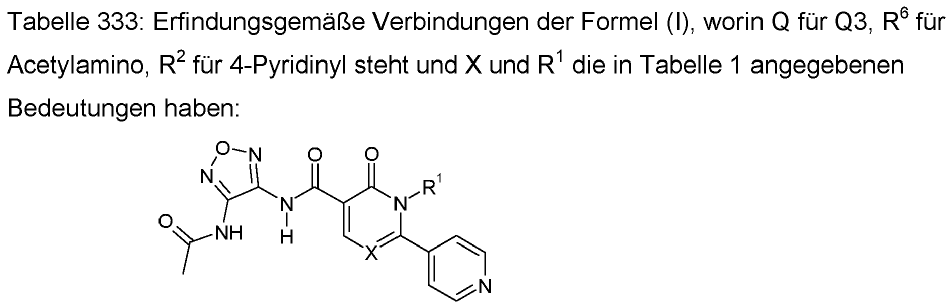

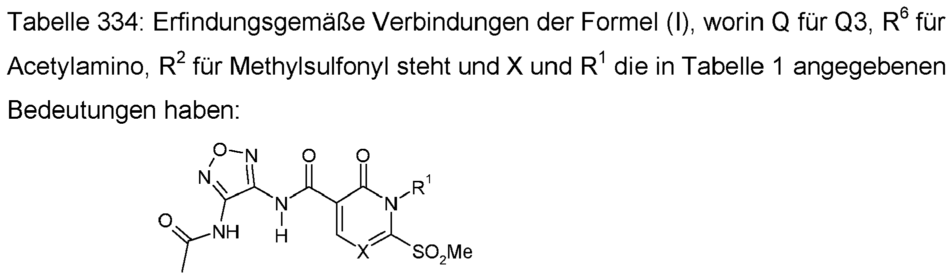

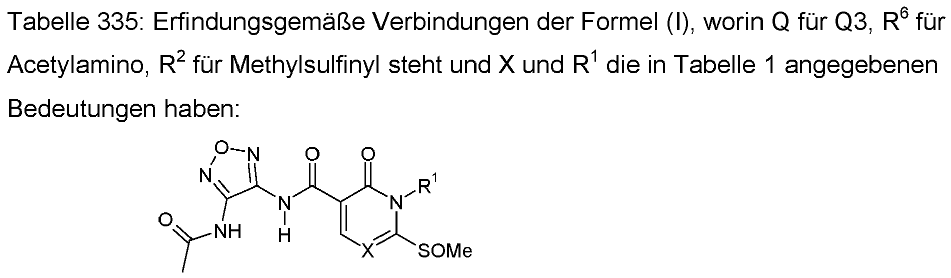

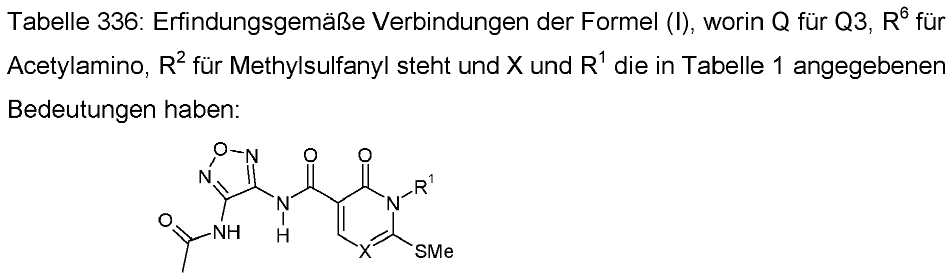

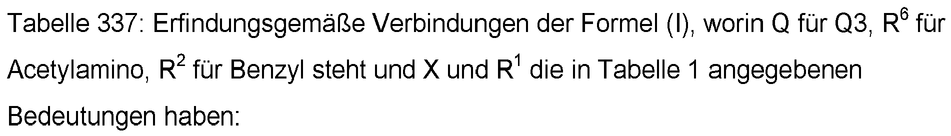

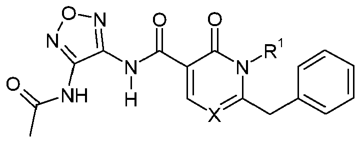

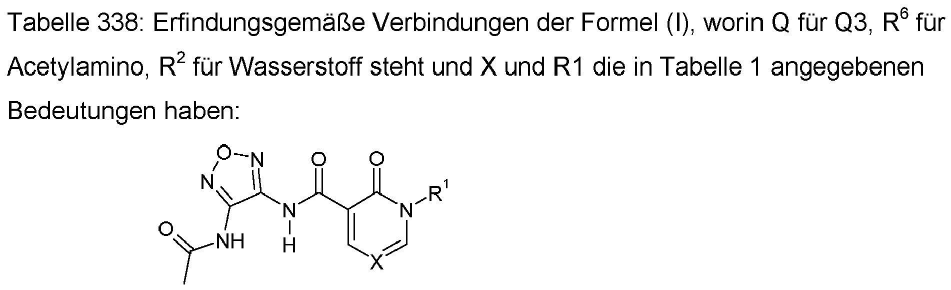

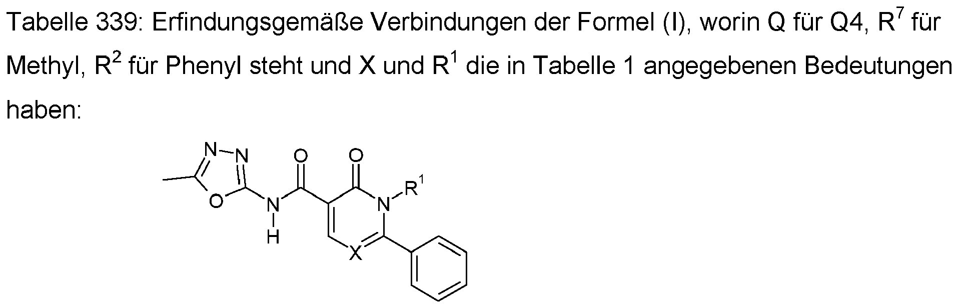

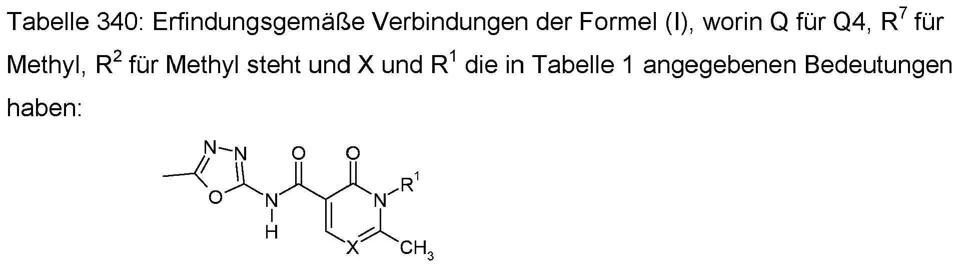

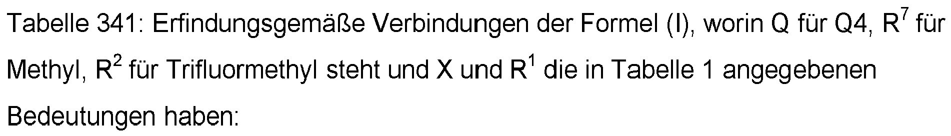

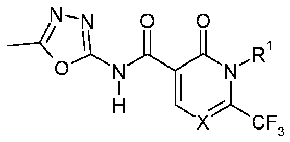

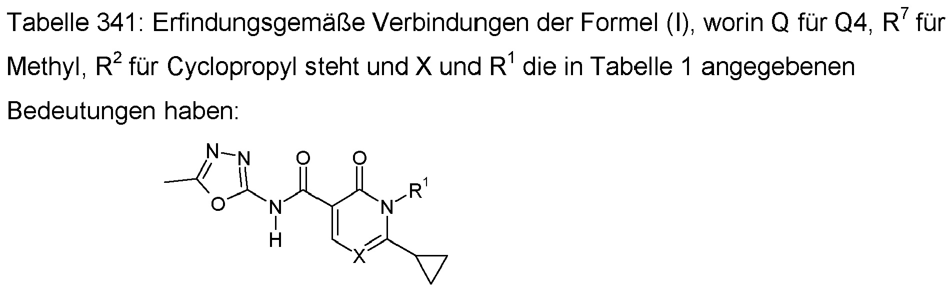

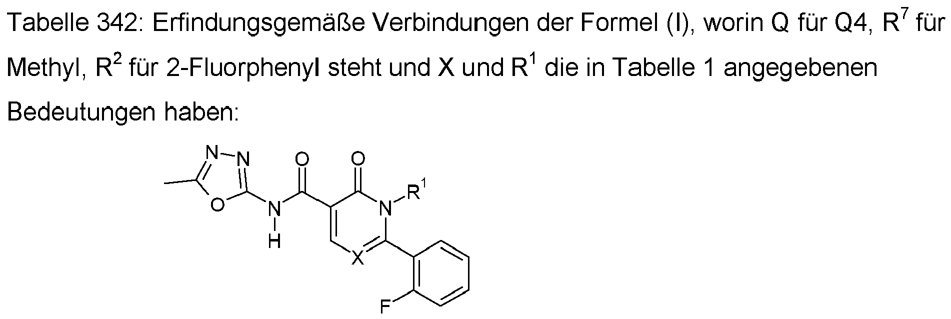

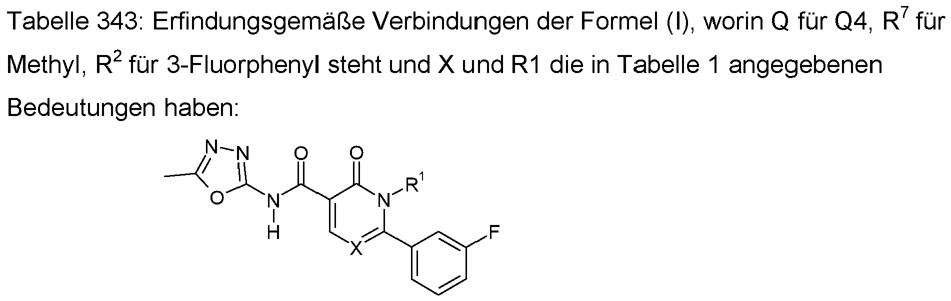

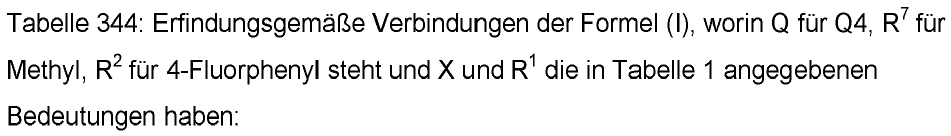

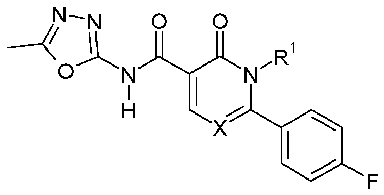

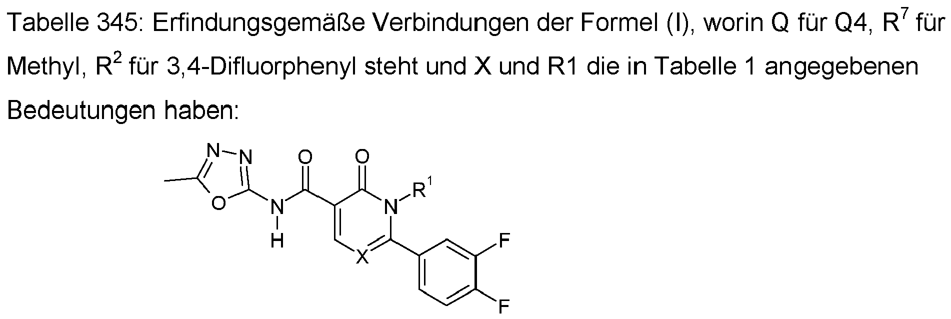

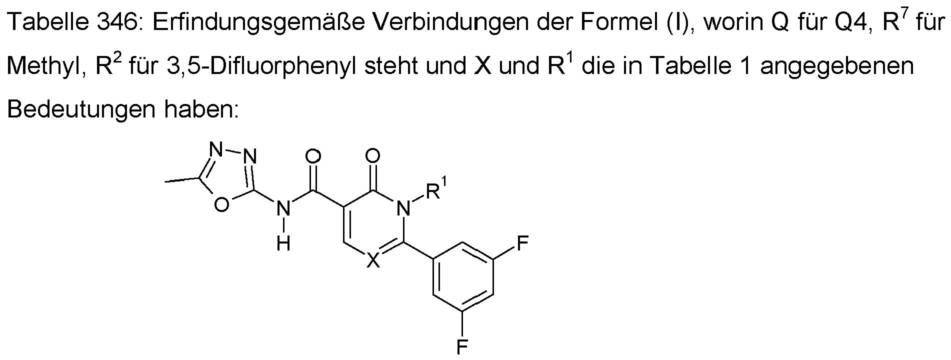

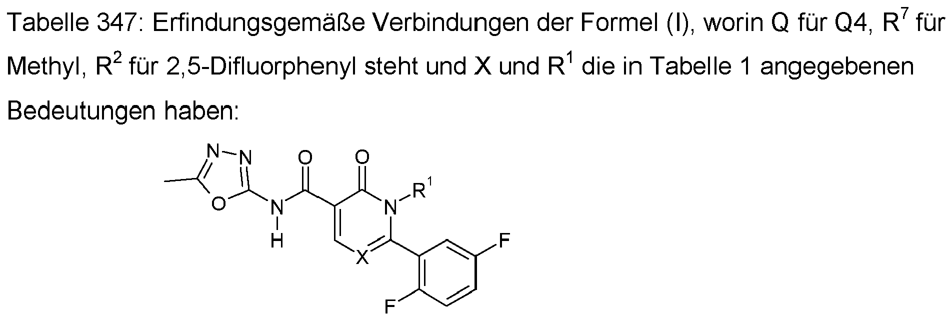

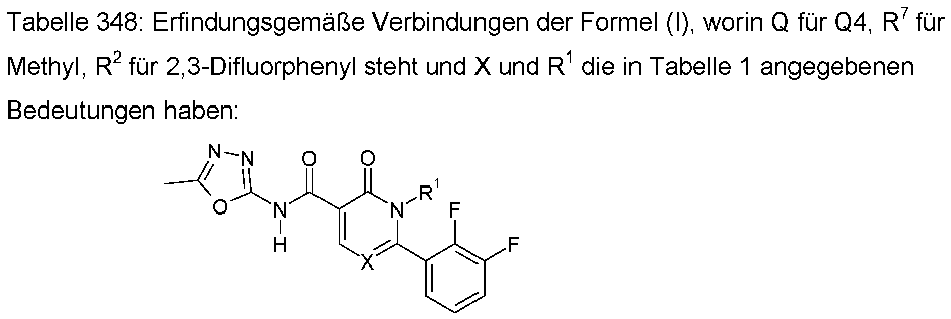

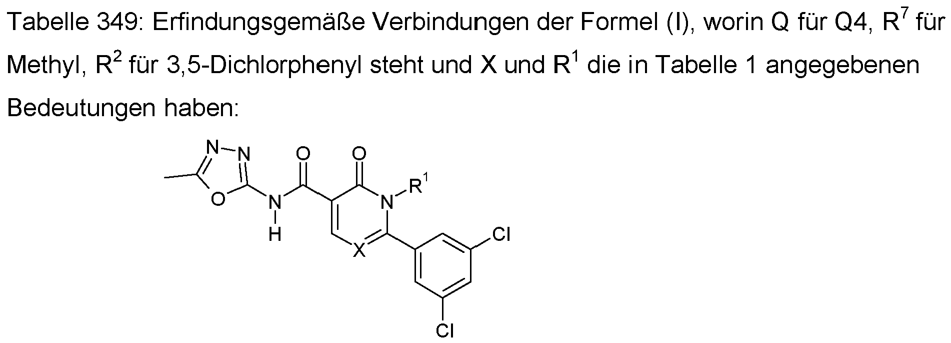

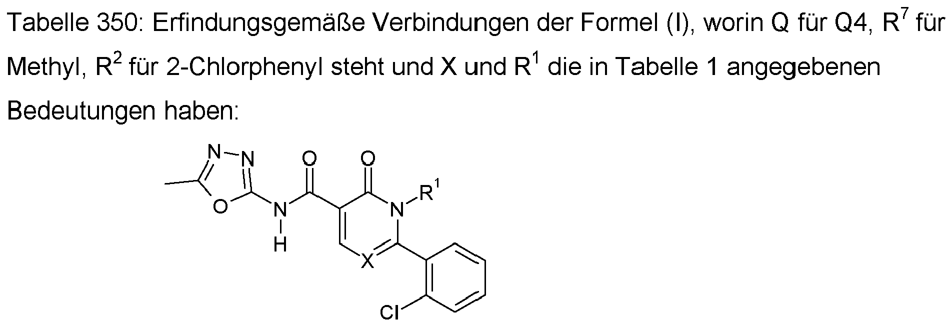

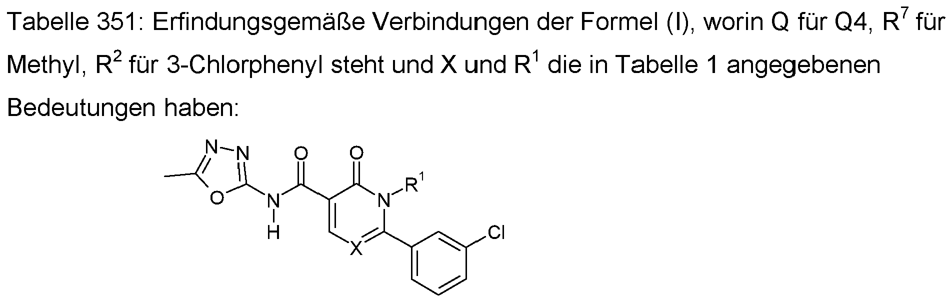

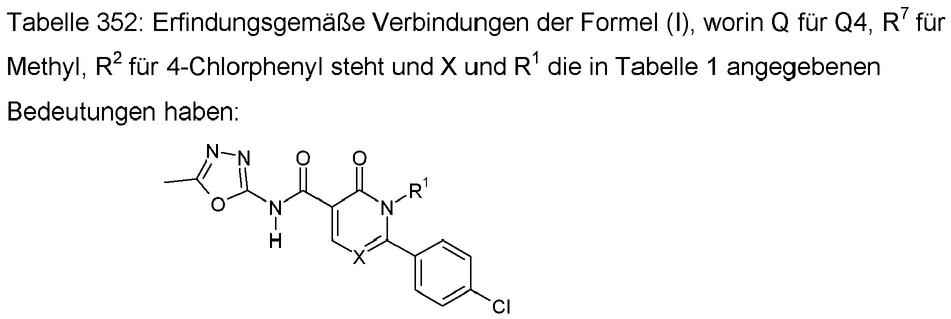

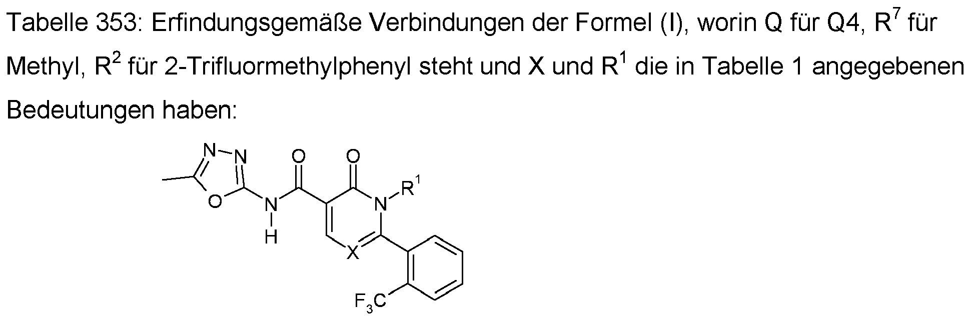

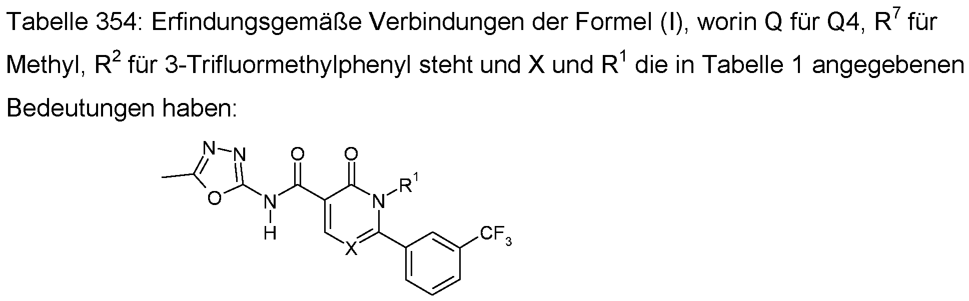

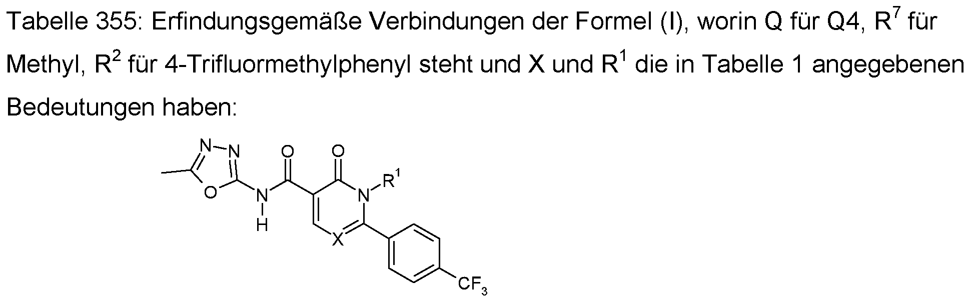

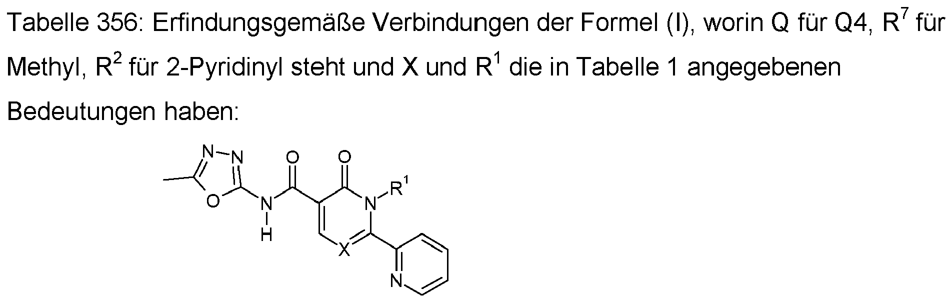

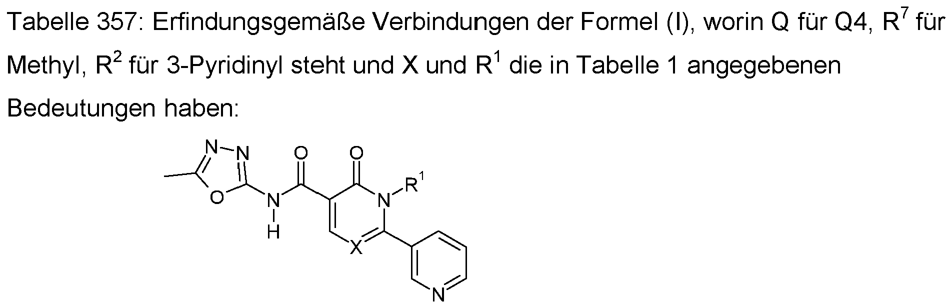

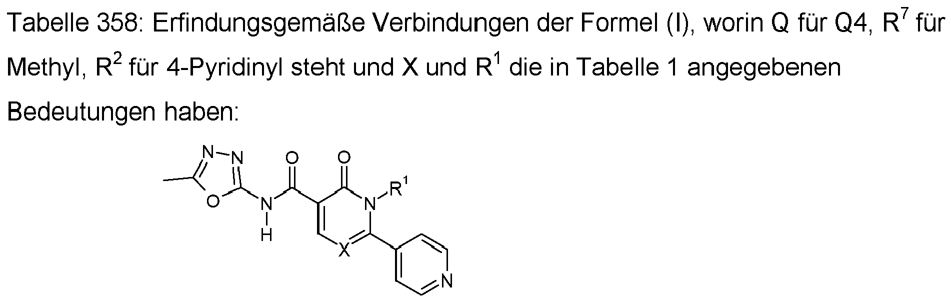

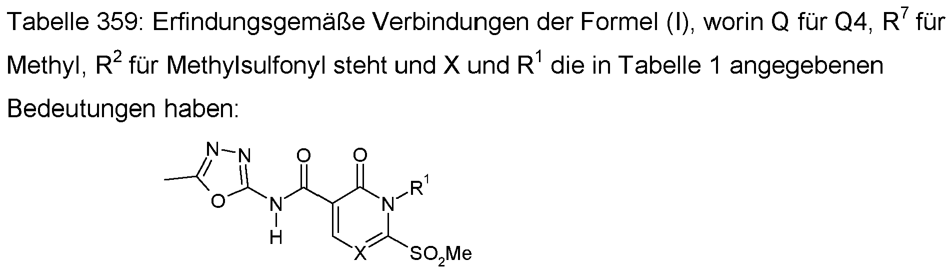

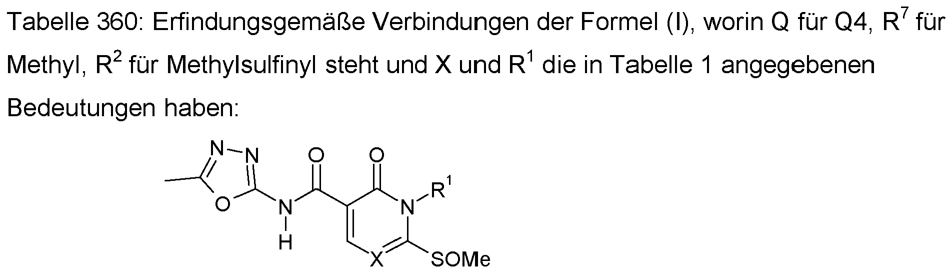

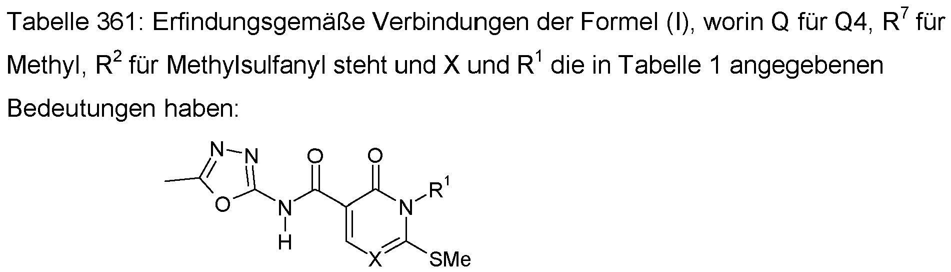

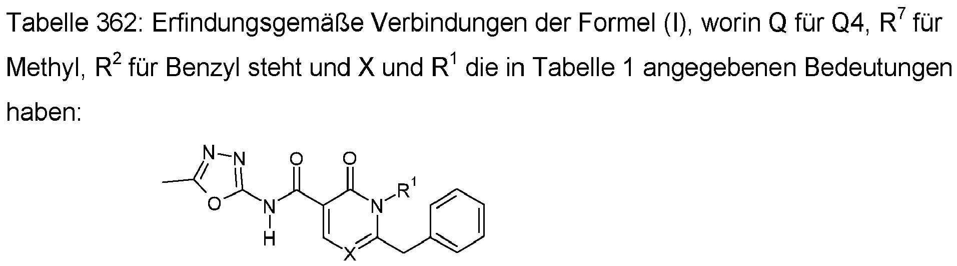

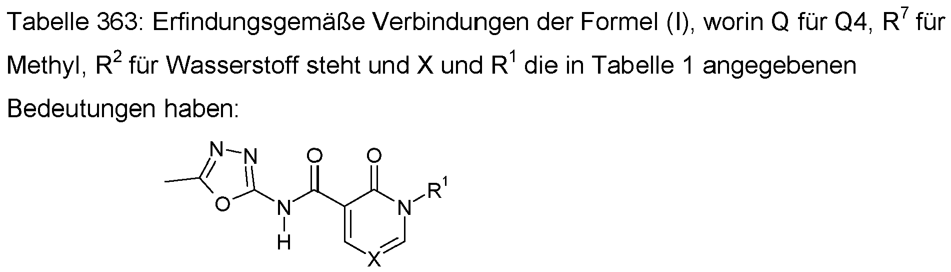

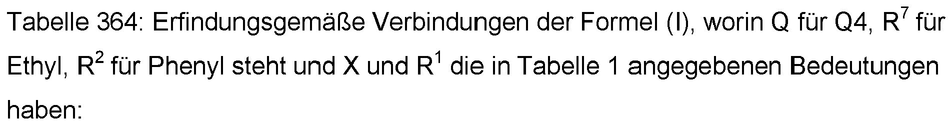

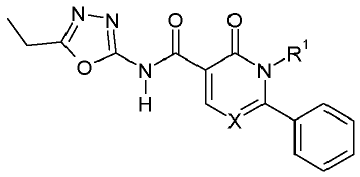

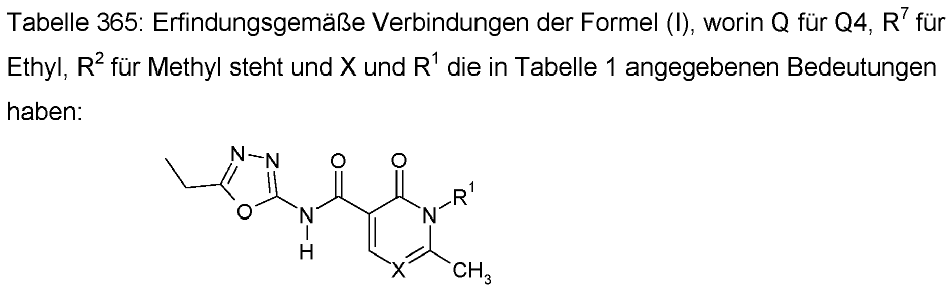

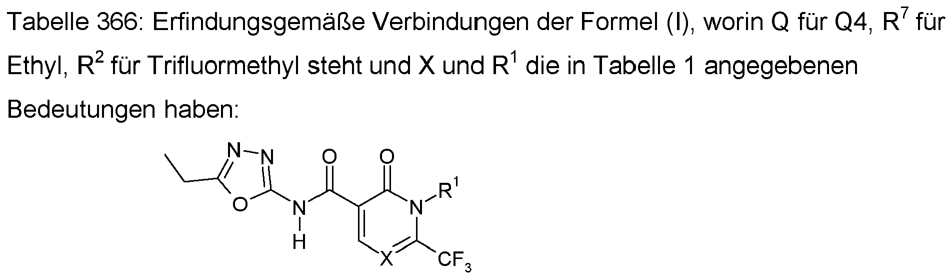

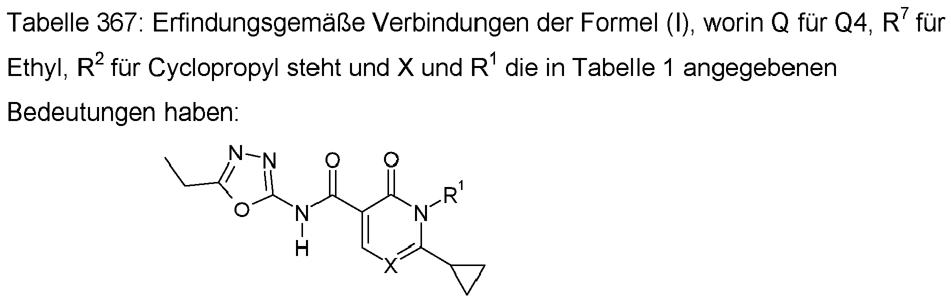

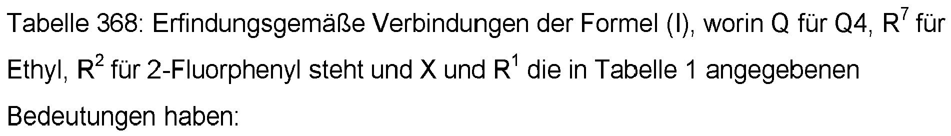

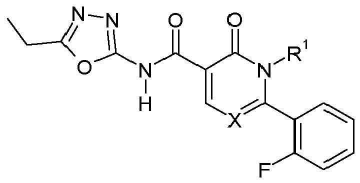

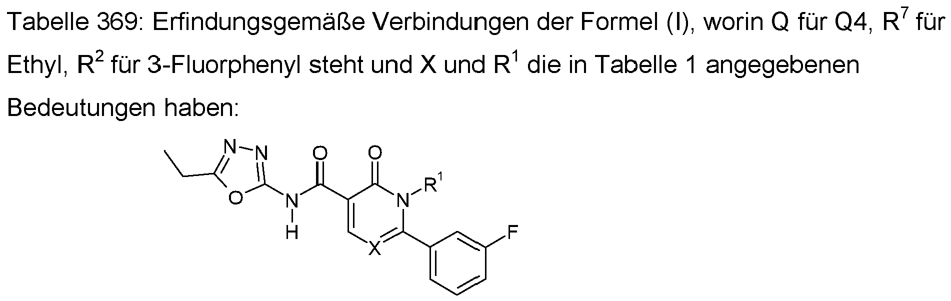

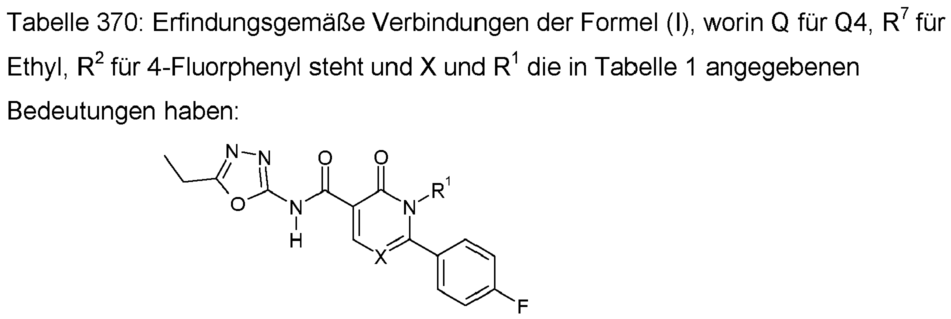

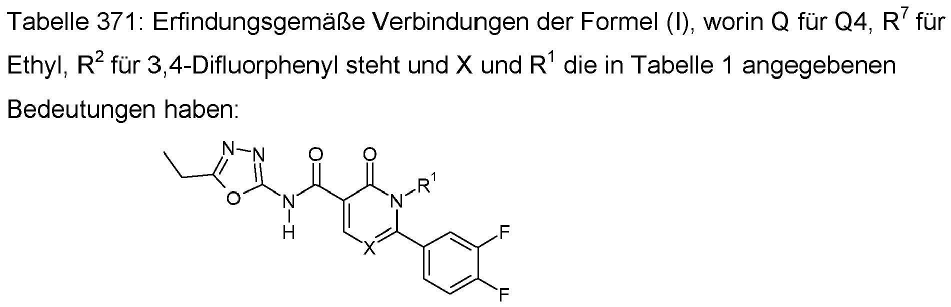

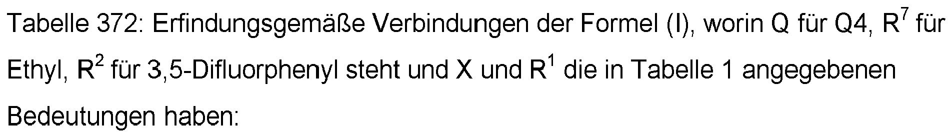

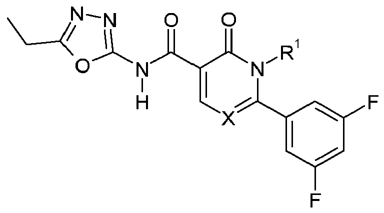

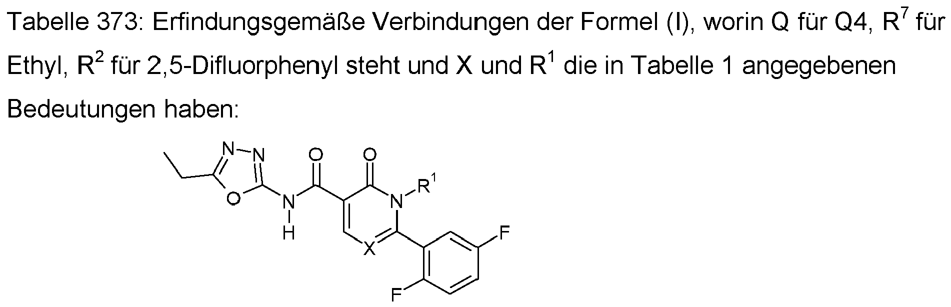

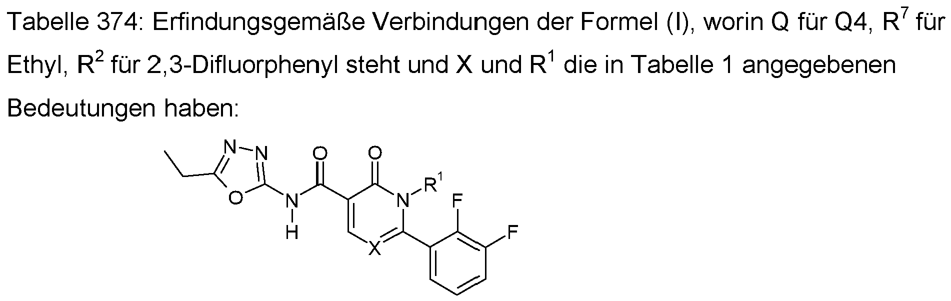

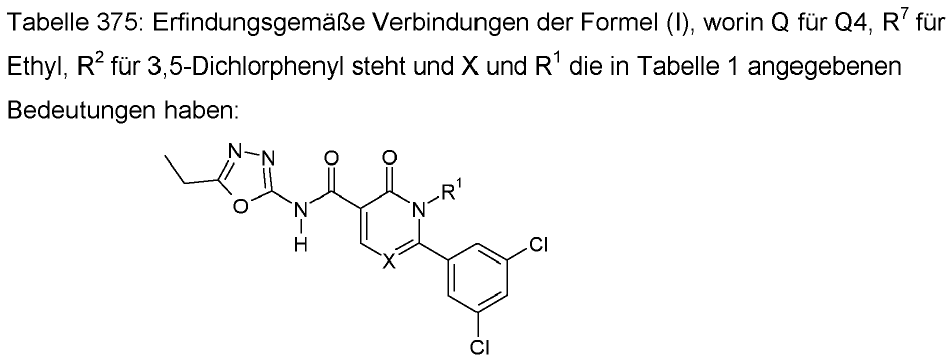

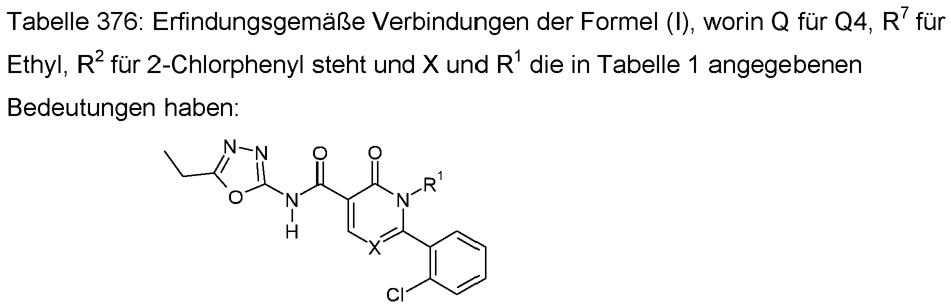

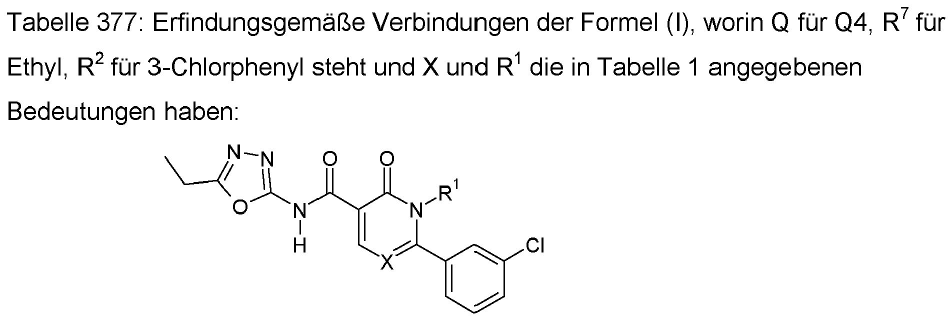

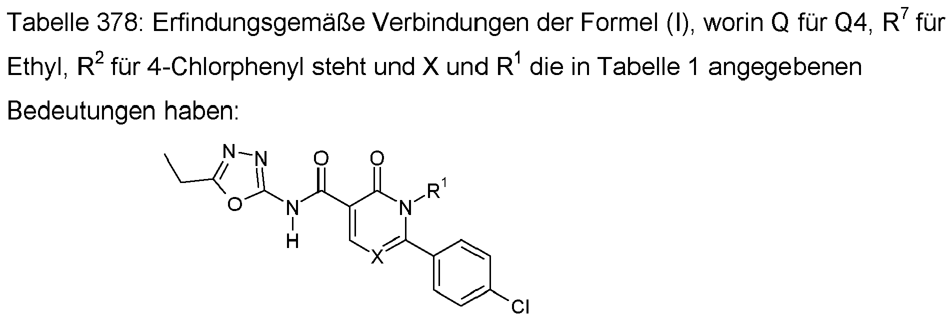

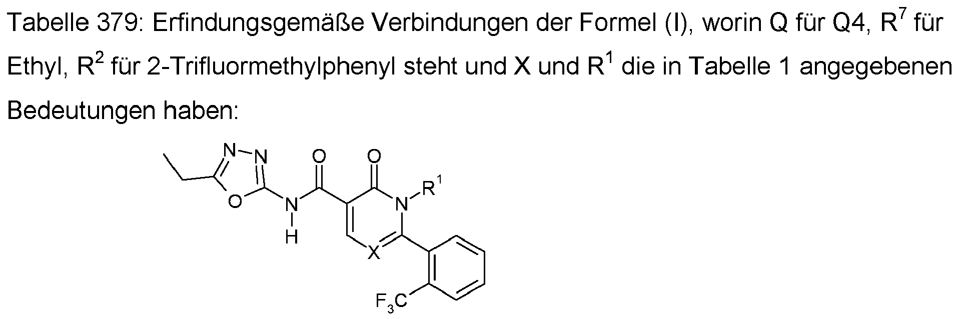

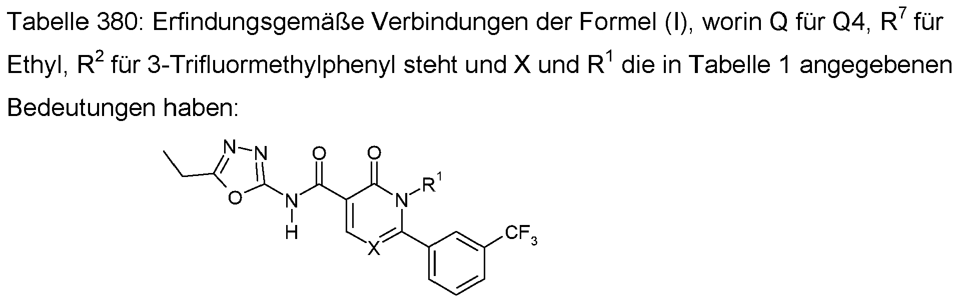

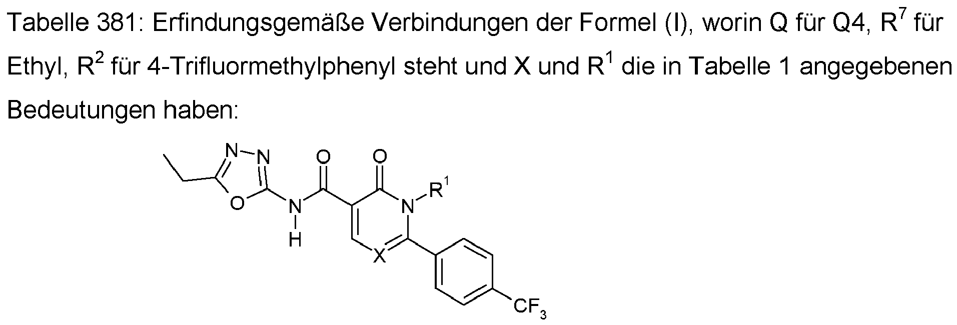

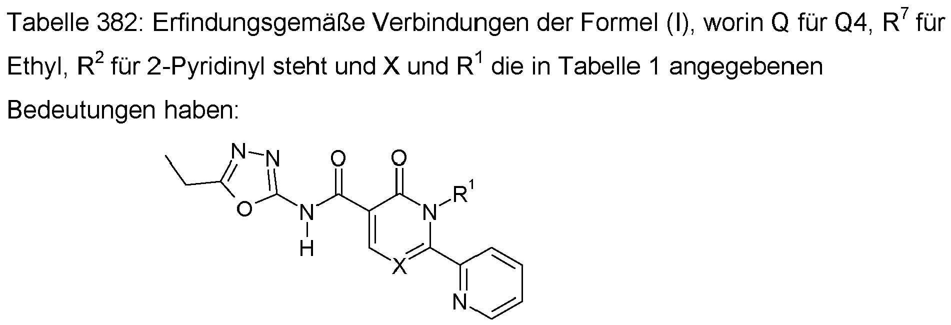

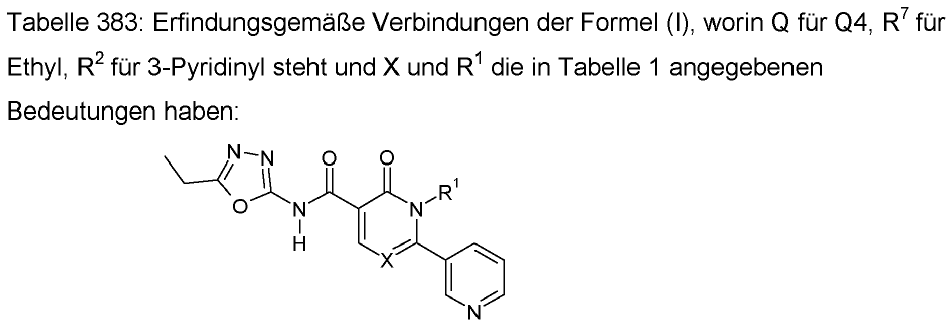

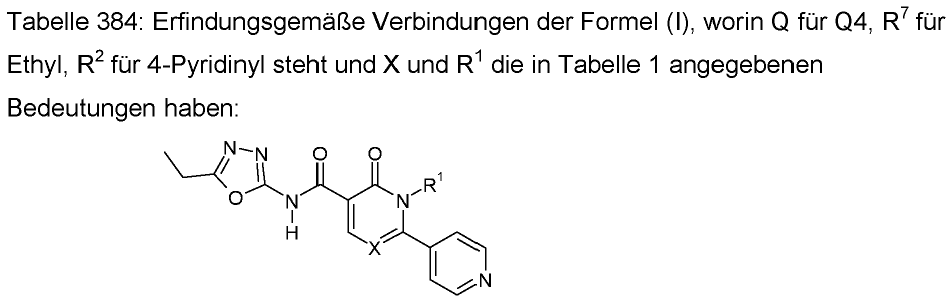

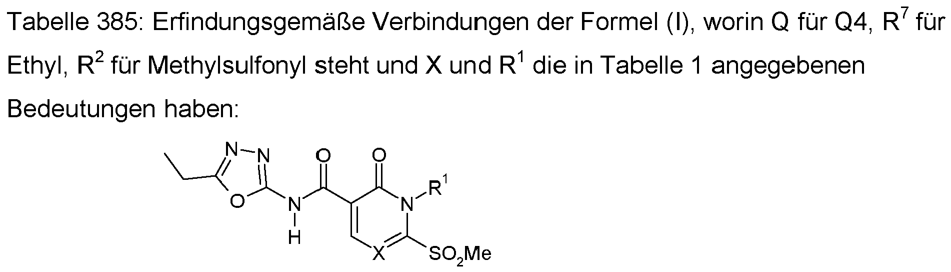

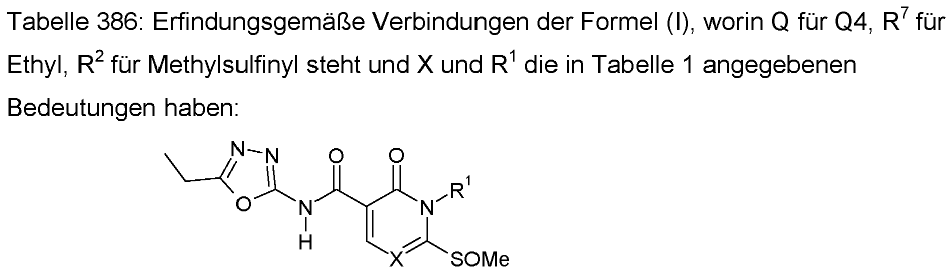

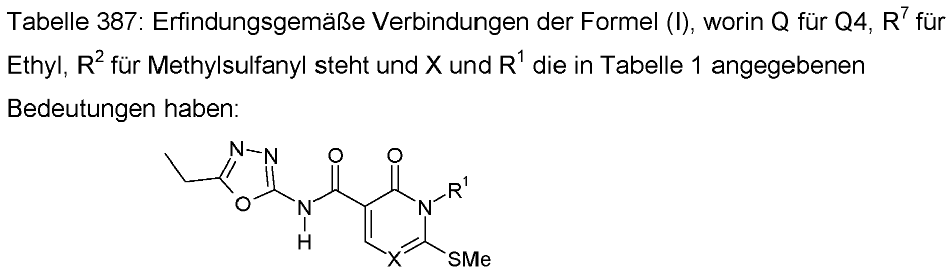

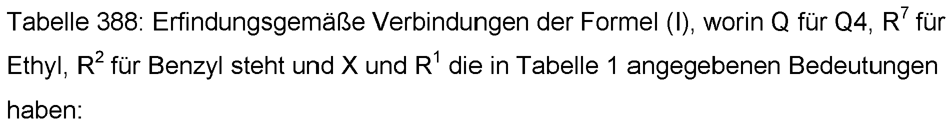

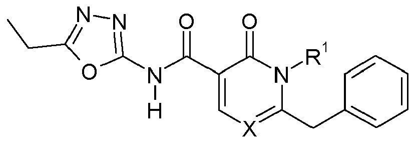

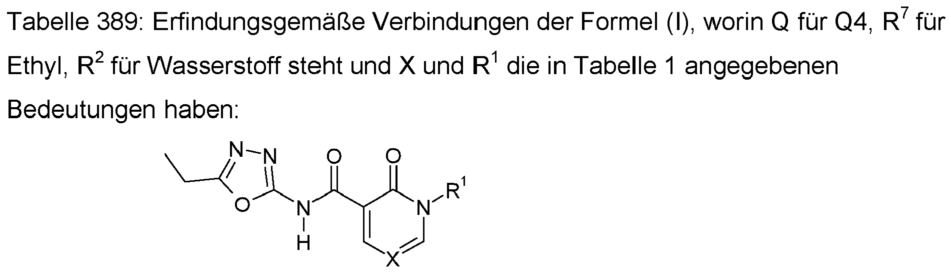

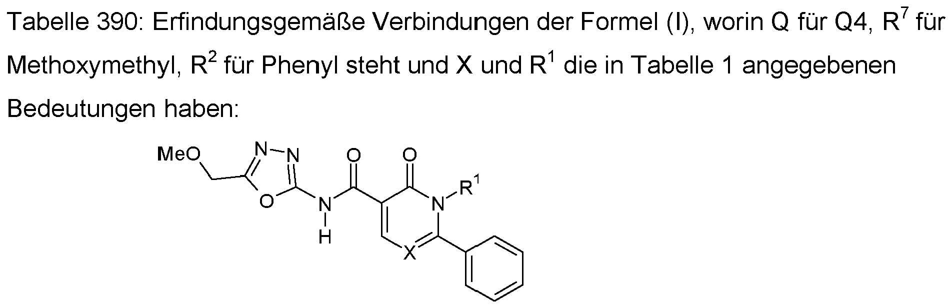

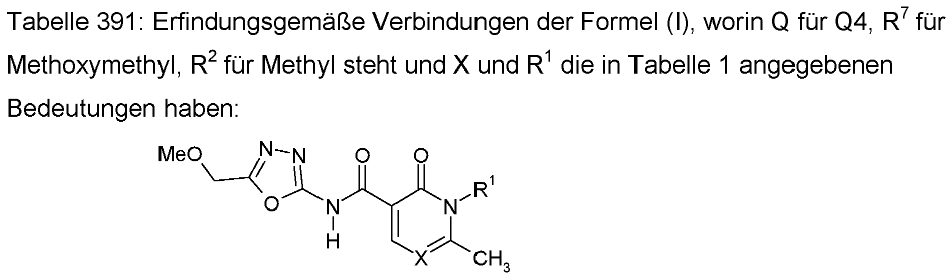

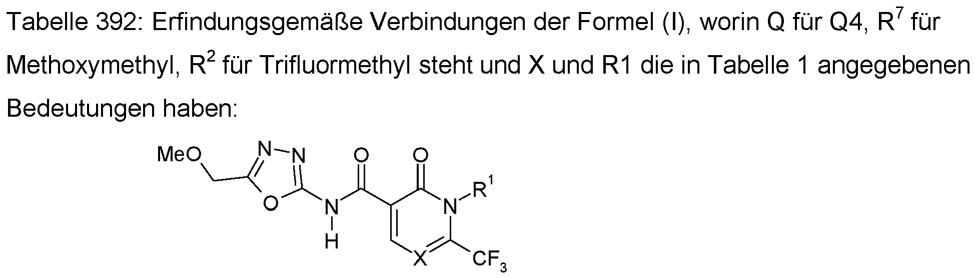

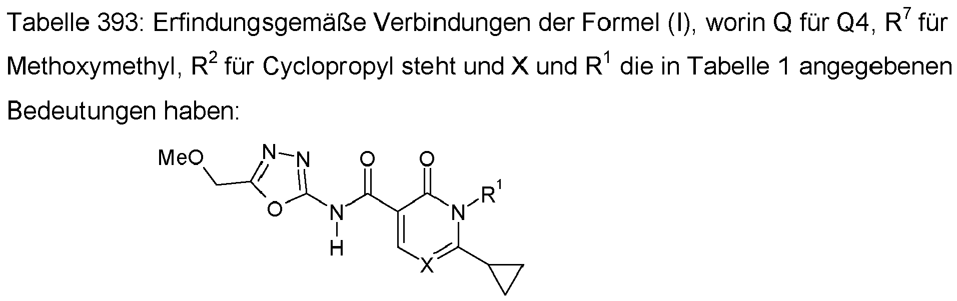

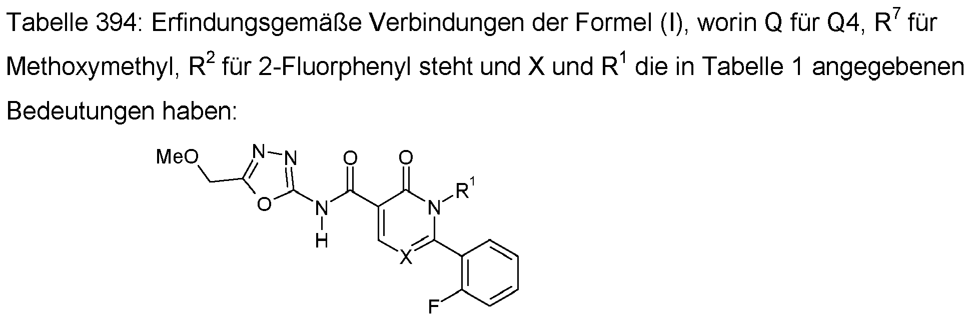

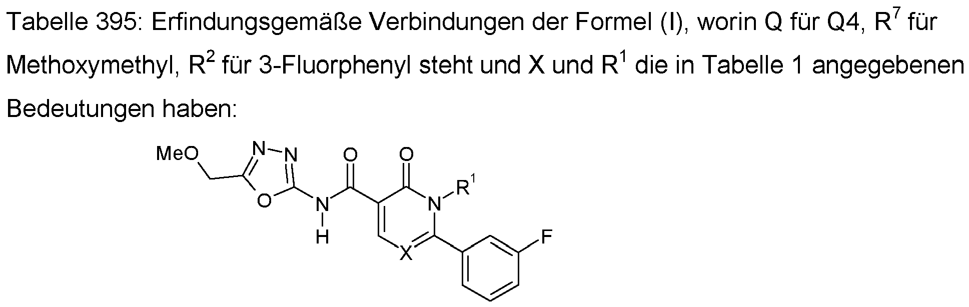

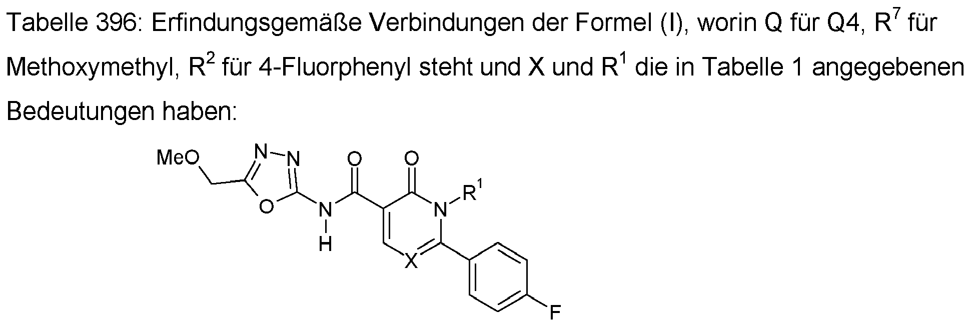

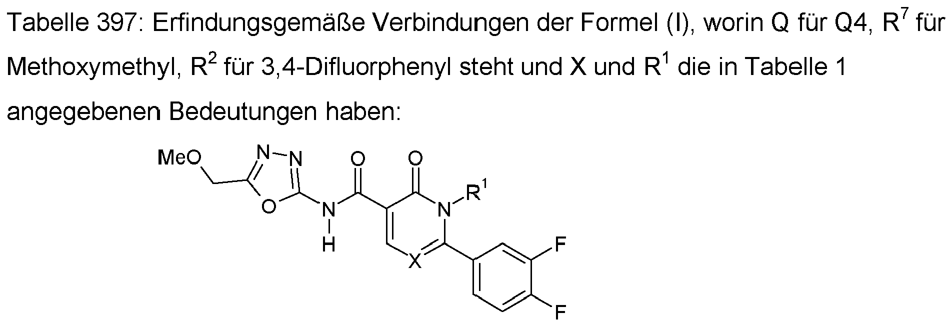

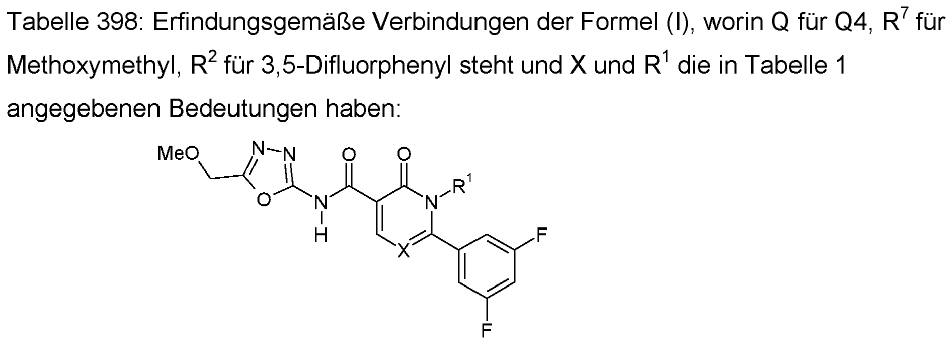

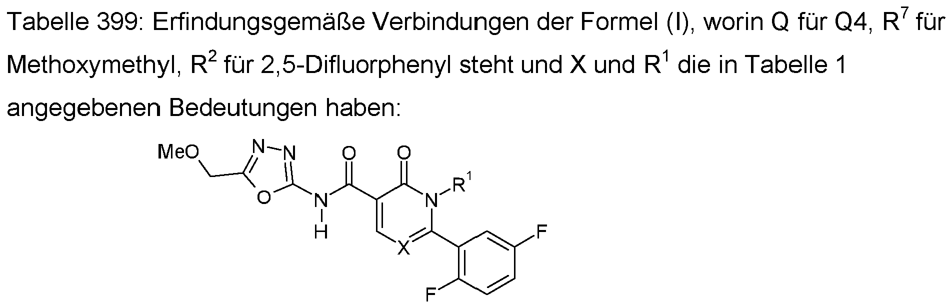

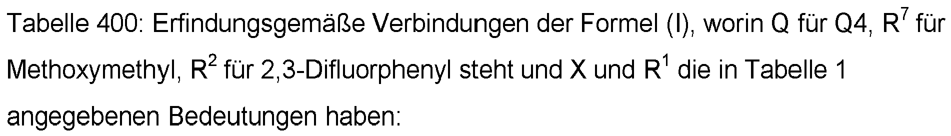

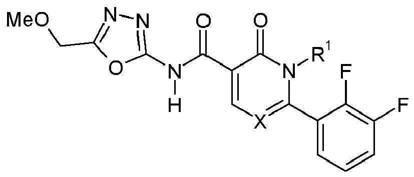

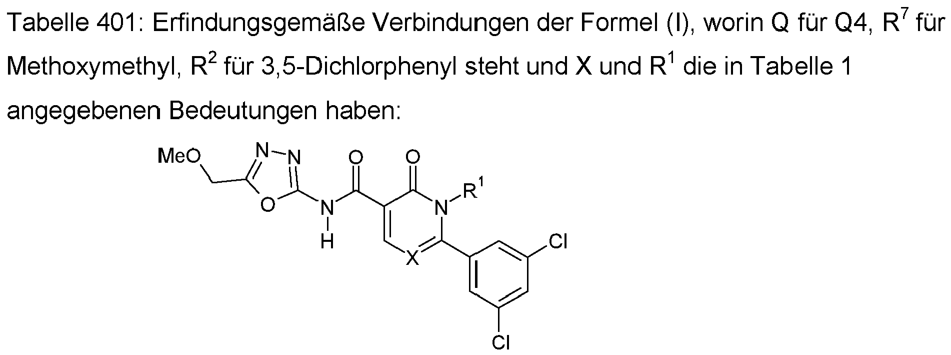

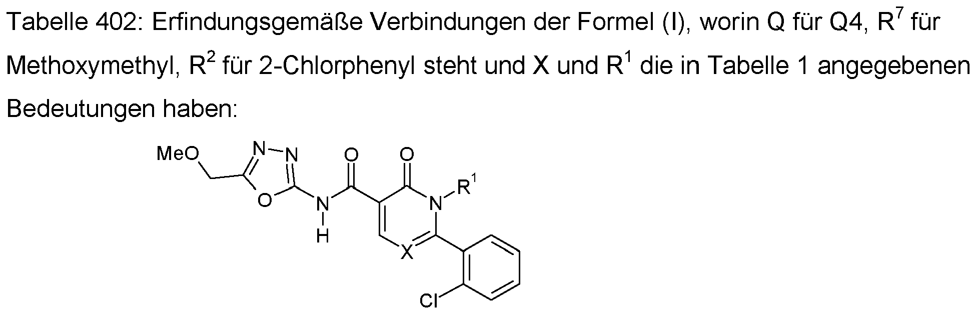

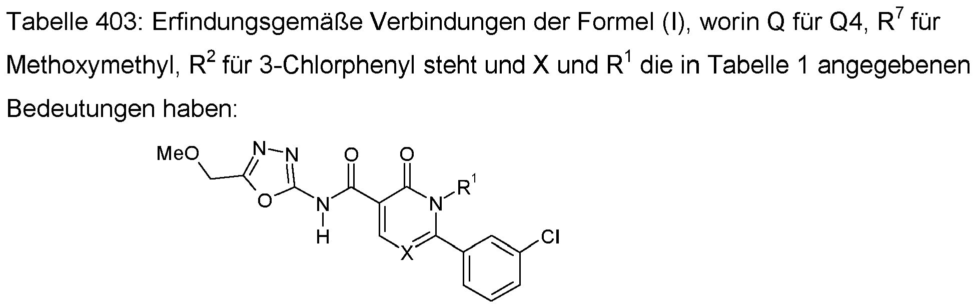

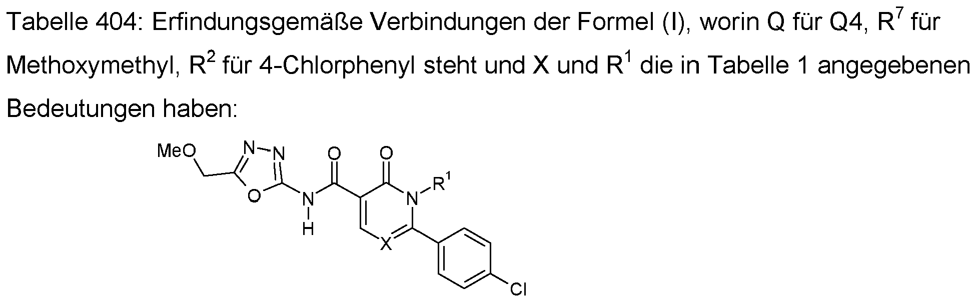

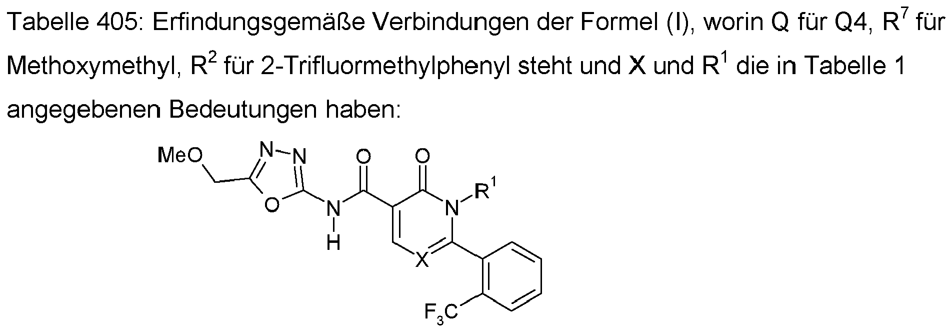

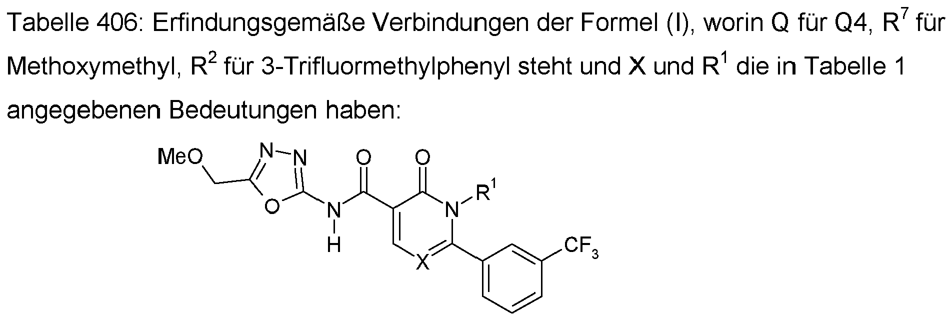

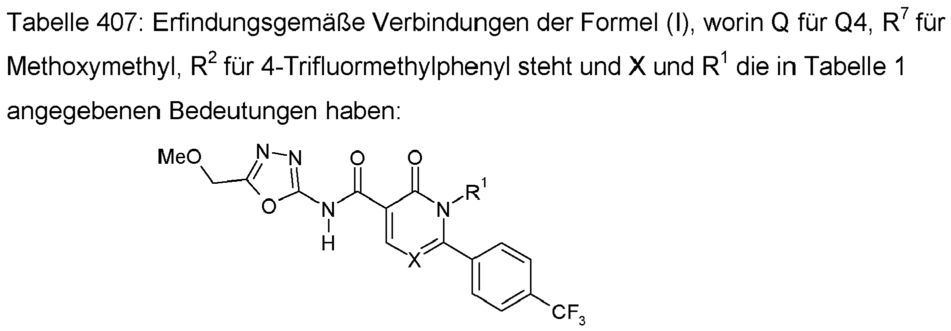

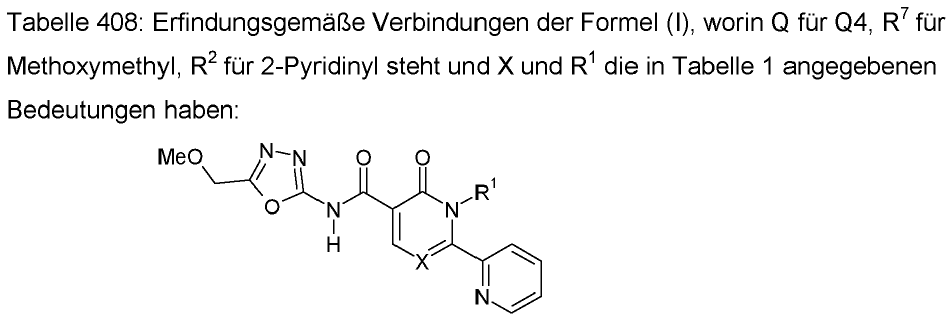

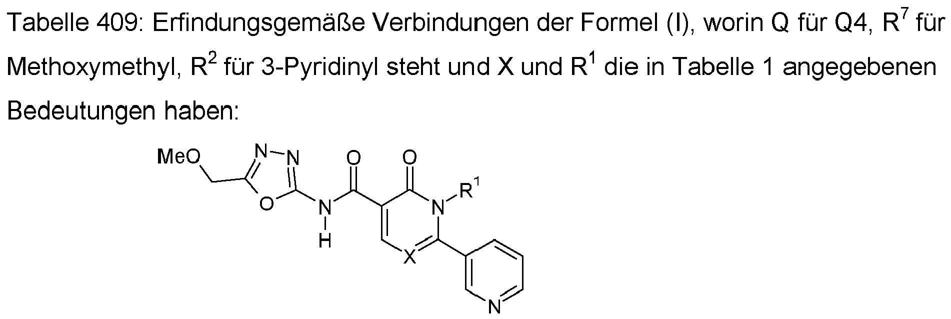

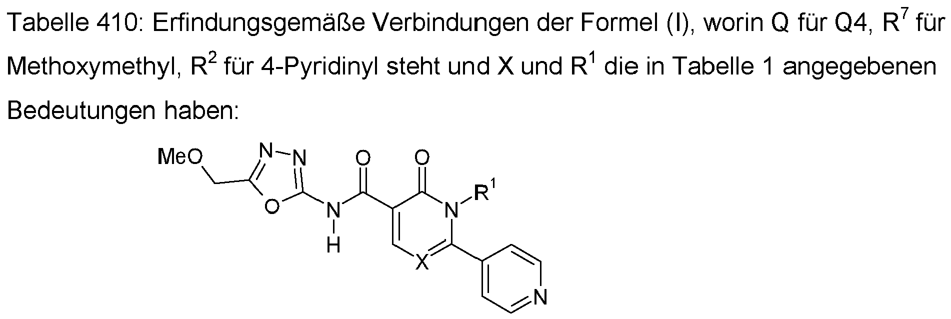

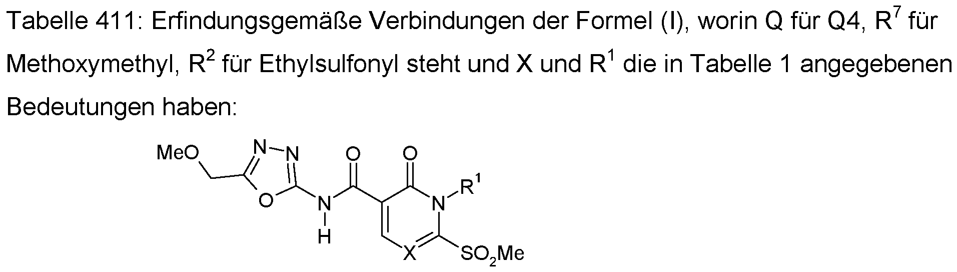

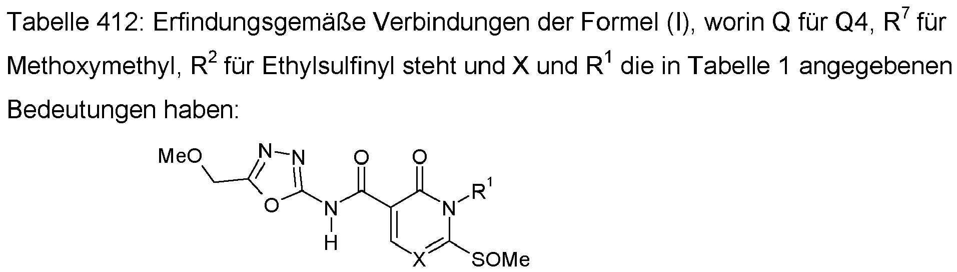

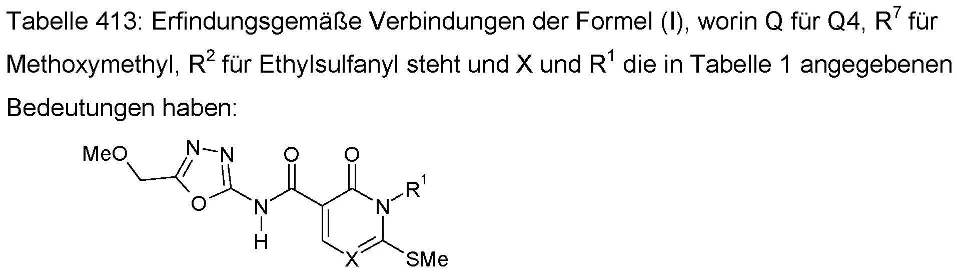

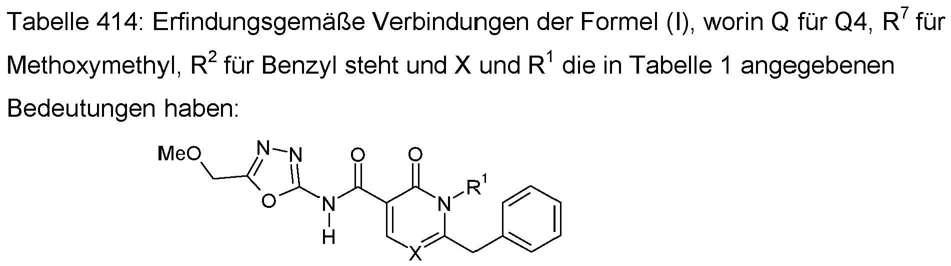

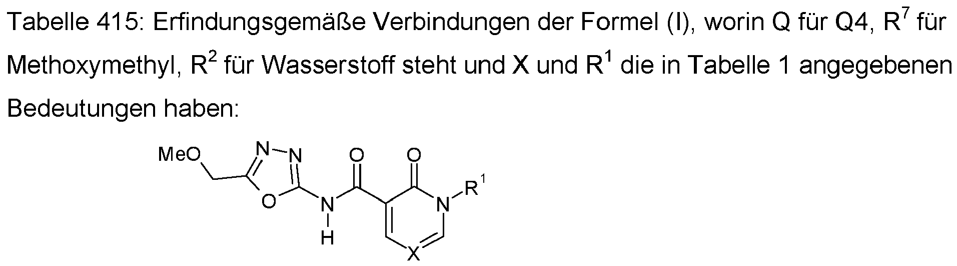

### A. Chemische Beispiele

### 1. Synthese von 2-(3,5-Difluorphenyl)-1-(2-methoxyethyl)-N-(1-methyl-1H-tetrazol-5-yl)-6-oxo-1,6-dihydropyrimidin-5-carboxamid

155 mg (0.5 mmol, 1.1 eq) von aus WO2011/031658 bekannter 2-(3,5-Difluorphenyl)-1-(2-methoxyethyl)-6-oxo-1,6-dihydropyrimidin-5-carbonsäure wurden in 2 ml Dichlormethan vorgelegt, 2 eq Oxalsäuredichlorid und 1 Tropfen DMF zugegeben und anschließend 2 h bei Raumtemperatur (RT) gerührt, Die Reaktionsmischung wurde eingeengt und in 3 ml Dioxan aufgenommen(Lsg.1).

1-Methyl-5-aminotetrazol wurde in 2 ml Dioxan gelöst und auf 70 °C erwärmt, anschließend wurde Lsg.1 zugetropft, und zwei Stunden bei 70°C gerührt. Das Reaktionsgemisch wurde eingeengt und in Dichlormethan aufgenommen und mit NaHCO3- Lsg. gewaschen, die organische Phase getrocknet und eingeengt. Der erhaltenene Rückstand wurde säulenchromatographisch an Kieselgel gereinigt (Gradient Ethylacetat/n-Heptan 1:1 to 100:0). Man erhielt so 38 mg an Zielverbindung Q1-I-4.

### 2. 2-(3,5-Difluorphenyl)-N-(5-ethyl-1,3,4-oxadiazol-2-yl)-1-(2-methoxyethyl)-6-oxo-1,6-dihydropyrimidin-5-carboxamid

250 mg (0.8 mmol, 1.0 eq) von aus WO2011/031658 bekannter 2-(3,5-Difluor-phenyl)-1-(2-methoxyethyl)-6-oxo-1,6-dihydropyrimidin-5-carbonsäure wurden in 10 ml CH₂Cl₂ vorgelegt und mit 0.77 g Propanphosphonsäureanhydrid als 50%ige Lösung in THF zugegeben. Man ließ 1 h bei RT rühren und versetzte mit 5 Equivalenten NEt₃ und einer Spatelspitze N,N-4-Dimethylaminopyridin. Nach 4 Tagen wurde die Reaktionslösung mit 20 ml CH₂Cl₂ verdünnt und zunächst mit 5 ml Wasser und dann zweimal mit je 5 ml 2n HCl extrahiert. Die organische Phase wurde getrocknet, eingeengt und der Rückstand säulenchromatographisch (RP18 Kieselgel, Gradient Wasser + 0.05% Trifluoressigsäure / Acetonnitril 80:20 nach 0:100) gereinigt. Man erhielt 10 mg an Zielverbindung Q4-II-2.

In Analogie zu der Herstellung der Verbindungen Nr. Q1-I-4 und Q4-II-2 sowie gemäß den allgemeinen Angaben zur Herstellung erhält man folgende Verbindungen der Formel (I):

**Tabelle 416: Erfindungsgemäße Verbindungen der allgemeinen Formel (I), worin Q für Q1 und X für N steht:**

| | | | | |
|---|---|---|---|---|
| 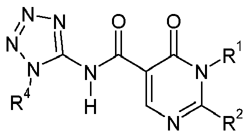 | | | | |

| Nr. | R¹ | R² | R⁴ | Physikalische Daten (¹H-NMR, 400 MHz) |
|---|---|---|---|---|
| Q1-I-1 | 2-Me-Ph | 3,5-F₂-Ph | Me | |
| Q1-I-2 | n-Pr | 3,5- F₂-Ph | Me | |
| Q1-I-3 | n-Pr | Ph | Me | |
| Q1-I-4 | -CH₂-CH₂-OMe | 3,5- F₂-Ph | Me | DMSO-d₆, 9.05 (s, 1H), 7.21 (m, 2H), 7.04 (m, 1H), 4.34 (t, 2H), 4.08 (s, 3H), 3.71 (t, 2H), 3.27 (s, 3H) ppm |
| Q1-I-5 | | | | |

**Tabelle 417: Erfindungsgemäße Verbindungen der allgemeinen Formel (I), worin Q für Q1 und X für C-R³ steht:**

| | | | | | |
|---|---|---|---|---|---|
| 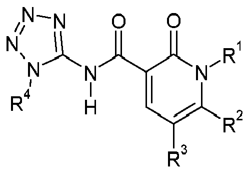 | | | | | |

| Nr. | R¹ | R² | R³ | R⁴ | Physikalische Daten (¹H-NMR, 400 MHz) |
|---|---|---|---|---|---|
| Q1-II-1 | 2-Me-Ph | CF₃ | H | Me | |
| Q1-II-2 | -CH₂-CH₂-OMe | CF₃ | H | Me | |
| Q1-II-3 | Me | CF₃ | H | Me | |

**Tabelle 418: Erfindungsgemäße Verbindungen der allgemeinen Formel (I), worin Q für Q2 und X für N steht:**

| | | | | |
|---|---|---|---|---|
| 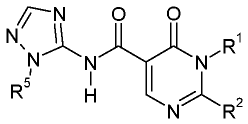 | | | | |

| Nr. | R¹ | R² | R⁵ | Physikalische Daten (¹H-NMR, 400 MHz) |
|---|---|---|---|---|
| Q2-I-1 | 2-Me-Ph | 3,5-F₂-Ph | Me | |
| Q2-I-2 | n-Pr | 3,5- F₂-Ph | Me | |
| Q2-I-3 | n-Pr | Ph | Me | |
| Q2-I-4 | -CH₂-CH₂-OMe | 3,5- F₂-Ph | Me | |

**Tabelle 419: Erfindungsgemäße Verbindungen der allgemeinen Formel (I), worin Q für Q2 und X für C-R³ steht:**

| | | | | | |
|---|---|---|---|---|---|
| 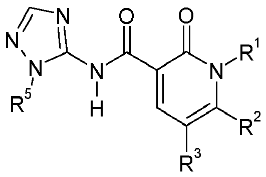 | | | | | |

| Nr. | R¹ | R² | R³ | R⁵ | Physikalische Daten (¹H-NMR, 400 MHz) |
|---|---|---|---|---|---|
| Q2-II-1 | 2-Me-Ph | CF₃ | H | Me | |
| Q2-II-2 | -CH₂-CH₂-OMe | CF₃ | H | Me | |
| Q2-II-3 | Me | CF₃ | H | Me | |

**Tabelle 420: Erfindungsgemäße Verbindungen der allgemeinen Formel (I), worin Q für Q3 und X für N steht:**

| | | | | |
|---|---|---|---|---|
| 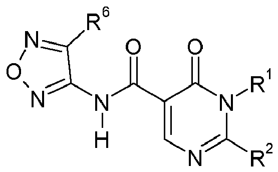 | | | | |

| Nr. | R¹ | R² | R⁴ | Physikalische Daten (¹H-NMR, 400 MHz) |
|---|---|---|---|---|
| Q3-I-1 | 2-Me-Ph | 3,5-F₂-Ph | Cl | |
| Q3-I-2 | n-Pr | 3,5- F₂-Ph | Cl | |
| Q3-I-3 | n-Pr | Ph | Cl | |
| Q3-I-4 | -CH₂-CH₂-OMe | 3,5- F₂-Ph | Cl | CDCl₃, 9.12 (s, 1H), 7.20 (m, 2H), 7.03 (m,1H), 4.35 (t, 2H), 3.71 (t, 2H), 3.26 (s, 3H) ppm |
| Q3-I-5 | -CH₂-CH₂-OMe | 3,5- F₂-Ph | CH₃ | CDCl₃, 9.08 (s, 1H), 7.20 (m, 2H), 7.03 (m,1H), 4.33 (t, 2H), 3.71 (t, 2H), 3.27 (s, 3H) ppm |

**Tabelle 421: Erfindungsgemäße Verbindungen der allgemeinen Formel (I), worin Q für Q3 und X für C-R³ steht:**

| | | | | | |
|---|---|---|---|---|---|
| 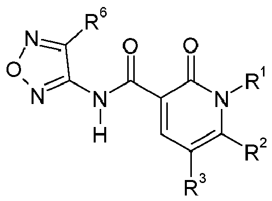 | | | | | |

| Nr. | R¹ | R² | R³ | R⁶ | Physikalische Daten (¹H-NMR, 400 MHz) |
|---|---|---|---|---|---|
| Q3-II-1 | 2-Me-Ph | CF₃ | H | Cl | |
| Q3-II-2 | -CH₂-CH₂-OMe | CF₃ | H | Cl | |
| Q3-II-3 | Me | CF₃ | H | Cl | |

**Tabelle 422: Erfindungsgemäße Verbindungen der allgemeinen Formel (I), worin Q für Q4 und X für N steht:**

| | | | | |
|---|---|---|---|---|
| 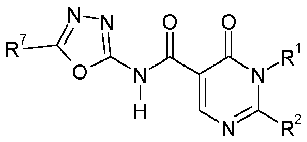 | | | | |

| Nr. | R¹ | R² | R⁷ | Physikalische Daten (¹H-NMR, 400 MHz) |
|---|---|---|---|---|
| Q4-I-1 | 2-Me-Ph | 3,5-F₂-Ph | Et | |
| Q4-I-2 | n-Pr | 3,5- F₂-Ph | Et | |
| Q4-I-3 | n-Pr | Ph | Et | |
| Q4-I-4 | -CH₂-CH₂-OMe | 3,5- F₂-Ph | Et | CDCl₃, 9.07 (s, 1H), 7.20 (m, 2H), 7.02 (m,1H), 4.32 (t, 2H), 3.70 (t, 2H), 3.26 (s, 3H), 2.90 (q, 2H), 1.41 (t, 3H) ppm |

**Tabelle 423: Erfindungsgemäße Verbindungen der allgemeinen Formel (I), worin Q für Q4 und X für C-R3 steht:**

| | | | | | |
|---|---|---|---|---|---|
| 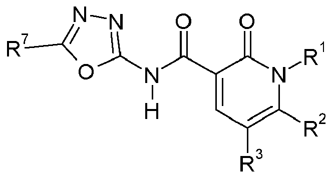 | | | | | |

| Nr. | R¹ | R² | R³ | R⁷ | Physikalische Daten (¹H-NMR, DMSO-d₆, 400 MHz) |
|---|---|---|---|---|---|
| Q4-II-1 | 2-Me-Ph | CF₃ | H | Et | |
| Q4-II-2 | -CH₂-CH₂-OMe | CF₃ | H | Et | |
| Q4-II-3 | Me | CF₃ | H | Et | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
      mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen zahlreiche Verbindungen bei einer Aufwandmenge von 320 g/ha jeweils eine mindestens 80 %-ige Wirkung gegen eine Vielzahl von monokotylen und dikotylen Schadpflanzen.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen zahlreiche Verbindungen bei einer Aufwandmenge von 80 g/ha jeweils eine mindestens 80 %-ige Wirkung gegen eine Vielzahl von monokotylen und dikotylen Schadpflanzen.

## Patentansprüche

1. 6-Oxo-1,6-dihydropyrimidin-5-carboxamide und 2-Oxo-1,2-dihydropyridin-3-carboxamide der Formel (I) oder deren Salze 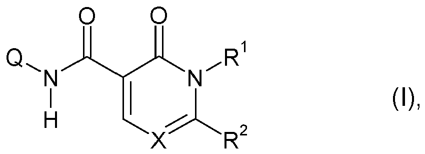 worin die Substituenten folgende Bedeutungen haben
X bedeutet N oder C-R³,
Q bedeutet einen Rest Q1, Q2, Q3 oder Q4, 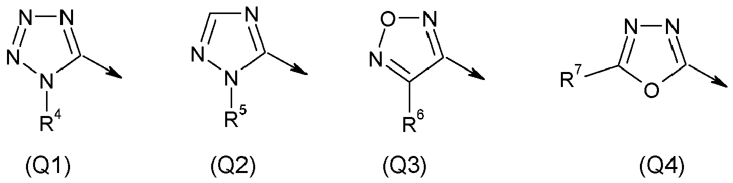
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-Cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfimyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl) thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(S-Phenyl), W¹-(SO₂-Phenyl), W²-(SO₂CH₂-Phenyl) oder W²-(SCH₂-Phenyl), wobei die Phenylringe der sieben vorstehend genannten Reste jeweils u Substituenten R⁸ tragen, oder
R¹ bedeutet G oder W²G,
R² bedeutet Phenyl oder W³-(Phenyl), wobei die Phenylringe der zwei vorstehend genannten Reste jeweils u Substituenten R⁸ tragen, oder
R² bedeutet G, W⁴G, Wasserstoff, Cyano, Hydroxy, Amino, Nitro, CHO, C(=O)OH, C(=O)NH₂, C(=S)NH₂, C(=O)NHCN, C(=O)NHOH, SH, SO₂NH₂, SO₂NHCN, SO₂NHOH, SF₅, NHCHO, NHNH₂, NHOH, NHCN, NHC(O)NH₂, (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₁-C₆)-Cycloalkyl-(C₁-C₆)-cycloalkyl, Halogen-(C₁-C₆)-Cycloalkyl-(C₁-C₆)-alkyl, (C₁-C₆)-Alkyl-(C₃-C₇)-cycloalkyl-(C₁-C₆)-alkyl, (C₃-C₈)-Cycloalkenyl, Halogen-(C₃-C₈)-Cycloalkenyl, (C₁-C₆)-Alkoxy (C₁-C₆)-alkyl, (C₁-C₆)-Alkoxy (C₁-C₆)-alkenyl, (C₁-C₆)-Alkyl-(C₃-C₇)-cycloalkyl, (C₁-C₆)-Alkoxy-(C₃-C₇)-Cycloalkyl, (C₁-C₆)-Cycloalkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₂-C₈)-Alkylthioalkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxycarbonyl, (C₁-C₆)-Alkylamino-carbonyl, Di-(C₁-C₆)-alkyl-amino-carbonyl, (C₃-C₇)-Cycloalkyl-amino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-(C₁-C₆)-halogen-alkyl, Halogen-(C₁-C₆)-alkoxy-(C₁-C₆)-halogen-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₇)-Cycloalkyl-(C₁-C₆)-alkoxy, (C₂-C₆)-Alkenyloxy, Halogen-(C₂-C₆)-alkenyloxy, (C₂-C₆)-Alkinyloxy, Halogen-(C₃-C₆)-alkinyloxy, (C₁-C₆)-Alkoxy-(C₁-C₆)-alkoxy, (C₁-C₈)-Alkyl-carbonyloxy, Halogen-(C₁-C₈)-alkylcarbonyloxy, (C₃-C₇)-Cycloalkyl-carbonyloxy, (C₁-C₆)-Alky-carbonyl-(C₁-C₆)-alkoxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₁₀)-Cylcoalkylsulfonyl, (C₁-C₆)-Alkylcarbonylthio, (C₁-C₆)-Alkyl(thiocarbonyl)thio, (C₃-C₈)-Cycloalkylsulfinyl, (C₁-C₆)-Alkylaminosulfonyl, Di-(C₁-C₆)-alkylaminosulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₈)-Cycloalkylamino, (C₂-C₈)-Alkylcarbonylamino, Halogen-(C₂-C₈)-alkylcarbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Halogen-tri-(C₁-C₄)-alkylsilyl,
oder
R¹ und R² bilden gemeinsam mit den beiden Atomen, an die sie gebunden sind, einen 5-, 6- oder 7-gliedrigen, ungesättigten, teilgesättigten oder gesättigten Ring, der neben Kohlenstoffatomen jeweils m Stickstoffatome, n Sauerstoffatome, n Schwefelatome und n Elemente S(O), S(O)₂ oder C(O) als Ringglieder umfasst, dessen Kohlenstoffatome durch s Reste aus der Gruppe bestehend aus Halogen, Cyano, (C₁-C₆)-Alkyl, (C₂-C₁₀)-Alkenyl, (C₂-C₁₀)-Alkinyl, (C₁-C₆)-Haloalkyl, (C₃-C₈)-Cycloalkyl, (C₂-C₈)-Alkoxyalkyl und Phenyl substituiert sind, dessen Stickstoffatome durch n Reste aus der Gruppe bestehend aus (C₁-C₆)-Alkyl und Phenyl substituiert sind,
und worin die vorstehend genannten Phenylreste durch u Reste aus der Gruppe bestehend aus Cyano, Nitro, Halogen, (C₁-C₆)-Alkyl, (C₁-C₆)-Haloalkyl und (C₁-C₆)-Alkoxy substituiert sind,
R³ bedeutet Wasserstoff, Halogen, Nitro. Cyano, (C₁-C₆)-Alkyl, (C₂-C₆)-Alkenyl, (C₂-C₆)-Alkinyl, (C₃-C₈)-Cycloalkyl, (C₃-C₈)-Cycloalkyl-(C₁-C₆)-alkyl, (C₁-C₆)-Alkoxy, (C₁-C₆)-Alkoxy-(C₁-C₆)-alkyl, (C₁-C₆)-Alkylthio, Halogen-(C₁-C₆)-alkylthio, (C₁-C₆)-Alkylsulfinyl, (C₁-C₆)-Alkylsulfonyl, (C₁-C₆)-Alkylsulfonyl-(C₁-C₆)-alkyl, Halogen-(C₁-C₆)-alkylsulfinyl, Halogen-(C₁-C₆)-alkylsulfonyl, Halogen-(C₁-C₆)-alkyl, Halogen-(C₂-C₆)-alkenyl, Halogen-(C₁-C₆)-alkoxy, Phenyl oder W³-(Phenyl), wobei die Phenylringe der zwei vorstehend genannten Reste jeweils u Substituenten R⁸ tragen,
R⁴ bedeutet jeweils durch s Reste aus der Gruppe bestehend aus Nitro, Cyano, SiR¹¹₃, PO(OR¹¹)₃, S(O)ₙ-(C₁-C₆)-Alkyl, (C₁-C₆)-Alkoxy, Halogen-(C₁-C₆)-alkoxy, N(R⁹)₂, COR⁹, COOR⁹, OCOR⁹, OCO₂R⁹, NR⁹COR⁹, NR⁹SO₂R¹⁰, (C₃-C₆)-Cycloalkyl, Heteroaryl, Heterocyclyl, Phenyl, D-Heteroaryl, D-Heterocyclyl, D-Phenyl und D-Benzyl substituiertes (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei Heteroaryl, Heterocyclyl, Phenyl, D-Heteroaryl, D-Heterocyclyl, D-Phenyl und D-Benzyl jeweils durch s Reste aus der Gruppe bestehend aus Methyl, Ethyl, Methoxy, Trifluormethyl und Halogen substituiert sind, und wobei Heterocyclyl n Oxogruppen trägt, oder
R⁴ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes (C₃-C₇)-Cycloalkyl, Heteroaryl, Heterocyclyl oder Phenyl,
R⁵ bedeutet jeweils durch s Reste aus der Gruppe bestehend aus Nitro, Cyano, SiR¹¹₃, PO(OR¹¹)₃, S(O)ₙ-(C₁-C₆)-Alkyl, (C₁-C₆)-Alkoxy, Halogen-(C₁-C₆)-alkoxy, N(R⁹)₂, COR⁹, COOR⁹, OCOR⁹, OCO₂R⁹, NR⁹COR⁹, NR⁹SO₂R¹⁰, (C₃-C₆)-Cycloalkyl, Heteroaryl, Heterocyclyl, Phenyl, D-Heteroaryl, D-Heterocyclyl, D-Phenyl und D-Benzyl substituiertes (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl oder Halogen-(C₂-C₆)-alkinyl, wobei Heteroaryl, Heterocyclyl, Phenyl, D-Heteroaryl, D-Heterocyclyl, D-Phenyl und D-Benzy jeweils durch s Reste aus der Gruppe bestehend aus Methyl, Ethyl, Methoxy, Trifluormethyl und Halogen substituiert sind, und wobei Heterocyclyl n Oxogruppen trägt, oder
R⁵ bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes (C₃-C₇)-Cycloalkyl, Heteroaryl, Heterocyclyl oder Phenyl,
R⁶ bedeutet Wasserstoff, (C₁-C₆)-Alkyl, (C₃-C₇)-Cycloalkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, Halogen-(C₂-C₆)-alkenyl, (C₂-C₆)-Alkinyl, (C₂-C₆)-Alkinyloxy, Halogen-(C₂-C₆)-alkinyl, Cyano, Nitro, Methylsulfanyl, Methylsulfinyl, Methylsulfonyl, (C₁-C₆)-Alkylcarbonyl-amino, (C₁-C₆)-Alkoxycarbonylamino, Benzoylamino, Methoxycarbonyl, Ethoxy-carbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus Methyl, Ethyl, Methoxy, Trifluormethyl und Halogen substituiertes Heteroaryl, Heterocyclyl oder Phenyl;
R⁷ bedeutet Wasserstoff, (C₁-C₆)-Alkyl, R¹²O-(C₁-C₆)-Alkyl, CH₂R¹³, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹², NHR¹², Methoxycarbonyl, Ethoxycarbonyl, Methylcarbonyl, Trifluormethyl-carbonyl, Dimethylamino, Acetylamino, Methylsulfanyl, Methylsulfinyl, Methylsulfonyl, oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl,
R⁸ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino;
R⁹ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R¹⁰ bedeutet (C₁-C₆)-Alkyl, (C₂-C₆)-Alkenyl, (C₂-C₆)-Alkinyl oder Phenyl,
R¹¹ bedeutet (C₁-C₆)-Alkyl,
R¹² bedeutet Wasserstoff, jeweils durch s Reste aus der Gruppe bestehend aus Cyano, Halogen, Nitro, Rhodano, OR¹⁴, S(O)ₙR¹⁵, N(R¹⁴)₂, NR¹⁴OR¹⁴, COR¹⁴, OCOR¹⁴ SCOR¹⁵ NR¹⁴COR¹⁴ NR¹⁴SO₂R^{15,} CO₂R¹⁴, COSR¹⁵, CON(R¹⁴)₂ und (C₁-C₄)-Alkoxy-(C₂-C₆)-alkoxycarbonyl substituiertes (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁴-Heteroaryl oder (C₁-C₆)-Alkyl-NR¹⁴-Heterocyclyl, wobei Heterocyclyl n Oxogruppen trägt,
R¹³ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Alkoxy, (C₃-C₆)-Cycloalkyl, oder jeweils durch s Reste aus der Gruppe bestehend aus Methyl, Ethyl, Methoxy, Trifluormethyl und Halogen substituiertes Heteroaryl, Heterocyclyl oder Phenyl-,
R¹⁴ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹⁵ bedeutet (C₁-C₆)-Alkyl, (C₂-C₆)-Alkenyl oder (C₂-C₆)-Alkinyl,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
W³ bedeutet (C₁-C₁₀)-Alkylen, (C₂-C₆)-Alkenylen oder (C₂-C₆)-Alkinylen,
W⁴ bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
D bedeutet O, S, oder NR¹⁰,
m bedeutet 1, 2, 3 oder 4,
n bedeutet 0, 1 oder 2,
s bedeutet 0, 1, 2 oder 3,
u bedeutet 0, 1, 2, 3, 4 oder 5.

2. Herbizide Mittel, **gekennzeichnet durch** einen herbizid wirksamen Gehalt an mindestens einer Verbindung der Formel (I) gemäß Anspruch 1.

3. Herbizide Mittel nach Anspruch 2 in Mischung mit Formulierungshilfsmitteln.

4. Herbizide Mittel nach Anspruch 2 oder 3 enthaltend mindestens einen weiteren pestizid wirksamen Stoff aus der Gruppe Insektizide, Akarizide, Herbizide, Fungizide, Safener und Wachstumsregulatoren.

5. Herbizide Mittel nach Anspruch 4 enthaltend einen Safener.

6. Herbizide Mittel nach Anspruch 5 enthaltend cyprosulfamid, cloquintocet-mexyl, mefenpyr-diethyl oder isoxadifen-ethyl.

7. Herbizide Mittel nach einem der Ansprüche 4 bis 6 enthaltend ein weiteres Herbizid.

8. Verfahren zur Bekämpfung unerwünschter Pflanzen, **dadurch gekennzeichnet, daß** man eine wirksame Menge mindestens einer Verbindung der Formel (I) gemäß Anspruch 1 oder eines herbiziden Mittels nach einem der Ansprüche 2 bis 7 auf die Pflanzen oder auf den Ort des unerwünschten Pflanzenwachstums appliziert.

9. Verwendung von Verbindungen der Formel (I) gemäß Anspruch 1 oder von herbiziden Mitteln nach einem der Ansprüche 2 bis 7 zur Bekämpfung unerwünschter Pflanzen.

10. Verwendung nach Anspruch 9, **dadurch gekennzeichnet, daß** die Verbindungen der Formel (I) zur Bekämpfung unerwünschter Pflanzen in Kulturen von Nutzpflanzen eingesetzt werden.

11. Verwendung nach Anspruch 10, **dadurch gekennzeichnet, daß** die Nutzpflanzen transgene Nutzpflanzen sind.
